# EUROPEAN PATENT APPLICATION

(11) **EP 1 857 444 A1**
(43) Date of publication of application: **21.11.2007**
(21) Application number: 06729291.2
(22) Date of filing: 10.03.2006
(51) Int. Cl.: C07D 217/26, A61K 31/472, A61K 31/496, A61K 31/5377, A61P 3/06, A61P 9/10, A61P 29/00, A61P 43/00, C07D 401/12

(54) **TETRAHYDROISOQUINOLINE COMPOUND AND MEDICINAL USE THEREOF**

(30) Priority: 10.03.2005 JP 2005067596
(71) Applicant: Kyoto Pharmaceutical Industries, Ltd., Kyoto-shi, Kyoto 604-8444 (JP)
(72) Inventor: TAKAHASHI, K., c/o KYOTO PHARMACEUTICAL IND. LTD., Kyoto-shi, Kyoto 604-8444 (JP); SHIRAHASE, H., c/o KYOTO PHARMACEUTICAL IND. LTD., Kyoto-shi, Kyoto 604-8444 (JP); KUNISHIRO, K., c/o KYOTO PHARMACEUTICAL IND. LTD., Kyoto-shi, Kyoto 604-8444 (JP); KASAI, M., c/o KYOTO PHARMACEUTICAL IND. LTD., Kyoto-shi, Kyoto 604-8444 (JP)
(74) Representative: von Kreisler Selting Werner
(86) International application number: PCT/JP2006/305301
(87) International publication number: WO 2006/095922

(57) **Abstract**

The present invention provide a tetrahydroisoquinoline compound having a superior ACAT-inhibitory activity and/or anti-oxidation action, particularly, novel compound represented by the formula (I) (wherein each symbol is as described in the specification) and a pharmaceutically acceptable salt thereof.

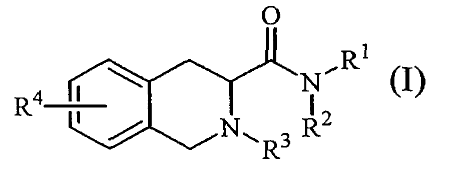

## Description

### Technical Field

The present invention relates to a tetrahydroisoquinoline compound and a pharmaceutical use thereof. More particularly, the present invention relates to a tetrahydroisoquinoline compound having an acyl coenzyme A: cholesterolacyltransferase (hereinafter to be also referred to as "ACAT")-inhibitory action, and further, a lipid peroxidation-inhibitory action, a pharmaceutically acceptable salt thereof, and a pharmaceutical agent comprising the compound or the pharmaceutically acceptable salt thereof.

### Background Art

It is well known that arteriosclerosis is extremely important as a factor of various circulatory diseases, and active studies aiming at suppression of progression of arteriosclerosis or regression thereof have been made.

In recent years, it has been clarified that accumulation of blood cholesterol as cholesterol ester in arterial walls is an important factor for the progression of arteriosclerosis. Accordingly, reduction of blood cholesterol level decreases accumulation of cholesteryl ester in arterial walls, and is effective for the suppression of progression and regression of arteriosclerosis. As a pharmaceutical agent for lowering the blood cholesterol level, cholesterol synthesis inhibitors, bile acid absorption inhibitors and the like are used. While the effectiveness thereof is acknowledged, an ideal pharmaceutical agent with a clear clinical effect and a fewer side effects has not been realized.

The cholesterol in foods is esterified in the mucous membrane of small intestine, absorbed as chylomicron and moves into the blood. It is known that ACAT plays an important role in the production of cholesterol ester in the mucous membrane of small intestine. In addition, cholesterol synthesized in the liver is esterified by ACAT and secreted in the blood as very low density lipoprotein (VLDL). Accordingly, it is considered that the blood cholesterol level can be decreased by suppressing the esterification of cholesterol by inhibiting the ACAT in the mucous membrane of the small intestine and the liver.

As a pharmaceutical agent for effectively preventing or treating arteriosclerosis, a pharmaceutical agent that more directly inhibits cholesterol deposition in the arterial wall is expected and actively studied. However, an ideal pharmaceutical agent has not been realized yet. In the arterial wall, cholesterol is esterified by ACAT in macrophage and smooth muscle cells, and accumulated as cholesterol ester. Therefore, accumulation of cholesterol ester is expected to be effectively suppressed by inhibiting ACAT in the arterial wall.

From the foregoing, an ACAT inhibitor is considered to be an effective pharmaceutical agent for hyperlipidemia, arteriosclerosis and the like since it suppresses cholesterol absorption in the small intestine, secretion of cholesterol from the liver and accumulation of cholesterol in the arterial wall.

Conventionally, as such ACAT inhibitor, for example, amide and urea derivatives have been reported (JP-A-2-117651, JP-A-3-7259, JP-A-4-234839, JP-A-4-327564, JP-A-5-32666, Vern G. DeVries et al., "Journal of Medicinal Chemistry", 1986, vol. 29, page 1131 etc.). However, the development and pharmacological study of these compounds are not entirely sufficient. First of all, whether or not the blood cholesterol-lowering action and arterial wall cholesterol accumulation-suppressing action due to the ACAT inhibitory action of these compounds is sufficiently effective for suppression of progression and regression of arteriosclerosis in clinical situations is not clear. In addition, most of the conventional ACAT inhibitors show low oral absorbability because they have extremely high liposolubility, and those having good oral absorbability are feared to cause organ disorders in adrenal gland, liver and the like. Moreover, a high-liposoluble and low-absorbable ACAT inhibitor may cause diarrhea in clinical situations.

On the other hand, denaturation by peroxidation of low density lipoprotein (LDL) plays an important role in the intracellular uptake of cholesterol accumulated as cholesterol ester in the arterial wall. Moreover, peroxidation of lipid in living organisms is known to be deeply involved in the onset of arteriosclerosis and ischemic disease in the cerebrovascular system and cardiovascular system.

Accordingly, a compound having both an ACAT inhibitory action and a lipid peroxidation inhibitory action more effectively and certainly decreases accumulation of cholesterol ester in the arterial wall and inhibits peroxidation of lipid in vivo, can prevent or treat various vascular pathologies caused thereby, and therefore, is highly useful as a pharmaceutical product.

### Disclosure of the Invention

The present invention aims at provision of a compound having a superior ACAT inhibitory action, and a pharmaceutical use thereof.

The present inventors have conducted intensive studies in an attempt to solve the above-mentioned problems and found that a compound having a tetrahydroisoquinoline skeleton has not only a strong ACAT inhibitory action but also superior oral absorbability, as well as a strong anti-hyperlipidemic action and an anti-arteriosclerotic action, which resulted in the completion of the present invention.

Accordingly, the present invention provides the following.
[1] A compound represented by the formula (I) 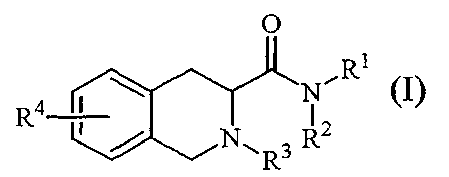 wherein
   R¹ and R² are the same or different and each is a hydrogen atom, an alkyl group, a cycloalkyl group, an optionally substituted aryl group, or an optionally substituted heterocycle, or, R¹ and R² form, together with the nitrogen atom bonded thereto, a heterocycle,
   R³ is an optionally substituted alkyl group, an optionally substituted carbamoyl group, an optionally substituted alkylcarbonyl group optionally having a double bond, an arylcarbonyl group, an alkylsulfonyl group, an alkylcarbonylcarbonyl group, or an optionally substituted aminosulfonyl group, and
   R⁴ is a hydrogen atom, a hydroxyl group, an optionally substituted alkoxy group, an optionally substituted amino group, an optionally substituted heterocycle, an optionally substituted aminoalkyl group, an optionally esterified carboxy group, an optionally esterified carboxyalkyl group, an optionally substituted heterocyclyl-alkyl group, a hydroxyalkyl group, or an alkoxyalkyl group,
      or a pharmaceutically acceptable salt thereof.
[2] The compound described in the above-mentioned [1], which is represented by the formula (Ia) 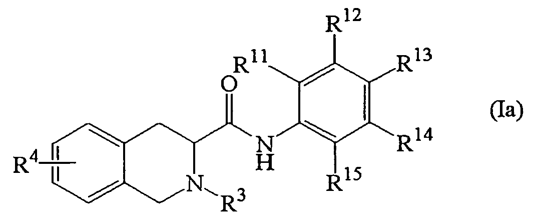 wherein
   R¹¹, R¹², R¹³, R¹⁴ and R¹⁵ are each independently a hydrogen atom, a halogen atom, an alkyl group, a hydroxyl group, an optionally substituted amino group, an alkoxy group, an optionally esterified carboxy group, an alkanoyloxy group, an optionally esterified carboxyalkyl group, an aminosulfonyl group or an optionally substituted carbamoyl group, and
   R³ and R⁴ are as defined in the formula (I), or a pharmaceutically acceptable salt thereof.
[3] The compound described in the above-mentioned [2], wherein R³ is an optionally substituted alkyl group, or an optionally substituted alkylcarbonyl group optionally having a double bond, and
   R⁴ is a hydrogen atom, a hydroxyl group or a group represented by 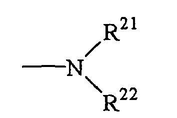 wherein R²¹ and R²² are the same or different and each is a hydrogen atom, an alkyl group or an alkylsulfonyl group, or,
   R²¹ and R²² form, together with the nitrogen atom bonded thereto, an optionally substituted heterocycle optionally containing hetero atom(s) besides the nitrogen atom bonded to R²¹ and R²², or a pharmaceutically acceptable salt thereof.
[4] The compound described in the above-mentioned [3], wherein the compound is selected from the group consisting of
   (1) N-(2,6-diisopropylphenyl)-2-(2,2-dimethylpropionyl)-1,2,3,4-tetrahydroisoquinoline-(3S)-carboxamide
   (2) N-(2,6-diisopropylphenyl)-2-octanoyl-1,2,3,4-tetrahydroisoquinoline-(3S)-carboxamide
   (3) N-(2,4-difluorophenyl)-2-octanoyl-1,2,3,4-tetrahydroisoquinoline-(3S)-carboxamide
   (4) 2-octanoyl-N-(2,4,6-trimethylphenyl)-1,2,3,4-tetrahydroisoquinoline-(3S)-carboxamide
   (5) 2-(2,2-dimethylhexanoyl)-N-(2,4,6-trimethylphenyl)-1,2,3,4-tetrahydroisoquinoline-(3S)-carboxamide
   (6) 2-hexanoyl-N-(2,4,6-trimethylphenyl)-1,2,3,4-tetrahydroisoquinoline-(3S)-carboxamide
   (7) 2-hexanoyl-N-(3-methanesulfonylamino-2,4,6-trimethylphenyl)-1,2,3,4-tetrahydroisoquinoline-(3S)-carboxamide
   (8) 2-(2,2-dimethylpropionyl)-N-(3-methanesulfonylamino-2,4,6-trimethylphenyl)-1,2,3,4-tetrahydroisoquinoline-(3S)-carboxamide
   (9) 2-[(2E,4E)-hexadienoyl]-N-(3-methanesulfonylamino-2,4,6-trimethylphenyl)-1,2,3,4-tetrahydroisoquinoline-(3S)-carboxamide
   (10) 2-(3,3-dimethylbutyryl)-N-(3-methanesulfonylamino-2,4,6-trimethylphenyl)-1,2,3,4-tetrahydroisoquinoline-(3S)-carboxamide
   (11) 2-(2,2-dimethylbutyryl)-N-(3-methanesulfonylamino-2,4,6-trimethylphenyl)-1,2,3,4-tetrahydroisoquinoline-(3S)-carboxamide
   (12) 2-[(2E,4E)-hexadienoyl]-N-(2,4,6-trimethylphenyl)-1,2,3,4-tetrahydroisoquinoline-(3S)-carboxamide
   (13) 2-octanoyl-N-(2,4,6-trimethylphenyl)-1,2,3,4-tetrahydroisoquinoline-(3R)-carboxamide
   (14) N-(4-amino-2,6-diisopropylphenyl)-2-octanoyl-1,2,3,4-tetrahydroisoquinoline-(3S)-carboxamide
   (15) 2-(2,2-dimethylbutyryl)-N-(2-isopropyl-5-methanesulfonylaminophenyl)-1,2,3,4-tetrahydroisoquinoline-(3S)-carboxamide
   (16) 7-dimethylamino-2-octanoyl-N-(2,4,6-trimethylphenyl)-1,2,3,4-tetrahydroisoquinoline-(3S)-carboxamide
   (17) 7-dimethylamino-2-(2,2-dimethylpropionyl)-N-(3-methanesulfonylamino-2,4,6-trimethylphenyl)-1,2,3,4-tetrahydroisoquinoline-(3S)-carboxamide
   (18) 7-dimethylamino-2-(2,2-dimethylpropionyl)-N-(2,4,6-trimethylphenyl)-1,2,3,4-tetrahydroisoquinoline-(3S)-carboxamide
   (19) N-(2,6-diisopropylphenyl)-7-hydroxy-2-octanoyl-1,2,3,4-tetrahydroisoquinoline-(3S)-carboxamide
   (20) N-(2,4-difluorophenyl)-7-hydroxy-2-octanoyl-1,2,3,4-tetrahydroisoquinoline-(3S)-carboxamide
   (21) N-(2,6-diisopropylphenyl)-2-(2,2-dimethylpropionyl)-7-hydroxy-1,2,3,4-tetrahydroisoquinoline-(3S)-carboxamide
   (22) N-(4-amino-2,6-diisopropylphenyl)-2-hexanoyl-7-hydroxy-1,2,3,4-tetrahydroisoquinoline-(3S)-carboxamide and
   (23) N-(2,6-diisopropylphenyl)-2-(2,2-dimethylpropyl)-1,2,3,4-tetrahydroisoquinoline-(3S)-carboxamide, or a pharmaceutically acceptable salt thereof.
[5] The compound described in the above-mentioned [2], wherein either of R¹³ and R¹⁴ is a hydroxyl group and the other is a hydrogen atom, an alkyl group or a halogen atom, R¹¹, R¹² and R¹⁵ are each independently a hydrogen atom, an alkyl group or a halogen atom,
   or a pharmaceutically acceptable salt thereof.
[6] The compound described in the above-mentioned [5], wherein R³ is an optionally substituted alkyl group, or an optionally substituted alkylcarbonyl group optionally having a double bond, and R¹³ is a hydroxyl group,
   or a pharmaceutically acceptable salt thereof.
[7] The compound described in the above-mentioned [6], wherein R⁹ is a hydrogen atom, hydroxyl group, or an optionally substituted alkoxy group,
   or a pharmaceutically acceptable salt thereof.
[8] The compound described in the above-mentioned [7], wherein the compound is selected from the group consisting of
   (1) 2-(2,2-dimethylpropyl)-N-(4-hydroxy-2,3,5-trimethylphenyl)-1,2,3,4-tetrahydroisoquinoline-(3S)-carboxamide
   (2) N-(4-hydroxy-2,3,5-trimethylphenyl)-2-octanoyl-1,2,3,4-tetrahydroisoquinoline-(3S)-carboxamide
   (3) N-(4-hydroxy-2,3,5-trimethylphenyl)-2-isobutyryl-1,2,3,4-tetrahydroisoquinoline-(3S)-carboxamide
   (4) N-(4-hydroxy-2,3,5-trimethylphenyl)-2-pentyl-1,2,3,4-tetrahydroisoquinoline-(3S)-carboxamide
   (5) 2-hexanoyl-7-hydroxy-N-(4-hydroxy-2,3,5-trimethylphenyl)-1,2,3,4-tetrahydroisoquinoline-(3S)-carboxamide
   (6) 7-(2-aminoethoxy)-2-hexanoyl-N-(4-hydroxy-2,3,5-trimethylphenyl)-1,2,3,4-tetrahydroisoquinoline-(3S)-carboxamide
   (7) 2-hexanoyl-N-(4-hydroxy-2,3,5-trimethylphenyl)-7-[2-(pyrrolidin-1-yl)ethoxy]-1,2,3,4-tetrahydroisoquinoline-(3S)-carboxamide
   (8) 2-hexanoyl-7-(3-hydroxypropoxy)-N-(4-hydroxy-2,3,5-trimethylphenyl)-1,2,3,4-tetrahydroisoquinoline-(3S)-carboxamide and
   (9) 2-{[2-(2,2-dimethylnonyl)-(3S)-(4-hydroxy-2,3,5-trimethylphenyl)-1,2,3,4-tetrahydroisoquinolin-7-yl]oxy}-2-methylpropionic acid, or a pharmaceutically acceptable salt thereof.
[9] The compound described in the above-mentioned [6], wherein R⁴ is a group represented by 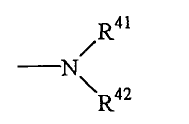 wherein R⁴¹ and R⁴² are the same or different and each is a hydrogen atom, an alkyl group or an alkylsulfonyl group, or R⁴¹ and R⁴² form, together with the nitrogen atom bonded thereto, an optionally substituted heterocycle optionally containing hetero atom(s) besides the nitrogen atom bonded to R⁴¹ and R⁴², or a pharmaceutically acceptable salt thereof.
[10] The compound described in the above-mentioned [9], which is selected from the group consisting of
   (1) 7-dimethylamino-2-(2,2-dimethylbutyryl)-N-(4-hydroxy-2,3,5-trimethylphenyl)-1,2,3,4-tetrahydroisoquinoline-(3S)-carboxamide
   (2) 7-dimethylamino-2-[(2E,4E)-hexadienoyl]-N-(4-hydroxy-2,3,5-trimethylphenyl)-1,2,3,4-tetrahydroisoquinoline-(3S)-carboxamide
   (3) 2-butyryl-7-dimethylamino-N-(4-hydroxy-2,3,5-trimethylphenyl)-1,2,3,4-tetrahydroisoquinoline-(3S)-carboxamide
   (4) 7-dimethylamino-N-(4-hydroxy-2,3,5-trimethylphenyl)-2-isobutyryl-1,2,3,4-tetrahydroisoquinoline-(3S)-carboxamide
   (5) 7-dimethylamino-2-(2,2-dimethylpropionyl)-N-(4-hydroxy-2,3,5-trimethylphenyl)-1,2,3,4-tetrahydroisoquinoline-(3S)-carboxamide
   (6) 7-dimethylamino-2-hexanoyl-N-(4-hydroxy-2,3,5-trimethylphenyl)-1,2,3,4-tetrahydroisoquinoline-(3R)-carboxamide
   (7) 7-dimethylamino-2-(2,2-dimethylbutyryl)-N-(4-hydroxy-2,3,5-trimethylphenyl)-1,2,3,4-tetrahydroisoquinoline-(3R)-carboxamide
   (8) 7-amino-2-hexanoyl-N-(4-hydroxy-2,3,5-trimethylphenyl)-1,2,3,4-tetrahydroisoquinoline-(3S)-carboxamide
   (9) 7-dimethylamino-2-hexanoyl-N-(4-hydroxy-2,3,5-trimethylphenyl)-1,2,3,4-tetrahydroisoquinoline-(3S)-carboxamide
   (10) 7-dimethylamino-N-(4-hydroxy-2,3,5-trimethylphenyl)-2-propyl-1,2,3,4-tetrahydroisoquinoline-(3S)-carboxamide
   (11) 7-amino-2-(2,2-dimethylpropionyl)-N-(4-hydroxy-2,3,5-trimethylphenyl)-1,2,3,4-tetrahydroisoquinoline-(3S)-carboxamide
   (12) 2-(2,2-dimethylpropionyl)-N-(4-hydroxy-2,3,5-trimethylphenyl)-7-methanesulfonylamino-1,2,3,4-tetrahydroisoquinoline-(3S)-carboxamide
   (13) N-(4-hydroxy-2,3,5-trimethylphenyl)-2-pentanoyl-7-(pyrrolidin-1-yl)-1,2,3,4-tetrahydroisoquinoline-(3S)-carboxamide
   (14) N-(4-hydroxy-2,3,5-trimethylphenyl)-2-propionyl-7-(pyrrolidin-1-yl)-1,2,3,4-tetrahydroisoquinoline-(3S)-carboxamide
   (15) 2-acetyl-N-(4-hydroxy-2,3,5-trimethylphenyl)-7-(pyrrolidin-1-yl)-1,2,3,4-tetrahydroisoquinoline-(3S)-carboxamide
   (16) N-(4-hydroxy-2,3,5-trimethylphenyl)-2-isobutyryl-7-(pyrrolidin-1-yl)-1,2,3,4-tetrahydroisoquinoline-(3S)-carboxamide
   (17) 2-hexanoyl-N-(4-hydroxy-2,3,5-trimethylphenyl)-7-methanesulfonylamino-1,2,3,4-tetrahydroisoquinoline-(3S)-carboxamide
   (18) 7-amino-N-(4-hydroxy-2,3,5-trimethylphenyl)-2-[5-methyl-(2E)-hexenoyl]-1,2,3,4-tetrahydroisoquinoline-(3S)-carboxamide
   (19) 7-amino-2-(5-hexenoyl)-N-(4-hydroxy-2,3,5-trimethylphenyl)-1,2,3,4-tetrahydroisoquinoline-(3S)-carboxamide
   (20) 7-amino-N-(4-hydroxy-2,3,5-trimethylphenyl)-2-(4-methylpentanoyl)-1,2,3,4-tetrahydroisoquinoline-(3S)-carboxamide
   (21) 7-amino-N-(4-hydroxy-2,3,5-trimethylphenyl)-2-pentanoyl-1,2,3,4-tetrahydroisoquinoline-(3S)-carboxamide
   (22) 7-amino-2-(2,2-dimethylbutyryl)-N-(4-hydroxy-2,3,5-trimethylphenyl)-1,2,3,4-tetrahydroisoquinoline-(3S)-carboxamide
   (23) 7-amino-2-(3,3-dimethylbutyryl)-N-(4-hydroxy-2,3,5-trimethylphenyl)-1,2,3,4-tetrahydroisoquinoline-(3S)-carboxamide
   (24) 2-(3,3-dimethylbutyryl)-N-(4-hydroxy-2,3,5-trimethylphenyl)-7-methanesulfonylamino-1,2,3,4-tetrahydroisoquinoline-(3S)-carboxamide
   (25) 2-hexanoyl-N-(4-hydroxy-2,3,5-trimethylphenyl)-7-(piperazin-1-yl)-1,2,3,4-tetrahydroisoquinoline-(3S)-carboxamide and
   (26) 2-(2,2-dimethylpropionyl)-N-(4-hydroxy-2,3,5-trimethylphenyl)-7-(4-methylpiperazin-1-yl)-1,2,3,4-tetrahydroisoquinoline-(3S)-carboxamide,
      or a pharmaceutically acceptable salt thereof.
[11] The compound described in the above-mentioned [6], wherein R⁴ is a group represented by 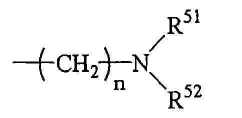 wherein n is an integer of 1 to 3, R⁵¹ and R⁵² are the same or different and each is a hydrogen atom or an optionally substituted alkyl group, or R⁵¹ and R⁵² form, together with the nitrogen atom bonded thereto, an optionally substituted heterocycle optionally containing hetero atom(s) besides the nitrogen atom bonded to R⁵¹ and R⁵²,
   or a pharmaceutically acceptable salt thereof.
[12] The compound described in the above-mentioned [11], which is selected from the group consisting of
   (1) 7-dimethylaminomethyl-2-(2,2-dimethylbutyryl)-N-(4-hydroxy-2,3,5-trimethylphenyl)-1,2,3,4-tetrahydroisoquinoline-(3S)-carboxamide
   (2) 7-diethylaminomethyl-2-(2,2-dimethylpropionyl)-N-(4-hydroxy-2,3,5-trimethylphenyl)-1,2,3,4-tetrahydroisoquinoline-(3S)-carboxamide
   (3) 7-diisopropylaminomethyl-N-(4-hydroxy-2,3,5-trimethylphenyl)-2-isobutyryl-1,2,3,4-tetrahydroisoquinoline-(3S)-carboxamide
   (4) 2-(2,2-dimethylpropionyl)-N-(4-hydroxy-2,3,5-trimethylphenyl)-7-(N-methylpropylamino)methyl-1,2,3,4-tetrahydroisoquinoline-(3S)-carboxamide
   (5) N-(4-hydroxy-2,3,5-trimethylphenyl)-2-isobutyryl-7-(N-methylpropylamino)methyl-1,2,3,4-tetrahydroisoquinoline-(3S)-carboxamide
   (6) 2-(2,2-dimethylpropionyl)-7-(2-fluoro-N-methylethylamino)methyl-N-(4-hydroxy-2,3,5-trimethylphenyl)-1,2,3,4-tetrahydroisoquinoline-(3S)-carboxamide
   (7) 2-(2,2-dimethylpropionyl)-7-(2-fluoroethylamino)methyl-N-(4-hydroxy-2,3,5-trimethylphenyl)-1,2,3,4-tetrahydroisoquinoline-(3S)-carboxamide
   (8) 2-(2,2-dimethylpropionyl)-7-(3-fluoroazetidin-1-yl)methyl-N-(4-hydroxy-2,3,5-trimethylphenyl)-1,2,3,4-tetrahydroisoquinoline-(3S)-carboxamide and
   (9) 2-(2,2-dimethylpropionyl)-N-(4-hydroxy-2,3,5-trimethylphenyl)-7-(3-methoxyazetidin-1-yl)methyl-1,2,3,4-tetrahydroisoquinoline-(3S)-carboxamide,
      or a pharmaceutically acceptable salt thereof.
[13] A pharmaceutical composition comprising a compound described in any of the above-mentioned [1] - [12] or a pharmaceutically acceptable salt thereof.
[14] An antioxidant comprising a compound described in any of the above-mentioned [6] - [12] or a pharmaceutically acceptable salt thereof.
[15] An acyl-Coenzyme A: cholesterol acyltransferase inhibitor comprising a 1,2,3,4-tetrahydroisoquinoline compound having an optionally substituted carbamoyl group at the 3-position, or a pharmaceutically acceptable salt thereof.
[16] The inhibitor of the above-mentioned [15], wherein the 2-position of the 1,2,3,4-tetrahydroisoquinoline compound is substituted by a group represented by R³ in the above-mentioned [1].
[17] The inhibitor of the above-mentioned [15], wherein the 1,2,3,4-tetrahydroisoquinoline compound is any of the compounds of the above-mentioned [1] - [12].
[18] An agent for the prophylaxis or treatment of hyperlipidemia, arteriosclerosis or inflammatory disease, which comprises any of the compounds of the above-mentioned [1] - [12], or a pharmaceutically acceptable salt thereof.
[19] A method of preventing or treating hyperlipidemia, arteriosclerosis or inflammatory disease, which comprises administering an effective amount of any of the compounds of the above-mentioned [1] - [12] or a pharmaceutically acceptable salt thereof.
[20] Use of any of the compounds of the above-mentioned [1] - [12] or a pharmaceutically acceptable salt thereof, for the production of a pharmaceutical agent for the prophylaxis or treatment of hyperlipidemia, arteriosclerosis or inflammatory disease.
[21] A commercial package comprising an agent for the prophylaxis or treatment of hyperlipidemia, arteriosclerosis or inflammatory disease, which comprises any of the compounds of the above-mentioned [1] - [12] or a pharmaceutically acceptable salt thereof, and a written matter stating that the agent can or should be used for the prophylaxis or treatment of hyperlipidemia, arteriosclerosis or inflammatory disease.

### Detailed Description of the Invention

A compound having a 1,2,3,4-tetrahydroisoquinoline skeleton to be used in the present invention (to be also simply referred to as "1,2,3,4-tetrahydroisoquinoline compound of the present invention" in the present specification) is not particularly limited but a compound having an optionally substituted carbamoyl group at the 3-position of the tetrahydroisoquinoline skeleton is preferable.

Since the 1,2,3,4-tetrahydroisoquinoline compound and a pharmaceutically acceptable salt thereof of the present invention have a superior ACAT inhibitory activity, they are useful as ACAT inhibitors.

The 1,2,3,4-tetrahydroisoquinoline compound and a pharmaceutically acceptable salt thereof of the present invention can effectively prevent and/or treat hyperlipidemia, arteriosclerosis, inflammatory disease, angina pectoris, myocardial infarction, cerebral infarction, cerebral apoplexy, Alzheimer's disease, renal diseases, ophthalmic diseases and the like based on the ACAT inhibitory activity. Of the compounds of the present invention, the compound of the below-mentioned formula (Ia') is also useful as an antioxidant. The antioxidant can be used for the prophylaxis and/or treatment of the aforementioned diseases.

The 1,2,3,4-tetrahydroisoquinoline compound of the present invention preferably has the structure of the formula (I)

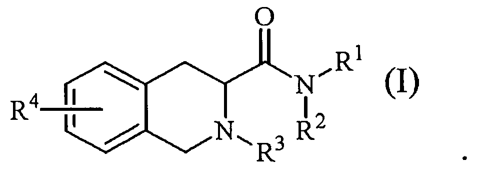

The definition of each symbol in the formula (I) is as follows.

R¹ and R² are the same or different and each is a hydrogen atom, an alkyl group, a cycloalkyl group, an optionally substituted aryl group, or an optionally substituted heterocycle, or R¹ and R² form, together with the nitrogen atom bonded thereto, a heterocycle.

The alkyl group for R¹ or R² is a straight chain or branched chain alkyl group having 1 to 6 carbon atoms, with preference given to a pentyl group.

The cycloalkyl group for R¹ or R² is a cyclic alkyl group having 3 to 6 carbon atoms, with preference given to a cyclopropyl group and a cyclohexyl group.

As the aryl group of the optionally substituted aryl group for R¹ or R², a phenyl group, a naphthyl group and the like can be mentioned, with preference given to a phenyl group.

As the substituent of the optionally substituted aryl group for R¹ or R², halogen atom (preferably, fluorine), hydroxyl group, a straight chain or branched chain alkyl group having 1 to 6 carbon atoms (preferably, methyl group, isopropyl group, tert-butyl group), an optionally substituted amino group (preferably, amino group), an alkoxy group (preferably, methoxy group), an alkanoyloxy group (preferably, acetoxy group), an optionally esterified carboxy group (preferably, carboxy group), an optionally esterified carboxyalkyl group (preferably, carboxymethyl group), an alkylsulfonylamino group (preferably, methylsulfonylamino group), an aminosulfonylamino group, an aminosulfonyl group, an optionally substituted carbamoyl group (preferably, N,N-dimethylcarbamoyl group) and the like can be mentioned.

As the ester moiety when an optionally esterified carboxy group and an optionally esterified carboxyalkyl group are esterified, a straight chain or branched chain alkyl ester having 1 to 6 carbon atoms (e.g., methyl, ethyl, isopropyl, tert-butyl), a cyclic alkyl ester having 3 to 7 carbon atoms (e.g., cyclopropyl, cyclohexyl), aryl (e.g., phenyl, p-nitrophenyl, p-methoxyphenyl), arylalkyl (e.g., benzyl, benzhydryl, trityl) and the like can be mentioned.

As the substituent of the optionally substituted carbamoyl group, a straight chain or branched chain alkyl having 1 to 6 carbon atoms (e.g., methyl, ethyl, isopropyl, tert-butyl) can be mentioned.

As the substituent of the optionally substituted amino group, a straight chain or branched chain alkyl having 1 to 6 carbon atoms (e.g., methyl, ethyl, isopropyl, tert-butyl) can be mentioned.

The heterocycle of the optionally substituted heterocycle for R¹ or R² is a 3- to 6-membered heterocycle having one or more hetero atoms (e.g., nitrogen atom, oxygen atom, sulfur atom and the like) as a ring-constituting atom, where the bond forming the ring may be saturated or unsaturated, with preference given to pyridinine.

As the substituent of the heterocycle optionally having substituents for R¹ or R², a hydroxyl group, an alkyl group (preferably, methyl group, propyl group), an aryl group (preferably, phenyl group), an oxo group junctioned with a ring-constituting carbon atom and the like can be mentioned.

As the heterocycle formed by R¹ and R² together with the nitrogen atom bonded thereto is a 3- to 6-membered heterocycle containing, as a ring-constituting atom, one or more hetero atoms (e.g., nitrogen atom, oxygen atom, sulfur atom and the like), where the bond forming the ring may be saturated or unsaturated, with preference given to piperidine.

R³ is an optionally substituted alkyl group, an optionally substituted carbamoyl group, an optionally substituted alkylcarbonyl group optionally having a double bond, an arylcarbonyl group, an alkylsulfonyl group, an alkylcarbonylcarbonyl group, or an optionally substituted aminosulfonyl group.

The alkyl group for R³ is a straight chain or branched chain alkyl group having 1 to 8 carbon atoms. Preferred are methyl group, propyl group, n-pentyl group, n-octyl group and neopentyl group.

As the substituent of the optionally substituted alkyl group for R³, a halogen atom, a hydroxyl group, an alkoxy group and an optionally substituted aryl group (preferably, phenyl group) can be mentioned. Preferred are a hydroxyl group, an alkoxy group and an optionally substituted aryl group.

Here, as the substituent of the optionally substituted aryl group, an amino group can be mentioned.

As the substituent of the optionally substituted carbamoyl group for R³, an optionally substituted aryl group can be mentioned.

Here, as the substituent of the optionally substituted aryl group, a hydroxyl group, an alkyl group (preferably, methyl group, isopropyl group) and an amino group can be mentioned.

As the alkyl moiety of the optionally substituted alkylcarbonyl group optionally having a double bond for R³, a straight chain or branched chain alkyl having 1 to 10 carbon atoms can be mentioned, where "optionally having a double bond" means the alkyl moiety thereof may contain 1 to 3 C=C double bonds. Preferably, the alkyl moiety is methyl, ethyl, propyl, isopropyl, n-butyl, sec-butyl, tert-butyl, neopentyl, n-pentyl, n-heptyl, n-nonyl, 1,1-dimethylpropyl, 1,1-dimethylpentyl, isopentyl, 1,1-dimethyloctyl, 4-pentenyl, 4-methyl-1-pentenyl, 1,3-pentadienyl or 1,3-heptadienyl.

As the substituent of the optionally substituted alkylcarbonyl group optionally having a double bond for R³, an optionally substituted amino group (preferably, amino, dimethylamino) and an aryl group (preferably, phenyl) can be mentioned.

As the aryl moiety of the arylcarbonyl group for R³, phenyl and naphthyl can be mentioned, with preference given to phenyl.

As the alkyl moiety of the alkylsulfonyl group for R³, a straight chain or branched chain alkyl having 1 to 8 carbon atoms can be mentioned, with preference given to methyl and n-octyl.

The alkylcarbonyl of the alkylcarbonylcarbonyl group for R³ is the same as the aforementioned alkylcarbonyl for R³. Preferably, the alkylcarbonyl moiety is ethylcarbonyl.

As the substituent of the optionally substituted aminosulfonyl group for R³, a straight chain or branched chain alkanoyl group having 1 to 7 carbon atoms can be mentioned, with preference given to a pentanoyl group.

R⁴ is a hydrogen atom, a hydroxyl group, an optionally substituted alkoxy group, an optionally substituted amino group, an optionally substituted heterocycle, an optionally substituted aminoalkyl group, an optionally esterified carboxy group, an optionally esterified carboxyalkyl group, an optionally substituted heterocyclyl-alkyl group, a hydroxyalkyl group, or an alkoxyalkyl group.

The alkoxy group of the optionally substituted alkoxy group for R⁴ is a straight chain or branched chain alkoxy group having 1 to 6 carbon atoms. Preferred are a methoxy group, an ethoxy group, a propoxy group and an isopropoxy group.

As the substituent of the optionally substituted alkoxy group for R⁴, a hydroxyl group, a carboxy group, an amino group and a heterocycle can be mentioned.

Here, the heterocycle is a 3- to 6-membered heterocycle containing, as a ring-constituting atom, one or more hetero atoms (e.g., nitrogen atom, oxygen atom, sulfur atom and the like), where the bond forming the ring may be saturated or unsaturated. Preferred is pyrrolidine.

As the substituent of the optionally substituted amino group for R⁴, an alkyl group (preferably, methyl group), an aminoalkylcarbonyl group (preferably, aminomethylcarbonyl group), an alkanoyl group (preferably, acetyl group), an alkylsulfonyl group (preferably, methylsulfonyl group), an alkaneimidoyl group (preferably, acetoimidoyl group) and an optionally substituted aminosulfonyl group (preferably, acetylaminosulfonyl group) can be mentioned.

The heterocycle of the optionally substituted heterocycle for R⁴ is a 3- to 6-membered heterocycle containing, as a ring-constituting atom, one or more hetero atoms (e.g., nitrogen atom, oxygen atom, sulfur atom and the like), where the bond forming the ring may be saturated or unsaturated. Preferred are pyrrolidine, morpholine and piperazine.

As the substituent of the optionally substituted heterocycle for R⁴, an alkyl group (preferably, methyl group) can be mentioned.

The alkyl group of the optionally substituted aminoalkyl group for R⁴ is an alkyl group having 1 to 6 carbon atoms, with preference given to a methyl group.

As the substituent of the optionally substituted aminoalkyl group for R⁴, an optionally substituted alkyl group can be mentioned. Preferred are a methyl group, an ethyl group, a propyl group, an isopropyl group, a halogenated ethyl group (preferably, ethyl fluoride group) and an alkoxyalkyl group (preferably, methoxyethyl group).

As the alkyl moiety of the optionally esterified carboxyalkyl group for R⁴, a straight chain or branched chain alkyl having 1 to 6 carbon atoms can be mentioned, with preference given to ethyl.

The ester moiety when the optionally esterified carboxyalkyl group for R⁴ is esterified is as defined for R¹ and R² in the formula (I).

As the alkyl moiety to which heterocycle is bonded in the optionally substituted heterocyclyl-alkyl group for R⁴, a straight chain or branched chain alkyl having 1 to 6 carbon atoms can be mentioned, with preference given to methyl.

As the heterocycle of the optionally substituted heterocyclyl-alkyl group for R⁴, 3- to 6-membered heterocycle containing, as a ring-constituting atom, one or more hetero atoms (e.g., nitrogen atom, oxygen atom, sulfur atom and the like), where the bond forming the ring may be saturated or unsaturated, can be mentioned. Preferred are pyrrolidine and azetidine.

As the substituent of the optionally substituted heterocyclyl-alkyl group for R⁴, a hydroxyl group, a halogen atom and an alkoxy group can be mentioned. Preferred are a hydroxyl group, a fluorine atom and a methoxy group.

As the alkyl moiety of the hydroxyalkyl group for R⁴, a straight chain or branched chain alkyl having 1 to 6 carbon atoms can be mentioned, with preference given to methyl.

As the alkoxyalkyl moiety of the hydroxyalkyl group for R⁴, straight chain or branched chain alkoxy having 1 to 6 carbon atoms can be mentioned. As the alkyl moiety, a straight chain or branched chain alkyl having 1 to 6 carbon atoms can be mentioned.

Of the above-mentioned compounds represented by the formula (I), a compound represented by the formula (Ia) 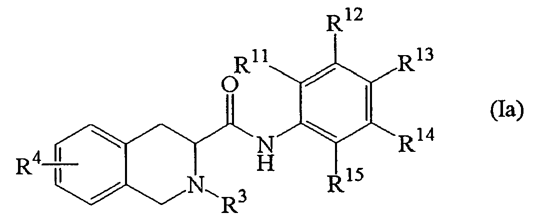 wherein
R¹¹, R¹², R¹³, R¹⁴ and R¹⁵ are each independently a hydrogen atom, a halogen atom, an alkyl group, a hydroxyl group, an optionally substituted amino group, an alkoxy group, an optionally esterified carboxy group, an alkanoyloxy group, an optionally esterified carboxyalkyl group, an aminosulfonyl group, or an optionally substituted carbamoyl group, and
R³ and R⁴ are as defined in the formula (I), and a pharmaceutically acceptable salt thereof are more preferable.

The halogen atom for R¹¹, R¹², R¹³, R¹⁴ or R¹⁵ is fluorine, chlorine, bromine and the like. Preferred is fluorine.

The alkyl group for R¹¹, R¹², R¹³, R¹⁴ or R¹⁵ is a straight chain or branched chain alkyl group having 1 to 6 carbon atoms. Preferred are a methyl group, an isopropyl group and a tert-butyl group.

As the substituent of the optionally substituted amino group for R¹¹, R¹², R¹³, R¹⁴ or R¹⁵, an alkylsulfonyl group, an aminosulfonyl group and the like can be mentioned. Preferred are a methylsulfonyl group and an aminosulfonyl group.

The alkoxy group for R¹¹, R¹², R¹³, R¹⁴ or R¹⁵ is a straight chain or branched chain alkoxy group having 1 to 6 carbon atoms. Preferred is a methoxy group.

The alkanoyl moiety of the alkanoyloxy group for R¹¹, R¹², R¹³, R¹⁴ or R¹⁵ is alkanoyl having a straight chain or branched chain alkyl moiety having 1 to 6 carbon atoms. Preferred is acetyl.

The carboxyalkyl group for R¹¹, R¹², R¹³, R¹⁴ or R¹⁵ is a straight chain or branched chain alkyl group having 1 to 6 carbon atoms, which is substituted by carboxy. Preferred is a carboxymethyl group.

As the substituent of the optionally substituted carbamoyl group for R¹¹, R¹², R¹³, R¹⁴ or R¹⁵, a straight chain or branched chain alkyl group having 1 to 6 carbon atoms can be mentioned. As the optionally substituted carbamoyl group, N,N-dimethylcarbamoyl is preferable.

The ester moiety when the optionally esterified carboxy group or the optionally esterified carboxyalkyl group for R¹¹, R¹², R¹³, R¹⁴ or R¹⁵ is esterified is as defined for R¹ or R² in the formula (I).

Of the compounds represented by the above-mentioned formula (Ia), a compound wherein R³ is an optionally substituted alkyl group or an optionally substituted alkylcarbonyl group optionally having a double bond,
R⁴ is a hydrogen atom, a hydroxyl group or a group represented by 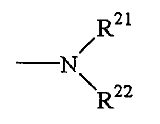 wherein R²¹ and R²² are the same or different and each is a hydrogen atom, an alkyl group or an alkylsulfonyl group, or R²¹ and R²² form, together with the nitrogen atom bonded thereto, an optionally substituted heterocycle optionally containing a hetero atom besides the nitrogen atom bonded to R²¹ and R²², or a pharmaceutically acceptable salt thereof is preferable.

The alkyl group for R²¹ or R²² is a straight chain or branched chain alkyl group having 1 to 6 carbon atoms. Preferred is a methyl group.

The alkylsulfonyl group for R²¹ or R²² is an alkylsulfonyl group having a straight chain or branched chain alkyl moiety having 1 to 6 carbon atoms. Preferred is a methylsulfonyl group.

As the optionally substituted heterocycle formed R²¹ and R²², together with the nitrogen atom bonded thereto, which optionally contains hetero atom(s) besides the nitrogen atom bonded to R²¹ and R²², a 3- to 6-membered heterocycle optionally containing, besides the nitrogen atom of the binding site, one or more hetero atom (e.g., nitrogen atom, oxygen atom, sulfur atom and the like) as a ring-constituting atom, can be mentioned. The bond forming the ring may be saturated or unsaturated. Preferred are pyrrolidine, morpholine and piperazine.

As the substituent of the optionally substituted heterocycle, a straight chain or branched chain alkyl group having 1 to 6 carbon atoms can be mentioned. Preferred is a methyl group.

Of the compounds represented by the above-mentioned formula (Ia), a compound wherein one of R¹³ and R¹⁴ is a hydroxyl group and the other is a hydrogen atom, an alkyl group or a halogen atom, R¹¹, R¹² and R¹⁵ are each independently a hydrogen atom, an alkyl group or a halogen atom, and a pharmaceutically acceptable salt thereof are more preferable.

Of the compounds represented by the above-mentioned formula (Ia), a compound wherein R³ is an optionally substituted alkyl group or an optionally substituted alkylcarbonyl group optionally having a double bond, R¹³ is a hydroxyl group, and R¹¹, R¹², R¹⁴ and R¹⁵ are each independently a hydrogen atom, an alkyl group or a halogen atom (hereinafter to be also referred to as the compound the formula (Ia')) and a pharmaceutically acceptable salt thereof are more preferable.

Of the compounds represented by the above-mentioned formula (Ia'), a compound wherein R⁴ is a hydrogen atom, a hydroxyl group, or an optionally substituted alkoxy group, and a pharmaceutically acceptable salt thereof are preferable.

Here, the optionally substituted alkoxy group for R⁴ is as defined above and preferably, a methoxy group, an ethoxy group, a propoxy group or an isopropoxy group.

The substituent of the optionally substituted alkoxy group for R⁴ is as defined above and preferably, a carboxy group, a hydroxyl group, an amino group or a pyrrolidinyl group.

Of the compounds represented by the above-mentioned formula (Ia'), a compound wherein R⁴ is a group represented by 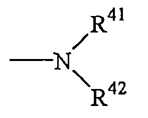 wherein R⁴¹ and R⁴² are the same or different and each is a hydrogen atom, an alkyl group or an alkylsulfonyl group, or R⁴¹ and R⁴² form, together with the nitrogen atom bonded thereto, an optionally substituted heterocycle optionally containing hetero atom(s) besides the nitrogen atom bonded to R⁴¹ and R⁴² and a pharmaceutically acceptable salt thereof are also preferable.

The alkyl group for R⁴¹ or R⁴² is a straight chain or branched chain alkyl group having 1 to 6 carbon atoms. Preferred is a methyl group.

The alkylsulfonyl group for R⁴¹ or R⁴² is an alkylsulfonyl group having a straight chain or branched chain alkyl moiety having 1 to 6 carbon atoms. Preferred is a methylsulfonyl group.

As the optionally substituted heterocycle formed R⁴¹ and R⁴², together with the nitrogen atom bonded thereto, which optionally contains hetero atom(s) besides the nitrogen atom bonded to R⁴¹ and R⁴², a 3- to 6-membered heterocycle optionally containing, besides the nitrogen atom of the binding site, one or more hetero atoms (e.g., nitrogen atom, oxygen atom, sulfur atom and the like) as a ring-constituting atom, can be mentioned. The bond forming the ring may be saturated or unsaturated. Preferred are pyrrolidine, morpholine and piperazine.

As the substituent of the optionally substituted heterocycle, a straight chain or branched chain alkyl group having 1 to 6 carbon atoms can be mentioned. Preferred is a methyl group.

Of the compounds represented by the above-mentioned formula (Ia'), a compound wherein R⁴ is a group represented by 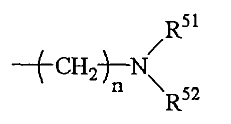 wherein n is an integer of 1 to 3, R⁵¹ and R⁵² are the same or different and each is a hydrogen atom or an optionally substituted alkyl group, or R⁵¹ and R⁵² form, together with the nitrogen atom bonded thereto, an optionally substituted heterocycle optionally containing hetero atom(s) besides the nitrogen atom bonded to R⁵¹ and R⁵², and a pharmaceutically acceptable salt thereof are also preferable.

The alkyl group of the optionally substituted alkyl group for R⁵¹ or R⁵² is a straight chain or branched chain alkyl group having 1 to 6 carbon atoms. Preferred are a methyl group, an ethyl group, a propyl group and an isopropyl group.

As the substituent of the optionally substituted alkyl group for R⁵¹ or R⁵², a halogen atom (fluorine, chlorine, bromine and the like) can be mentioned. Preferred is a fluorine atom.

As the optionally substituted heterocycle formed R⁵¹ and R⁵², together with the nitrogen atom bonded thereto, which optionally contains a hetero atom besides the nitrogen atom bonded to R⁵¹ and R⁵², a 3- to 6-membered heterocycle optionally containing, besides the nitrogen atom of the binding site, one or more hetero atoms (e.g., nitrogen atom, oxygen atom, sulfur atom and the like) as a ring-constituting atom, can be mentioned. The bond forming the ring may be saturated or unsaturated. Preferred are pyrrolidine and azetidine.

As the substituent of the optionally substituted heterocycle, a halogen atom, a hydroxyl group and a straight chain or branched chain alkoxy group having 1 to 6 carbon atoms can be mentioned. Preferred are a fluorine atom, a hydroxyl group and a methoxy group.

In the present specification, the pharmaceutically acceptable salt may be any as long as it is a pharmaceutically non-toxic and, for example, inorganic acid salts such as hydrochloride, hydrogen bromide, sulfate, nitrate, phosphate, carbonate and the like; organic acid salts such as formate, acetate, propionate, oxalate, glycolate, succinate, lactate, maleate, methylmaleate, fumarate, adipate, tartrate, malate, citrate, benzoate, cinnamate, ascorbate, salicylate, acetylsalicylate, nicotinate and the like; sulfonates such as methanesulfonate, ethanesulfonate, isethionate, benzenesulfonate, p-toluenesulfonate, naphthalenesulfonate and the like; acidic amino-acid salts such as aspartate, glutamate and the like; alkali metal salts such as sodium salt, potassium salt and the like; alkaline earth metal salts such as magnesium salt, calcium salt and the like; ammonium salts; organic base salts such as ethanolamine salt, diethylamine salt, triethanolamine salt, piperazine salt, N,N'-dibenzylethylenediamine salt and the like; amino-acid salts such as lysine salt, arginine salt and the like; and the like can be mentioned. Preferred are hydrochloride, sulfate, maleate, fumarate, tartrate, citrate, methanesulfonate, sodium salt, potassium salt and calcium salt.

In the following, the aforementioned 1,2,3,4-tetrahydroisoquinoline compound and a pharmaceutically acceptable salt thereof of the present invention are also collectively referred simply to the compound of the present invention.

The compound of the present invention may be appropriately mixed with a binder, an excipient, a lubricant and the like known in the field of drug making to give a pharmaceutical composition.

The subject of administration when the compound of the present invention is used as a pharmaceutical agent includes mammals (e.g., mouse, rat, hamster, rabbit, cat, dog, bovine, sheep, monkey, human and the like).

While the dose of the compound of the present invention for pharmaceutical use varies depending on the subject of administration, age, symptom, administration route and the like, for example, a dose of 0.1 - 50 mg/kg body weight can be administered 1 - 3 times a day for oral administration to an adult patient with hyperlipidemia or arteriosclerosis.

While the Production Methods of the present invention are explained in the following, they are examples and do not limit the Production Method of the present invention.

In the following explanation of the Production Methods, a lower alkyl group means a straight chain or branched chain alkyl group having 1 to 6 carbon atoms.

### (Production Method 1)

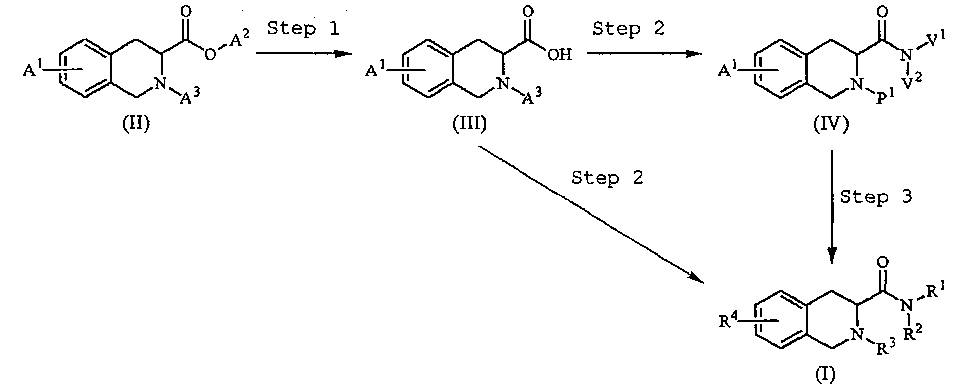

R¹, R², R³ and R⁴ in the above-mentioned formulas and the following description are as defined above.

In Production Method 1, a tetrahydroisoquinoline compound of the formula (I) is produced from a compound of the formula (II). This method also includes a production method comprising eliminating a protecting group (deprotection) from a compound represented by the formula (II), which has a conventional protecting group in a molecule, at a desired stage by a method known per se to give a compound of the formula (I).

As the above-mentioned conventional protecting group, an amino-protecting group, a hydroxyl-protecting group and a carboxy-protecting group can be mentioned.

As the amino-protecting group, formyl group, acetyl group, monochloroacetyl group, dichloroacetyl group, trichloroacetyl group, trifluoroacetyl group, methoxycarbonyl group, ethoxycarbonyl group, p-nitrobenzyloxycarbonyl group, diphenylmethyloxycarbonyl group, methoxymethyloxycarbonyl group, 2,2,2-trichloroethoxycarbonyl group, 2-methylsulfonylethyloxycarbonyl group, tert-butoxycarbonyl (Boc) group, benzyloxycarbonyl group, benzyl group, trimethylsilyl group, trityl group and the like can be mentioned. Preferably, trifluoroacetyl group, p-nitrobenzyloxycarbonyl group and benzyloxycarbonyl group can be mentioned.

As the hydroxyl-protecting group, acetyl group, benzyl group, trimethylsilyl group, tert-butyldimethylsilyl group, benzoyl group, formyl group, propionyl group, methoxymethyl group and the like can be mentioned. Preferably, acetyl group and benzyl group can be mentioned.

As the carboxy-protecting group, methyl group, ethyl group, propyl group, benzyl group, methoxyethyl group, isopropoxymethyl group, tert-butyl group, tert-butoxymethyl group, 2,2,2-trichloroethoxymethyl group, methylthiomethyl group and the like can be mentioned. Preferably methyl group, ethyl group, benzyl group and tert-butyl group can be mentioned.

As the above-mentioned deprotection means known *per* se, depending on the kind of the protecting group, decomposition with acid (Boc group for amino group, tert-butyl group for carboxy group etc.), decomposition with base (trifluoroacetyl group for amino group, acetyl group for hydroxyl group, methyl and ethylester group for carboxy group etc.), catalytic reduction (benzyloxycarbonyl group and p-nitrobenzyloxycarbonyl group for amino group, benzyl group for hydroxyl group, benzyl group for carboxy group etc.) can be mentioned.

As the acid to be used for decomposition with acid, hydrochloric acid, hydrobromic acid, hydroiodic acid, trifluoroacetic acid and the like can be mentioned.

The decomposition with acid is performed without solvent, or in a solvent that does not inhibit the reaction (methylene chloride, chloroform, methanol, ethanol, acetic acid, formic acid, water etc.) or a mixture thereof.

As the base to be used for decomposition with base, lithium hydroxide, potassium hydroxide, sodium hydroxide, potassium carbonate, sodium carbonate and the like can be mentioned.

The decomposition with base is performed in a water-containing solvent that does not inhibit the reaction (methanol, ethanol, isopropanol, tert-butanol, acetone, tetrahydrofuran, dioxane, ethylene glycol etc.) or a mixture thereof.

As the catalyst to be used for catalytic reduction, palladium carbon, palladium hydroxide carbon, palladium black and the like can be mentioned.

The decomposition by catalytic reduction is performed in a solvent that does not inhibit the reaction (water, methanol, ethanol, acetic acid, ethyl acetate, benzene, toluene, tetrahydrofuran, dioxane, ethylene glycol etc.) or a mixture thereof and, when desired, in the co-presence of an acid such as hydrochloric acid and the like.

V¹ and V² represent R¹ and R², or R¹ and R² protected by the above-mentioned conventional protecting group.

A¹ represents R⁴ or R⁴ protected by a conventional protecting group.

A² represents A⁵ (lower alkyl group such as methyl group, ethyl group and the like) or A⁶ (arylalkyl group such as benzyl group and the like), which is a conventional carboxy-protecting group.

A³ represents V³ (V³ represents R³, or R³ protected by a conventional protecting group) or P¹.

P¹ represents the above-mentioned conventional amino-protecting group.

A compound of the formula (II) wherein A¹ is a hydrogen atom can be produced using phenylalanine as a starting material by a method known per se [Organic Reactions, 6, 151-190 (1951)].

In step 1, a compound of the formula (III) is produced from a compound of the formula (II).

When A² is A⁵, the reaction is carried out using a base in water or a water-containing organic solvent. As the organic solvent, acetone, dioxane, tetrahydrofuran, methanol, ethanol, isopropanol, hexamethylphosphoric triamide (HMPA), dimethyl sulfoxide and the like, and a mixture thereof can be mentioned. The reaction is preferably carried out in a water-containing organic solvent, and the reaction is more preferably carried out in water-containing methanol, or a water-containing mixed solvent of tetrahydrofuran and methanol.

As the base, sodium hydroxide, potassium hydroxide, lithium hydroxide and the like can be mentioned. Preferred is lithium hydroxide.

The reaction temperature is preferably from 0°C to 50°C. The reaction time is generally from 30 min to 6 hr.

When A² is A⁶, a catalytic reduction [normal pressure - 0.3 MPa, in an inert solvent (methanol, ethanol etc.)] or catalytic hydrogen transfer reaction and the like (as the hydrogen source, formic acid, formate (lithium formate, ammonium formate etc.), cyclohexenone and the like can be mentioned) is performed in the presence of a catalyst (palladium carbon, palladium hydroxide carbon, palladium black etc.). A catalytic reduction using palladium carbon in methanol is preferable.

In step 2, a compound of the formula (IV) wherein A³ is P¹, or a compound of the formula (I) wherein A³ is V³ is produced from a compound of the formula (III).

The reaction is performed in a solvent that does not inhibit the reaction, for example, dioxane, acetonitrile, tetrahydrofuran, chloroform, methylene chloride, benzene, toluene, xylene, ethyl acetate, N,N-dimethylformamide, N,N-dimethylacetamide, dimethyl sulfoxide and the like, or a mixture thereof, in the presence of HNV¹V² and a dehydrating condensing agent and, when desired, in the co-presence of a base such as triethylamine, pyridine and the like. A preferable solvent is methylene chloride.

As the dehydrating condensing agent, dicyclohexylcarbodiimide, 1-ethyl-3-(3-dimethylaminopropyl)carbodiimide, 1,1'-carbonyldiimidazole, thionyldiimidazole, bis(2-oxo-1-oxazolidinyl)phosphinic acid chloride, 2-chloro-1-methylpyridinium tosylate and phosphorus oxychloride and the like can be mentioned. Preferred is 1-ethyl-3-(3-dimethylaminopropyl)carbodiimide or phosphorus oxychloride.

In step 3, a compound of the formula (I) is produced from a compound of the formula (IV). P¹ is removed under deprotection conditions known *per se* and V³ is introduced into the 2-position of isoquinoline.

When V³ is an optionally substituted alkyl group, the reaction is performed in the presence of V³-X (X is a leaving group, and chlorine atom, bromine atom, iodine atom, methanesulfonyl, ethanesulfonyl, benzenesulfonyl, p-toluenesulfonyl, fluorosulfonyl, trifluoromethanesulfonyl and the like can be mentioned) and base (triethylamine, pyridine, diisopropylethylamine, lutidine, 4-dimethylaminopyridine, potassium carbonate, sodium carbonate, potassium hydrogen carbonate, sodium hydrogencarbonate and the like can be mentioned) in a solvent that does not inhibit the reaction (step 3a). A preferable base is triethylamine or potassium carbonate.

When V³ is -CH₂V^{3a} (V^{3a} is V³ less bonded carbon), a catalytic reduction using a catalyst (palladium carbon, palladium black, platinum oxide etc.) in the presence of V^{3a}-CHO and a reducing agent (sodium cyanoborohydride, sodium acetoxyborohydride and the like can be mentioned), or in the presence of V^{3a}-CHO, can also be performed (step 3b).

The reducing agent is preferably sodium cyanoborohydride, and the catalyst used for the catalytic reduction is preferably palladium carbon.

When V³ is -COV⁴ (-COV⁴ is an optionally substituted alkylcarbonyl group optionally having a double bond, arylcarbonyl group, alkylcarbonylcarbonyl group, or these groups protected by the above-mentioned conventional protecting groups), the reaction is performed in the presence of V⁴CO₂H, a salt thereof (salt with sodium, potassium, calcium, triethylamine, pyridine and the like), or a reactive derivative of the carboxy group thereof, in a solvent that does not inhibit the reaction and, when desired, in the presence of base (triethylamine, pyridine, potassium carbonate etc.), all of which embodiments are encompassed in this reaction (step 3c).

As the reactive derivative, acid halide (acid chloride, acid bromide etc.), acid anhydride [mixed acid anhydride [substituted phosphoric acid (dialkylphosphoric acid etc.), alkylcarbonic acid (monoethylcarbonic acid etc.) and the like], active amide (amide with imidazole and the like), ester (cyanomethylester, 4-nitrophenylester and the like] and the like can be mentioned. Preferred is acid chloride.

When V⁴CO₂H is used in the form of a free acid or salt, the reaction is performed in the presence of a condensing agent. As the condensing agent, the condensing agent in step 2 can be mentioned. Preferred is 1,1'-carbonyldiimidazole.

When V³ is an alkylsulfonyl group or an optionally substituted aminosulfonyl group, the reaction is performed in the presence of the corresponding acid halide [acid chloride (methanesulfonyl chloride, octanesulfonyl chloride, acetylaminosulfonyl chloride etc.) and the like] and base (triethylamine, pyridine etc.) in an inert solvent (methylene chloride, chloroform, tetrahydrofuran etc.) (step 3d). It is preferable to use acid chloride in methylene chloride in the presence of triethylamine.

When V³ is -CONHR^{7a} (-CONHR^{7a} is optionally substituted carbamoyl group or that protected by a conventional protecting group), the reaction is performed in the presence of R^{7a}N=C=O in an inert solvent (methylene chloride, chloroform, tetrahydrofuran etc.), or by subjecting R^{7a}NH₂ in a toluene solvent after refluxing (from 1 hr to 4 hr) in the presence of trichloromethyl chloroformate (step 3e).

### (Production Method 2)

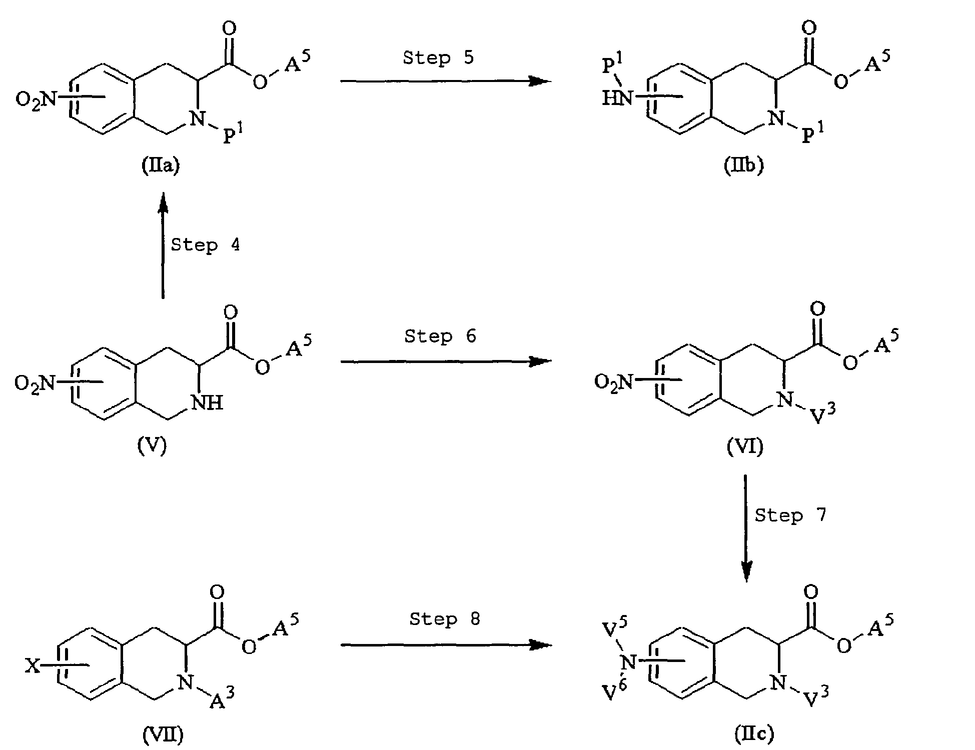

R⁴¹, R⁴², A³, A⁵, P¹, V³ and X in the above-mentioned formulas and the following description are as defined above.

In Production Method 2, compounds of the formulas (IIa), (IIb) and (IIc) (wherein R⁹ in the formula (I) is a nitro group, -NH-P¹ or -NV⁵V⁶, respectively), which are starting materials in Production Method 1, are produced from a compound of the formula (V).

V⁵ and V⁶ represent R⁴¹ and R⁴², or R⁴¹ and R⁴² protected by the aforementioned general protecting groups.

A compound of the formula (V) can be produced by a method known *per* se [Journal of Takeda Research Laboratories, 77-82(1984)] using phenylalanine as a starting material.

In step 4, a compound of the formula (IIa) is produced from a compound of the formula (V). Protection of amino group, which is known *per* se, is performed.

In step 5, a compound of the formula (IIb) is produced from a compound of the formula (IIa) wherein P¹ is a protecting group (benzyl, p-methoxybenzyl etc.) easily eliminated by a catalytic reduction. After selective reduction of nitro group, protection of amino group, which is known per se, is performed.

As a method of reducing a nitro group, a reaction in the presence of metal (iron, tin etc.) in a weak acidic solvent (water-containing acetic acid, diluted hydrochloric acid-methanol, diluted hydrochloric acid ethanol etc.) at a reaction temperature of from 40°C to 70°C can be mentioned. In addition, reduction can also be performed in the presence of metal hydride (sodium borohydride, potassium borohydride etc.) and ammonium sulfate, or in the presence of the above-mentioned metal hydride and metal chloride (copper chloride, cobalt chloride, nickel chloride etc.) in lower alcohol. Reduction using iron in diluted hydrochloric acid-methanol is preferable.

In step 6, a compound of the formula (VI) is produced from a compound of the formula (V). According to the methods of steps 3a - 3d, V³ can be introduced.

In step 7, a compound of the formula (IIc) is produced from a compound of the formula (VI).

After reduction of nitro group by a method known per se, the produced amino group is subjected to the methods described in steps 3a - 3d alone or in combination.

When -NV⁵V⁶ forms the aforementioned heterocycle, the compound can be produced according to the method of step 3a and using X-V⁵-V⁶-X.

In step 8, a compound of the formula (IIc) is produced from a compound of the formula (VII).

The reaction is performed in a solvent that does not inhibit the reaction, for example, dioxane, tetrahydrofuran, benzene, toluene, xylene, ethyl acetate, N,N-dimethylformamide, N,N-dimethylacetamide, N-methyl-2-pyrrolidinone, dimethyl sulfoxide and the like, or a mixture thereof, in the presence of HNV⁵V⁶, organic transition metal compound (palladium acetate, tetrakis(triphenylphosphine)palladium, tri(o-tolyl)phosphine, bis(triphenylphosphine)palladium dichloride, tris(dibenzylideneacetone)dipalladium, bis(triphenylphosphine)nickel bromide etc.), ligand (triphenylphosphine, bis(diphenylphosphino)propane, bis(diphenylphosphino)butane, 2,2'-bis(diphenylphosphino)-1,1'-binaphthyl(BINAP), tri-tert-butylphosphine etc.) and base (potassium carbonate, triethylamine, sodium tert-butoxide, cesium carbonate, diisopropylethylamine etc.). The reaction is preferably performed in dioxane in the presence of palladium acetate, BINAP and cesium carbonate.

The reaction temperature is preferably from 60°C to 120°C. The reaction time is generally from 1 hr to 12 hr.

### (Production Method 3)

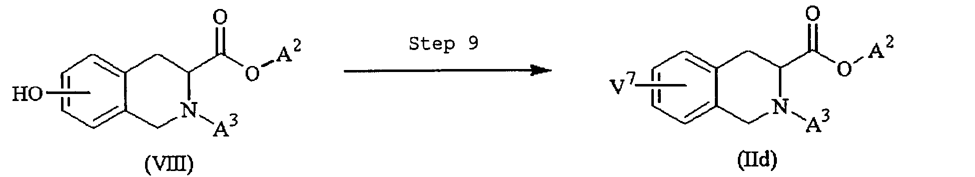

A², A³ and X in the above-mentioned formulas and the following description are as defined above.

In Production Method 3, a compound of the formula (IId) (the formula (I) wherein R⁴ is V⁷), which is a starting material of Production Method 1, is produced from a compound of the formula (VIII).

V⁷ is an optionally substituted alkoxy group or an optionally substituted alkoxy group protected by the aforementioned conventional protecting group.

In step 9, the reaction is performed in a solvent that does not inhibit the reaction, for example, acetone, dioxane, tetrahydrofuran, benzene, toluene, xylene, N,N-dimethylformamide, N,N-dimethylacetamide, N-methyl-2-pyrrolidinone, dimethyl sulfoxide and the like, or a mixture thereof, in the presence of W-X (W is V⁷ without oxygen atom bonded to isoquinoline skeleton) and a base (potassium carbonate, triethylamine, cesium carbonate etc.). When desired, a catalytic amount of a phase transfer catalyst (crown ether, benzyltriethylammonium chloride, tetraethylammonium fluoride etc.) may be co-present. The reaction is preferably performed in toluene in the presence of potassium carbonate and tetraethylammonium fluoride.

The reaction temperature is preferably from 60°C to 120°C. The reaction time is generally from 6 hr to 20 hr.

### (Production Method 4)

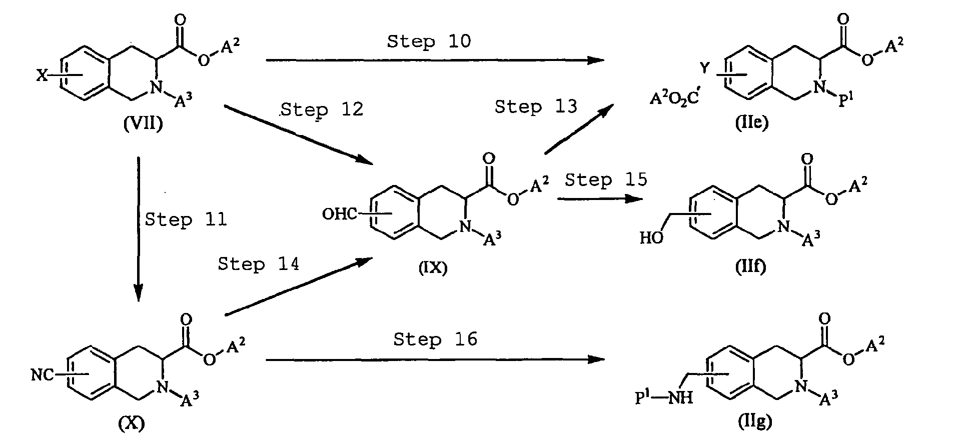

A², A³, P¹ and X in the above-mentioned formulas and the following description are as defined above. Y is alkyl or alkenyl.

In Production Method 4, compounds of the formulas (IIe), (IIf) and (IIg) (the formula (I) wherein R⁴ is -Y-CO₂A², a hydroxymethyl group or -CH₂-NH-P¹, respectively), which are starting materials of Production Method 1, are produced from a compound of the formula (VII).

In step 10, a compound of the formula (IIe) wherein -Y-CO₂A² is bonded to the position of X is produced from a compound of the formula (VII).

The reaction is performed in a solvent that does not inhibit the reaction, for example, dioxane, tetrahydrofuran, benzene, toluene, xylene, N,N-dimethylformamide, N,N-dimethylacetamide, N-methyl-2-pyrrolidinone, dimethyl sulfoxide and the like, or a mixture thereof in the presence of aliphatic carboxylic acid ester having an unsaturated end (for example, ethyl acrylate), the same organic transition metal compound as used in step 8, ligand and base. The reaction is preferably performed in dioxane in the presence of palladium acetate, BINAP and diisopropylethylamine.

The reaction temperature is preferably from 60°C to 120°C. The reaction time is generally from 6 hr to 24 hr.

When Y is alkenyl, reduction to alkyl may also be performed by a method known per se (hydrogenation using palladium catalyst etc.).

In step 11, a compound of the formula (X) wherein a cyano group is bonded to the position of X is produced from a compound of the formula (VII)).

The reaction is performed in a solvent that does not inhibit the reaction, for example, dioxane, tetrahydrofuran, benzene, toluene, xylene, N,N-dimethylformamide, N,N-dimethylacetamide, N-methyl-2-pyrrolidinone, dimethyl sulfoxide and the like, or a mixture thereof, in the presence of metal cyanide (sodium cyanide, potassium cyanide, zinc cyanide etc.), the same organic transition metal compound and ligand as used in step 8. The reaction is preferably performed in N-methyl-2-pyrrolidinone in the presence of potassium cyanide, palladium acetate and BINAP.

The reaction temperature is preferably from 60°C to 120°C. The reaction time is generally from 1 hr to 3 hr.

In step 12, a compound of the formula (IX) wherein a formyl group is bonded to the position of X is produced from a compound of the formula (VII).

The reaction is performed in a solvent that does not inhibit the reaction, for example, dioxane, tetrahydrofuran, benzene, toluene, xylene, N,N-dimethylformamide, N,N-dimethylacetamide, N-methyl-2-pyrrolidinone, dimethyl sulfoxide and the like, or a mixture thereof in the presence of a reducing agent (triethylsilane, trioctylsilane, lithium formate etc.) and the same organic transition metal compound, ligand and, when desired, a base, as used in step 8, under carbon monoxide atmosphere or carbon monoxide. The reaction is preferably performed in N,N-dimethylformamide, in the presence of lithium formate, bis(triphenylphosphine)palladium dichloride and triphenylphosphine with aeration with carbon monoxide.

The reaction temperature is preferably from 60°C to 120°C. The reaction time is generally from 1 hr to 12 hr.

In step 13, a compound of the formula (IIe) is produced from a compound of the formula (IX).

The reaction is performed in a solvent that does not inhibit the reaction, for example, dioxane, tetrahydrofuran, benzene, toluene, xylene, N,N-dimethylformamide, N,N-dimethylacetamide, N-methyl-2-pyrrolidinone, dimethyl sulfoxide and the like, or a mixture thereof in the presence of a phosphonic acid derivative (triethyl phosphonoacetate etc.) and a base (sodium hydride, potassium hydride etc.).

In step 14, a compound of the formula (IX) is produced from a compound of the formula (X).

As the reducing agent to a formyl group, Raney-nickel-aluminum alloy, Raney-nickel, platinum oxide and the like can be mentioned.

When Raney-nickel-aluminum alloy is used, the reaction is performed in formic acid or aqueous formic acid solution. The reaction temperature is preferably from 90°C to 120°C. The reaction time is generally from 10 min to 30 min.

When Raney-nickel is used, the reaction is performed in formic acid, aqueous formic acid solution or a mixed solvent of pyridine-acetic acid-water in the presence of a reducing agent (sodium hypophosphite etc.). The reaction temperature is preferably from 0°C to 30°C. The reaction time is generally from 1 hr to 6 hr.

When platinum oxide is used, the reaction is performed in formic acid or aqueous formic acid solution. The reaction temperature is preferably from 20°C to 60°C. The reaction time is generally from 1 hr to 6 hr.

A preferable reducing agent is Raney-nickel-aluminum alloy or Raney-nickel.

In step 15, a compound of the formula (IIf) is produced from a compound of the formula (IX).

The reaction is performed in a solvent that does not inhibit the reaction, for example, dioxane, tetrahydrofuran, benzene; toluene, xylene, ethyl acetate, methanol, ethanol, isopropanol, N,N-dimethylformamide, N,N-dimethylacetamide, N-methyl-2-pyrrolidinone and the like, or a mixture thereof, in the presence of a reducing agent (sodium borohydride, pyridine-borane complex, tert-butylamine-borane complex etc.). The reaction is preferably performed in ethyl acetate in the presence of tert-butylamine-borane complex.

The reaction temperature is preferably from 0°C to 40°C. The reaction time is generally from 1 hr to 4 hr.

In step 16, a compound of the formula (IIg) is produced from a compound of the formula (X).

The reaction is performed by a catalytic reduction (from normal pressure to 0.4 MPa) in ammonia-containing lower alcohol, in the presence of a catalyst (palladium carbon, palladium hydroxide carbon, Raney-nickel etc.), and protection of the produced aminomethyl group by a method known *per* se. A preferable catalyst is palladium hydroxide carbon.

### (Production Method 5)

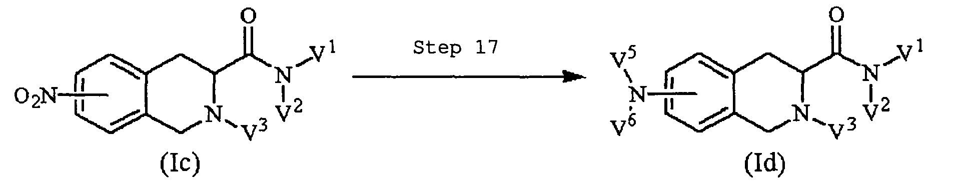

In the formula, V¹, V², V³, V⁵ and V⁶ are as defined above.

In Production Method 5, from a compound of the formula (Ic), a compound of the formula (Id) is directly produced.

Step 17 can be performed according to the method of step 7.

### (Production Method 6)

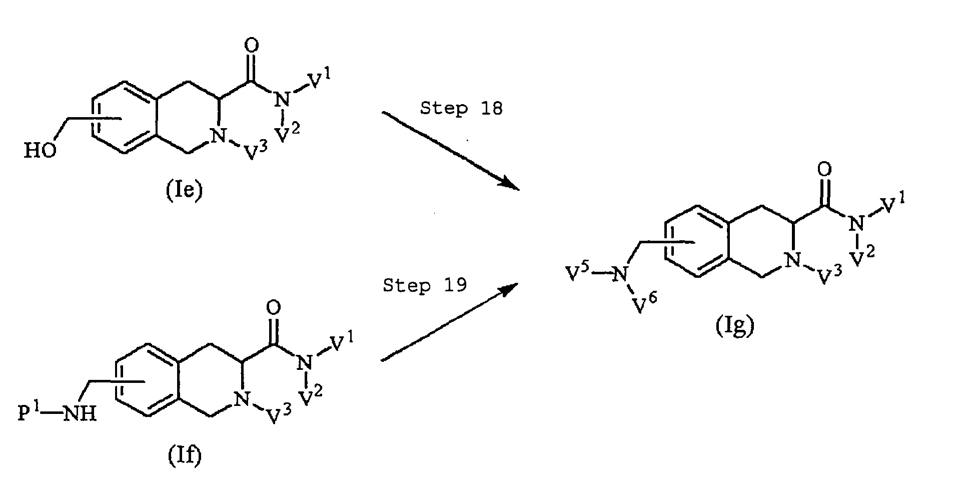

V¹, V², V³, V⁵, V⁶, P¹ and X in the above-mentioned formulas and the following description are as defined above.

In Production Method 6, a compound of the formula (Ig) is produced from compounds of the formulas (Ie) and (If).

In step 18, a compound of the formula (Ig) is produced from a compound of the formula (Ie). Hydroxyl group of hydroxymethyl group is converted to a leaving group (X) by a method known per se, which is followed by amination.

The amination is performed in an inert solvent, for example, dioxane, tetrahydrofuran, acetonitrile, benzene, toluene, xylene, N,N-dimethylformamide, N,N-dimethylacetamide, N-methyl-2-pyrrolidinone, dimethyl sulfoxide and the like, or a mixture thereof in the presence of HNV⁵V⁶ or a salt thereof. When desired, base (triethylamine, diisopropylethylamine, pyridine, potassium carbonate, sodium hydrogencarbonate etc.) may be co-present. A preferable solvent is tetrahydrofuran or acetonitrile and a preferable base is diisopropylethylamine.

The reaction temperature is preferably from 0°C to 60°C. The reaction time is generally from 2 hr to 24 hr.

In step 19, a compound of the formula (Ig) is produced from compound of the formula (If). After eliminating P¹ by a method known per se, the compound can be produced according to the methods of steps 3a - e.

The compound of the present invention obtained as in the above-mentioned can be purified by a known purification method (e.g., chromatography, recrystallization etc.).

In addition, the compound of the present invention can be converted to a pharmaceutically acceptable salt thereof by a method known per se.

### Examples

The present invention is hereinafter described in more detail by means of the following Reference Example, Example and Experimental Example, which, however, are not to be construed as limiting the invention.

### Reference Example 1: 2,6-di-tert-butyl-4-nitrophenol

2,6-Di-tert-butylphenol (5.00 g) was dissolved in isooctane (20 mL), 70% nitric acid (1.54 mL) was added under ice-cooling, and the mixture was allowed to warm to room temperature and stirred for 1 hr. The precipitate was collected by filtration, washed with water, and recrystallized from methanol to give the title compound as crystals (3.00 g).
IR ν (Nujol) cm⁻¹; 3543, 1510.
¹H-NMR (CDCl₃) δ (ppm); 1.47 (18H, s), 5.91 (1H, s), 8.12 (2H, s).

### Reference Example 2: N-(2,4,6-trimethylphenyl)acetamide

2,4,6-Trimethylaniline (3.0 g) was dissolved in methylene chloride (30 mL), pyridine (2.69 mL) and acetyl chloride (2.37 mL) were added under ice-cooling, and the mixture was stirred at the same temperature for 0.5 hr. The reaction solution was washed successively with 10% aqueous citric acid solution and saturated brine, and dried over sodium sulfate, and then methylene chloride was evaporated under reduced pressure. The obtained residue was washed with a mixed solution of diisopropyl ether - n-hexane to give the title compound as crystals (3.67 g).
IR ν (Nujol) cm⁻¹; 3234, 3188, 3040, 1660, 1645.
¹H-NMR (CDCl₃) δ (ppm); 2.18 (6H, s), 2.21 (3H, s), 2.25 (3H, s), 6.40-7.20 (1H, br), 6.87 (2H, s).

### Reference Example 3: N-(3-nitro-2,4,6-trimethylphenyl)acetamide

The compound (3.64 g) of Reference Example 2 was dissolved in acetic acid (35 mL), concentrated sulfuric acid (10.9 mL) and fuming nitric acid (8.6 mL) were successively added dropwise under ice-cooling, and the mixture was stirred at the same temperature for 0.5 hr. Water (150 mL) was added to the reaction solution, and the precipitated crystals were collected by filtration. The obtained crystals were dissolved in chloroform (50 mL), and the solution was washed with saturated brine and dried over sodium sulfate. Then, chloroform was evaporated under reduced pressure. The obtained residue was washed with diisopropyl ether to give the title compound as a powder (3.76 g).
IR ν (Nujol) cm⁻¹; 3219, 3172, 1676, 1651.
¹H-NMR (CDCl₃) δ (ppm); 2.12 (3H, s), 2.19 (6H, s), 2.24 (3H, s), 6.60-7.20 (1H, br), 6.98 (1H, s).

### Reference Example 4: 3-nitro-2,4,6-trimethylaniline

The compound (1.00 g) of Reference Example 3 was dissolved in methanol (10 mL), concentrated hydrochloric acid (1.0 mL) was added, and the mixture was refluxed for 2.5 hr. Then, concentrated hydrochloric acid (1.0 mL) was added, and the mixture was further refluxed for 12 hr. Chloroform (50 mL) was added to the reaction solution, and the mixture was washed successively with saturated aqueous sodium hydrogen carbonate and saturated brine, and dried over sodium sulfate. Chloroform was evaporated under reduced pressure to give the title compound as a powder (0.81 g).
IR ν (Nujol) cm⁻¹; 3417, 3348, 3230, 1634.
¹H-NMR (CDCl₃) δ (ppm); 2.06 (3H, s), 2.16 (6H, s), 3.40-3.90 (2H, br), 6.83 (1H, s).

### Reference Example 5: N-tert-butoxycarbonyl-N'-(3-nitro-2,4,6-trimethylphenyl)sulfamide

tert-Butanol (0.84 mL) was dissolved in methylene chloride (8.0 mL), chlorosulfonyl isocyanate (0.76 mL) was added at -10°C, and the mixture was stirred at the same temperature for 20 min. A solution of the compound (0.76 g) of Reference Example 4 in methylene chloride (2.0 mL) and triethylamine (1.22 mL) were successively added to the reaction mixture, and the mixture was stirred at the same temperature for 0.5 hr. The reaction solution was washed successively with 10% aqueous citric acid solution, saturated aqueous sodium hydrogen carbonate and saturated brine, and dried over sodium sulfate. Then, methylene chloride was evaporated under reduced pressure. The obtained residue was washed with diethyl ether to give the title compound as a powder (1.01 g).
IR ν (Nujol) cm⁻¹; 3287, 3238, 1722, 1352, 1257, 1142.
¹H-NMR (DMSO-d₆) δ (ppm); 1.41 (9H, s), 2.19 (6H, s), 2.49 (3H, s), 2.60-3.80 (2H, br), 7.17 (1H, s).

### Reference Example 6: N-(3-amino-2,4,6-trimethylphenyl)-N'-tert-butoxycarbonylsulfamide

10% Palladium carbon (100 mg) was suspended in methanol (20 mL), the compound (990 mg) of Reference Example 5 was added thereto, and the mixture was subjected to catalytic hydrogenation at room temperature for 17 hr under 4.0 kgf/cm². After palladium carbon was removed by filtration, methanol was evaporated under reduced pressure. The obtained residue was washed with diisopropyl ether - diethyl ether to give the title compound as a powder (684 mg).
IR ν (Nujol) cm⁻¹; 3265, 1728, 1685, 1346, 1249, 1153.
¹H-NMR (CDCl₃+DMSO-d₆) δ (ppm); 1.50 (9H, s), 2.12 (3H, s), 2.20 (3H, s), 2.27 (3H, s), 3.00-4.60 (4H, br), 6.80 (1H, s). Reference Example 7: 6-amino-2-methyl-3-nitropyridine

According to the method described in Tetrahedron Lett., 24, 2211-2212 (1971), the title compound was synthesized by means of the following.

To concentrated sulfuric acid (75 mL) was added 2-amino-6-methylpyridine (19.3 g) to be dissolved at 20°C or below, a solution of fuming nitric acid (8.0 mL) in concentrated sulfuric acid (8 mL) was added dropwise at 5°C or below over 15 min, and the mixture was further stirred at the same temperature for 2 hr. The reaction mixture was poured into ice water (1.4 L), pH of the mixture was adjusted to 10 with aqueous ammonia (300 mL), and the precipitate was collected by filtration. The precipitate was dissolved in ethyl acetate (600 mL), and the solution was washed successively with 10% aqueous ammonia and saturated brine, and dried over sodium sulfate. Then, ethyl acetate was evaporated under reduced pressure to give a powder (22.5 g). Toluene (200 mL) was added to this powder, and the mixture was refluxed for 1 hr and allowed to cool. The obtained powder was collected by filtration. The obtained powder was washed with methylene chloride (100 mL) and collected by filtration to give the title compound as a powder (14.5 g).
IR ν (Nujol) cm⁻¹; 1662, 1594, 1404, 1287, 1076.
¹H-NMR (DMSO-d₆) δ (ppm); 2.62 (3H, s), 6.38 (1H, d, J=9.3 Hz), 7.34 (2H, br-s), 8.09 (1H, d, J=9.3 Hz).

### Reference Example 8: 6-hydroxy-2-methyl-3-nitropyridine

The compound (5.00 g) of Reference Example 7 was suspended in water (80 mL), concentrated sulfuric acid (5.8 mL) was added, and the mixture was heated until the compound was dissolved, and then allowed to cool with stirring. A solution of sodium nitrite (3.43 g) in water (17 mL) was added under ice-cooling, and the mixture was stirred at the same temperature for 3.5 hr. Then, an additional solution of sodium nitrite (1.14 g) in water (6 mL) was added, and the mixture was stirred at the same temperature for 3 hr. The precipitate was collected by filtration to give the title compound as a powder (3.60 g).
¹H-NMR (DMSO-d₆) δ (ppm); 2.64 (3H, s), 6.29 (1H, d, J=10.1 Hz), 8.11 (1H, d, J=10.1 Hz), 12.48 (1H, br-s).

### Reference Example 9: 1,6-dimethyl-5-nitro-1H-pyridin-2-one

The compound (1.00 g) of Reference Example 8 was dissolved in N,N-dimethylformamide (10 mL), methyl iodide (0.81 mL) and sodium hydride (60% w/w) (338 mg) were successively added under ice-cooling, and the mixture was stirred at the same temperature for 1 hr, and then at room temperature for 30 min. The reaction mixture was poured into ice water, and the mixture was extracted twice with ethyl acetate (20 mL). The ethyl acetate layers were combined, washed successively with saturated aqueous sodium hydrogen carbonate, 5% aqueous citric acid solution and saturated brine, and dried over sodium sulfate. Then, ethyl acetate was evaporated under reduced pressure. The residue was purified by column chromatography to give the title compound as a powder (357 mg).
¹H-NMR (DMSO-d₆) δ (ppm); 2.80 (3H, s), 3.64 (3H, s), 6.49 (1H, d, J=10.2 Hz), 8.01 (1H, d, J=10.2 Hz).

### Reference Example 10: tert-butyl 2-methyl-3-nitro-6-oxo-1H-pyridine-1-carboxylate

The compound (1.00 g) of Reference Example 8 and di-tert-butyl bicarbonate (1.56 g) were dissolved in N,N-dimethylformamide (10 mL), sodium hydride (60%w/w, 338 mg) was added under ice-cooling, and the mixture was stirred at the same temperature for 40 min, and then at room temperature for 1 hr. The reaction mixture was poured into ice water, and the mixture was extracted three times with diethyl ether (20 mL). The diethyl ether layers were combined, washed successively with saturated aqueous sodium hydrogen carbonate and saturated brine, and dried over sodium sulfate. Then, diethyl ether was evaporated under reduced pressure. The residue was purified by column chromatography to give the title compound as an oil (1.57 g).
IR ν (neat) cm⁻¹; 1765, 1609, 1576, 1528, 1348, 1234, 1144, 876, 839.
¹H-NMR (CDCl₃) δ (ppm); 1.58 (9H, s), 2.85 (3H, s), 7.17 (1H, d, J=8.7 Hz), 8.41 (1H, d, J=8.7 Hz).

### Reference Example 11: tert-butyl 3-amino-2-methyl-6-oxo-1H-pyridine-1-carboxylate

10% Palladium carbon (70 mg) was suspended in methanol (15 mL), the compound (700 mg) of Reference Example 10 was added thereto, and the mixture was subjected to catalytic hydrogenation at room temperature for 19 hr under 3 kgf/cm². After palladium carbon was removed by filtration, methanol was evaporated under reduced pressure, and the residue was purified by column chromatography. The obtained residue was washed with n-hexane (10 mL) and collected by filtration to give the title compound as a powder (414 mg).
¹H-NMR (CDCl₃) δ (ppm); 1.54 (9H, s), 2.36 (3H, s), 3.58 (2H, br-s), 6.82 (1H, d, J=8.3 Hz), 7.01 (1H, d, J=8.3 Hz). Reference Example 12: acetylsulfamoyl chloride

Chlorosulfonyl isocyanate (3.04 mL) was added dropwise to acetic acid (2.0 mL) under ice-cooling. n-Hexane was added, and the precipitate was collected by filtration to give the title compound as a powder (5.31 g). Reference Example 13: 2,3,6-trimethyl-4-nitrophenol

2,3,6-Trimethylphenol (10.0 g) was dissolved in isooctane (300 mL), 70% nitric acid (4.65 mL) was added under ice-cooling, and the mixture was stirred at room temperature for 2 hr. The precipitate was collected by filtration and dissolved in chloroform (200 mL), and the solution was washed successively with water and saturated brine, and dried over sodium sulfate. Then, chloroform was evaporated under reduced pressure. The residue was purified by column chromatography to give the title compound as a powder (4.50 g).
IR ν (Nujol) cm⁻¹; 3396, 2923, 2854, 1508.
¹H-NMR (CDCl₃) δ (ppm); 2.23, 2.26 (6H, s, s), 2.42 (3H, s), 5.22 (1H, br-s), 7.59 (1H, s).

### Reference Example 14: 2,3,6-trimethyl-4-nitrophenyl acetate

The compound (8.17 g) of Reference Example 13 was dissolved in methylene chloride (80 mL), acetyl chloride (3.5 mL) and triethylamine (8.2 mL) were successively added under ice-cooling, and the mixture was stirred at the same temperature for 10 min. The reaction mixture was washed successively with 5% aqueous citric acid solution, saturated aqueous sodium hydrogen carbonate and saturated brine, and dried over sodium sulfate. Methylene chloride was evaporated under reduced pressure, and the residue was purified by column chromatography. The obtained residue was washed with n-hexane and collected by filtration to give the title compound as a powder (8.81 g).
IR ν (Nujol) cm⁻¹; 1759, 1518, 1188, 1084, 901, 766.
¹H-NMR (CDCl₃) δ (ppm); 2.14, 2.18 (6H, s, s), 2.38 (6H, s), 7.54 (1H, s).

### Reference Example 15: 4-amino-2,3,6-trimethylphenyl acetate

10% Palladium carbon (0.9 g) was suspended in methanol (150 mL), the compound (8.87 g) of Reference Example 14 was added thereto, and the mixture was subjected to catalytic hydrogenation at 30°C for 3 hr under 4.0 kgf/cm². After palladium carbon was removed by filtration, methanol was evaporated under reduced pressure. The obtained residue was dissolved by heating in ethyl acetate (5 mL), n-hexane (100 mL) was added, and the mixture was stirred for 2 hr. The precipitate was collected by filtration to give the title compound as a powder (6.71 g).
IR ν (Nujol) cm⁻¹; 1759, 1518, 1188, 1084, 901, 766.
¹H-NMR (CDCl₃) δ (ppm); 2.04, 2.18 (9H, s), 2.30 (3H, s), 3.20-3.60 (2H, br), 6.41 (1H, s).

### Reference Example 16: 5-amino-1,6-dimethyl-1H-pyridin-2-one

10% Palladium carbon (35 mg) was suspended in methanol (10 mL), the compound (357 mg) of Reference Example 9 was added thereto, and the mixture was subjected to catalytic hydrogenation at 25°C for 1 hr under 3 kgf/cm². After palladium carbon was removed by filtration, methanol was evaporated under reduced pressure to give the title compound as a powder (304 mg).
¹H-NMR (CDCl₃) δ (ppm); 2.27 (3H, s), 2.92 (2H, br-s), 3.54 (3H, s), 6.41 (1H, d, J=9.4 Hz), 7.00 (1H, d, J=9.4 Hz). Example 1: 2-(2,6-diisopropylphenyl)aminocarbonyl-3-(piperidine-1-carbonyl)-1,2,3,4-tetrahydroisoquinoline

### (1) 2-(2,6-diisopropylphenyl)aminocarbonyl-1,2,3,4-tetrahydroisoquinoline-3-carboxylic acid

1,2,3,4-Tetrahydroisoquinoline-3-carboxylate hydrochloride (1.50 g) was suspended in methylene chloride (15 mL), triethylamine (1.8 mL) and a solution of 2,6-diisopropylphenylisocyanate (1.34 g) in methylene chloride (5 mL) were added under ice-cooling, and the mixture was stirred at room temperature for 2 hr. The reaction mixture was poured into ice-cooled 1 M hydrochloric acid (30 mL), and the precipitate was collected by filtration. The precipitate was dissolved in heated chloroform, and the mixture was dried over sodium sulfate. Then, chloroform was evaporated under reduced pressure. The obtained residue was recrystallized from ethyl acetate (75 mL) to give the title compound as crystals (2.34 g).
IR ν (Nujol) cm⁻¹; 1722, 1616, 1520.
¹H-NMR (CDCl₃) δ (ppm); 1.17 (12H, dd, J=6.7, 4.1Hz), 2.90-3.40 (4H, m), 4.66 (2H, s), 5.20 (1H, dd, J=5.5, 3.5Hz), 6.15 (1H, br-s), 7.00-7.40 (7H, m), 8.20-8.70 (1H, br).

### (2) 2-(2,6-diisopropylphenyl)aminocarbonyl-3-(piperidine-1-carbonyl)-1,2,3,4-tetrahydroisoquinoline

The compound (1.50 g) of (1) was suspended in methylene chloride (15 mL), 1-ethyl-3-(3-dimethylaminopropyl)carbodiimide hydrochloride (736 mg) and piperidine (0.38 mL) were added under ice-cooling, and the mixture was stirred for 1.5 hr. Methylene chloride was evaporated under reduced pressure. 1 M hydrochloric acid (15 mL) was added to the residue, and the mixture was extracted with ethyl acetate (30 mL), washed successively with 2 M aqueous sodium hydroxide solution (15 mL) and saturated brine, and dried over sodium sulfate. Then, ethyl acetate was evaporated under reduced pressure. The residue was purified by column chromatography, and the obtained residue was washed with diisopropyl ether (15 mL) and collected by filtration to give the title compound as a powder (1.03 g).
IR ν (Nujol) cm⁻¹; 1630, 1501.
¹H-NMR (CDCl₃) δ (ppm); 1.17 (12H, dd, J=6.8, 2.4Hz), 1.30-2.00 (6H, m), 2.90-3.90 (8H, m), 4.69, 5.02 (2H, AB-q, J=14.3Hz), 5.57 (1H, dd, J=6.2, 4.0Hz), 5.99 (1H, br-s), 7.00-7.50 (7H, m).

### Example 2: N-(2,6-diisopropylphenyl)-2-(2,2-dimethylpropionyl)-1,2,3,4-tetrahydroisoquinoline-(3S)-carboxamide

### (1) methyl 2-(2,2-dimethylpropionyl)-1,2,3,4-tetrahydroisoquinoline-(3S)-carboxylate

Methyl 1,2,3,4-tetrahydroisoquinoline-(3S)-carboxylate (1.00 g) was dissolved in methylene chloride (10 mL), triethylamine (1.09 mL) and pivaloyl chloride (0.70 mL) were added under ice-cooling, and the mixture was stirred at room temperature for 1 hr. The reaction mixture was washed successively with 10% aqueous citric acid solution and saturated brine, and dried over sodium sulfate. Then, methylene chloride was evaporated under reduced pressure. The residue was purified by column chromatography to give the title compound as a powder (1.45 g).
IR ν (Nujol) cm⁻¹; 3470, 1922, 1746, 1622, 1586, 1557.
¹H-NMR (CDCl₃) δ (ppm); 1.35 (9H, s), 3.19 (2H, d, J=5.4 Hz), 3.65 (3H, s), 4.59, 5.01 (2H, AB-q, J=16.3 Hz), 5.19 (1H, t, J=5.4 Hz), 7.01-7.32 (4H, m).

### (2) 2-(2,2-Dimethylpropionyl)-1,2,3,4-tetrahydroisoquinoline-(3S)-carboxylic acid

The compound (5.17 g) of (1) was dissolved in a mixed solution of tetrahydrofuran - methanol (3:1, 120 mL), 1 M aqueous lithium hydroxide solution (38 mL) was added at room temperature, and the mixture was stirred at the same temperature for 1 hr. The reaction mixture was acidified with 10% aqueous citric acid solution, tetrahydrofuran-methanol was evaporated under reduced pressure, and the residue was extracted twice with ethyl acetate (50 mL). The ethyl acetate layers were combined, washed with saturated brine, and dried over sodium sulfate. Then, ethyl acetate was evaporated under reduced pressure. The residue was triturated with n-hexane (100 mL), and the powder was collected by filtration to give the title compound as a powder (4.08 g).
IR ν (Nujol) cm⁻¹; 2690, 1733, 1699, 1560, 1583, 1557, 1502.
¹H-NMR (CDCl₃) δ (ppm); 1.34 (9H, s), 3.19 (2H, d, J=5.7 Hz), 4.56, 4.98 (2H, AB-q, J=16.1 Hz), 5.13 (1H, t, J=5.7 Hz), 7.00-7.35 (4H, m), 8.00-8.27 (1H, br).

### (3) N-(2,6-diisopropylphenyl)-2-(2,2-dimethylpropionyl)-1,2,3,4-tetrahydroisoquinoline-(3S)-carboxamide

The compound (1.05 g) of (2) was dissolved in methylene chloride (10 mL), 2,6-diisopropylaniline (0.76 mL) and 1-ethyl-3-(3-dimethylaminopropyl)carbodiimide hydrochloride (923 mg) were added under ice-cooling, and the mixture was stirred at room temperature for 2.5 hr. The reaction mixture was washed successively with 10% aqueous citric acid solution and saturated brine, and dried over sodium sulfate. Then, methylene chloride was evaporated under reduced pressure. The residue was purified by column chromatography and recrystallized from benzene (3.7 mL) to give the title compound (824 mg).
IR ν (Nujol) cm⁻¹; 3272, 1659, 1616, 1590, 1524.
¹H-NMR (CDCl₃) δ (ppm); 1.05, 1.08 (12H, d, d, J=6.8 Hz, 6.8 Hz), 1.41 (9H, s), 2.55-2.92 (2H, m), 3.10 (1H, dd, J=15.6, 6.0 Hz), 3.58 (1H, dd, J=15.6, 6.0 Hz), 4.63, 4.98 (2H, AB-q, J=15.0 Hz), 5.27 (1H, t, J=6.0 Hz), 6.99-7.50 (8H, m).

The compounds of Example 3-Example 26 were synthesized according to Example 2. Example 3: N-(2,6-diisopropylphenyl)-2-octanoyl-1,2,3,4-tetrahydroisoquinoline-(3S)-carboxamide
IR ν (neat) cm⁻¹; 1699, 1634, 1539, 1431, 1258, 1207, 1142, 1097, 964, 847.
¹H-NMR (CDCl₃) δ (ppm); 0.88 (3H, br-t), 1.0-2.0 (10H, m), 2.3-2.7 (2H, m), 3.01 (1H, dd, J=15.8, 6.4 Hz), 3.47 (1H, dd, J=15.8, 4.7 Hz), 4.4-5.0 (2H, m), 5.33 (1H, dd, J=6.4, 4.7 Hz), 6.6-7.0 (2H, m), 7.0-7.5 (4H, m), 7.8-8.3 (1H, m), 8.68 (1H, br-s).
Example 4: 2-benzoyl-N-(2,6-diisopropylphenyl)-1,2,3,4-tetrahydroisoquinoline-(3S)-carboxamide
IR ν (Nujol) cm⁻¹; 3270, 1678, 1616, 1587, 1576.
¹H-NMR (CDCl₃) δ (ppm); 0.72-1.32 (12H, m), 2.60-3.80 (4H, m), 4.32-4.80 (2H, m), 5.10-5.60 (1H, m), 6.90-7.70 (13H, m).

### Example 5: 2-(2,2-dimethylpropionyl)-N-(2,4,6-trimethylphenyl)-1,2,3,4-tetrahydroisoquinoline-(3S)-carboxamide

IR ν (Nujol) cm⁻¹; 3273, 1678, 1628, 1529.
¹H-NMR (CDCl₃) δ (ppm); 1.39 (9H, s), 1.98, 2.21 (9H, s, s), 3.07 (1H, dd, J=16.7, 5.8 Hz), 3.58 (1H, dd, J=16.7, 5.8 Hz), 4.59, 4.98 (2H, AB-q, J=15.9 Hz), 5.23 (1H, t, J=5.8 Hz), 6.80 (2H, s), 7.07-7.28 (4H, m), 7.36-7.55 (1H, br).

### Example 6: N-(2,4-difluorophenyl)-2-(2,2-dimethylpropionyl)-1,2,3,4-tetrahydroisoquinoline-(3S)-carboxamide

IR ν (neat) cm⁻¹; 3420, 3287, 3152, 3071, 2976, 2876, 1699, 1614, 1539, 1504.
¹H-NMR (CDCl₃) δ (ppm); 1.38 (9H, s), 3.08 (1H, dd, J=14.4, 7.4 Hz), 3.49 (1H, dd, J=14.4, 7.4 Hz), 4.50, 5.01 (2H, AB-q, J=15.7 Hz), 5.26 (1H, t, J=7.4 Hz), 6.65-6.97 (2H, m), 7.08-7.36 (4H, m), 7.99-8.40 (2H, m).

### Example 7: N-(2,4-difluorophenyl)-2-octanoyl-1,2,3,4-tetrahydroisoquinoline-(3S)-carboxamide

IR ν (neat) cm⁻¹; 3281, 3030, 2928, 2856, 1736, 1699, 1634, 1539, 1516.
¹H-NMR (CDCl₃) δ (ppm); 0.87 (3H, br-t), 1.03-1.91 (10H, m), 2.25-2.70 (2H, m), 3.01 (1H, dd, J=15.5, 5.6 Hz), 3.47 (1H, dd, J=15.5, 5.6 Hz), 4.34-5.00 (2H, m), 5.33 (1H, t, J=5.7 Hz), 6.60-6.98 (2H, m), 7.07-7.50 (4H, m), 7.90-8.32 (1H, m), 8.60-8.88 (1H, br).

### Example 8: N-cyclohexyl-2-octanoyl-1,2,3,4-tetrahydroisoquinoline-(3S)-carboxamide

IR ν (neat) cm⁻¹; 3425, 3308, 3069, 2930, 2855, 1634, 1589, 1537.
¹H-NMR (CDCl₃) δ (ppm); 0.88 (3H, br-t), 1.00-1.92 (20H, m), 2.20-2.60 (2H, m), 2.92-3.75 (3H, m), 4.30-4.90 (2H, m), 4.90-5.43 (1H, m), 6.05-6.31 (1H, br), 7.03-7.41 (4H, m).

### Example 9: 2-octanoyl-N-(2,4,6-trimethylphenyl)-1,2,3,4-tetrahydroisoquinoline-(3S)-carboxamide

IR ν (Nujol) cm⁻¹; 3231, 3123, 1651, 1609, 1537.
¹H-NMR (CDCl₃) δ (ppm); 0.88 (3H, br-t), 1.08-1.80 (10H, m), 1.66, 1.91, 2.19 (9H, s, s, s), 2.30-2.60 (2H, m), 2.82-3.65 (2H, m), 4.39-5.06 (2H, m), 5.30 (1H, br-t), 6.76 (2H, s), 6.92-7.35 (4H, m), 7.35-7.53 (1H, br).

### Example 10: N-cyclopropyl-2-octanoyl-1,2,3,4-tetrahydroisoquinoline-(3S)-carboxamide

IR ν (neat) cm⁻¹; 3433, 3294, 3009, 2955, 2928, 2855, 1647, 1526.
¹H-NMR (CDCl₃) δ (ppm); 0.21-0.75 (4H, m), 0.88 (3H, br-t), 1.08-1.90 (10H, m), 2.13-2.75 (3H, m), 2.90-3.60 (2H, m), 4.31-4.78 (2H, m), 5.04 (1H, br-t), 6.30-6.60 (1H, br), 7.00-7.40 (4H, m).

### Example 11: 2-(2,2-dimethylhexanoyl)-N-(2,4,6-trimethylphenyl)-1,2,3,4-tetrahydroisoquinoline-(3S)-carboxamide

IR ν (Nujol) cm⁻¹; 3258, 2729, 1693, 1651, 1614.
¹H-NMR (CDCl₃) δ (ppm); 0.80 (3H, br-t), 0.97-1.82 (6H, m), 1.33, 1.36 (6H, s, s), 1.99, 2.21 (9H, s, s), 3.07 (1H, dd, J=15.2, 5.8 Hz), 3.53 (1H, dd, J=15.2, 5.8 Hz), 4.59, 4.97 (2H, AB-q, J=15.0 Hz), 5.23 (1H, t, J=5.8 Hz), 6.81 (2H, s), 7.07-7.31 (4H, m), 7.46 (1H, br-s).

### Example 12: 2-hexanoyl-N-(2,4,6-trimethylphenyl)-1,2,3,4-tetrahydroisoquinoline-(3S)-carboxamide

IR ν (Nujol) cm⁻¹; 3272, 1652, 1607, 1505.
¹H-NMR (CDCl₃) δ (ppm); 0.91 (3H, br-t), 1.10-2.00 (6H, m), 1.66, 1.91, 2.19 (9H, s, s, s), 2.29-2.66 (2H, m), 2.85-3.68 (2H, m), 4.40-5.10 (2H, m), 5.30 (1H, br-t), 6.76 (2H, s), 6.98-7.39 (4H, m), 7.39-7.66 (1H, br).

### Example 13: N-(3,5-di-tert-butyl-4-hydroxyphenyl)-2-pentanoyl-1,2,3,4-tetrahydroisoquinoline-(3S)-carboxamide

¹H-NMR (CDCl₃) δ (ppm); 0.94 (3H, br-t), 1.10-1.90 (4H, m), 1.39 (18H, s), 2.30-2.65 (2H, m), 3.00 (1H, dd, J=14.9, 6.3 Hz), 3.51 (1H, dd, J=14.9, 5.4 Hz), 4.46-5.11 (2H, m), 5.24 (1H, t, J=5.4 Hz), 6.80-6.93 (1H, br), 7.00-7.46 (6H, m), 8.28-8.47 (1H, br).

### Example 14: 2-acetyl-N-(2,6-diisopropylphenyl)-1,2,3,4-tetrahydroisoquinoline-(3S)-carboxamide

IR ν (Nujol) cm⁻¹; 3402, 3271, 1645.
¹H-NMR (CDCl₃) δ (ppm); 0.92 (12H, br-d), 2.33 (3H, s), 2.1-2.8 (2H, m), 2.9-3.7 (2H, m), 4.4-5.0 (2H, m), 5.40 (1H, br-t), 6.8-7.5 (8H, m).

### Example 15: 2-hexanoyl-N-(3-methanesulfonylamino-2,4,6-trimethylphenyl)-1,2,3,4-tetrahydroisoquinoline-(3S)-carboxamide

IR ν (Nujol) cm⁻¹; 3371, 3126, 1680, 1632, 1296, 1136.
¹H-NMR (DMSO-d₆) δ (ppm); 0.90 (3H, br-t), 1.30-1.40 (4H, m), 1.60-1.90 (2H, m), 1.78 (3H, s), 1.88 (3H, s), 2.15 (3H, s), 2.40-2.70 (2H, m), 2.95 (3H, s), 3.07 (3/4H, dd, J=14.7, 6.0 Hz), 3.14 (1/4H, dd, J=14.7, 6.0 Hz), 3.53 (3/4H, dd, J=14.7, 3.9 Hz), 3.59 (1/4H, dd, J=14.7, 3.9 Hz), 4.58 (1/4H, d, J=16.6 Hz), 4.64 (3/2H, s), 4.80-4.90 (1/4H, m), 4.90 (1/4H, d, J=16.6 Hz), 5.20-5.40 (3/4H, m), 6.29 (1/4H, s), 6.45 (3/4H, s), 6.78 (1H, s), 7.10-7.30 (17/4H, m), 7.43 (3/4H, s).

### Example 16: 2-(2,2-dimethylpropionyl)-N-(3-methanesulfonylamino-2,4,6-trimethylphenyl)-1,2,3,4-tetrahydroisoquinoline-(3S)-carboxamide

IR ν (Nujol) cm⁻¹; 3251, 3125, 1682, 1327, 1153.
¹H-NMR (CDCl₃) δ (ppm); 1.42 (9H, s), 1.95 (3H, s), 1.97 (3H, s), 2.18 (3H, s), 2.94 (3H, s), 3.14 (1H, dd, J=15.7, 6.7 Hz), 3.51 (1H, dd, J=15.7, 5.4 Hz), 4.67, 4.94 (2H, AB-q, J=15.4 Hz), 5.19 (1H, dd, J=6.7, 5.4 Hz), 6.45 (1H, s), 6.83 (1H, s), 7.10-7.30 (4H, m), 7.52 (1H, s).

### Example 17: 2-[(2E,4E)-hexadienoyl]-N-(3-methanesulfonylamino-2,4,6-trimethylphenyl)-1,2,3,4-tetrahydroisoquinoline-(3S)-carboxamide

IR ν (Nujol) cm⁻¹; 3202, 1651, 1624, 1317, 1151.
¹H-NMR (CDCl₃) δ (ppm); 1.78 (3H, s), 1.83 (3H, s), 2.18 (3H, s), 1.89 (3H, d, J=5.1 Hz), 2.92 (3H, s), 3.07 (1/2H, dd, J=15.1, 5.3 Hz), 3.19 (1/2H, dd, J=15.1, 4.5 Hz), 3.57 (1H, d, J=15.1 Hz), 4.53, 5.03 (1H, AB-q, J=16.4 Hz), 4.75 (1H, s), 4.90-5.00 (1/2H, m), 5.30-5.40 (1/2H, m), 6.10-6.50 (4H, m), 6.78 (1H, s), 7.20-7.40 (9/2H, m), 7.30-7.50 (1H, m), 7.54 (1/2H, s).

### Example 18: 2-(3,3-dimethylbutyryl)-N-(3-methanesulfonylamino-2,4,6-trimethylphenyl)-1,2,3,4-tetrahydroisoquinoline-(3S)-carboxamide

IR ν (Nujol) cm⁻¹; 3182, 2361, 2343, 1653, 1313, 1150.
¹H-NMR (CDCl₃) δ (ppm); 1.11 (9H, s), 1.86 (3H, s), 1.91 (3H, s), 2.20 (3H, s), 2.43, 2.50 (2H, AB-q, J=14.8 Hz), 2.95 (3H, s), 3.07 (7/10H, dd, J=17.1, 6.2 Hz), 3.15 (3/10H, dd, J=17.1, 6.2 Hz), 3.52 (7/10H, dd, J=15.4, 4.1 Hz), 3.58 (3/10H, br-d, J=15.4 Hz), 4.57 (3/10H, d, J=16.8 Hz), 4.65, 4.70 (14/10H, AB-q, J=14.7 Hz), 4.90-5.00 (3/10H, m), 4.93 (3/10H, br-d, J=16.8 Hz), 5.30 (7/10H, dd, J=6.2, 4.1 Hz), 6.13 (3/10H, s), 6.24 (7/10H, s), 6.82 (1H, s), 7.20-7.35 (43/10H, m), 7.39 (7/10H, s).

### Example 19: 2-(2,2-dimethylbutyryl)-N-(3-methanesulfonylamino-2,4,6-trimethylphenyl)-1,2,3,4-tetrahydroisoquinoline-(3S)-carboxamide

IR ν (Nujol) cm⁻¹; 3256, 3144, 1680, 1327, 1153.
¹H-NMR (CDCl₃) δ (ppm); 0.86 (3H, t, J=7.4 Hz), 1.34 (3H, s), 1.37 (3H, s), 1.70-1.90 (2H, m), 1.98 (6H, s), 2.22 (3H, s), 2.97 (3H, s), 3.12 (1H, dd, J=15.7, 6.1 Hz), 3.50 (1H, dd, J=15.7, 6.1 Hz), 4.66, 4.94 (2H, AB-q, J=15.2 Hz), 5.20 (1H, t, J=6.1 Hz), 6.26 (1H, s), 6.86 (1H, s), 7.10-7.30 (4H, m), 7.50 (1H, s).

### Example 20: 2-(2,2-dimethylpropionyl)-N-(2-methyl-5-sulfamoylphenyl)-1,2,3,4-tetrahydroisoquinoline-(3S)-carboxamide

IR ν (Nujol) cm⁻¹; 3246, 1678, 1606, 1336, 1157.
¹H-NMR (CDCl₃) δ (ppm); 1.35 (9H, s), 2.19 (3H, s), 3.14 (1H, dd, J=15.6, 6.8 Hz), 3.40 (1H, dd, J=15.6, 6.8 Hz), 4.56, 5.00 (2H, AB-q, J=15.5 Hz), 4.90-5.10 (2H, br), 5.19 (1H, t, J=6.8 Hz), 7.10-7.30 (5H, m), 7.54 (1H, dd, J=8.0, 2.0 Hz), 8.14 (1H, br-s), 9.09 (1H, br-s).

### Example 21: N-(3-methanesulfonylamino-2,4,6-trimethylphenyl)-2-propionyl-1,2,3,4-tetrahydroisoquinoline-(3S)-carboxamide IR ν (Nujol) cm⁻¹; 3186, 1654, 1502.

¹H-NMR (CDCl₃) δ (ppm); 1.21 (3H, t, J=7.1Hz), 1.70, 1.88, 2.11 (9H, s, s, s), 2.40-2.70 (2H, m), 2.90 (3H, s), 3.05-3.20 (1H, m), 3.40-3.60 (1H, m), 4.55-4.90 (2H, m), 5.20-5.30 (1H, m), 6.30-7.50 (7H, m).

### Example 22: 2-[(2E,4E)-hexadienoyl]-N-(2,4,6-trimethylphenyl)-1,2,3,4-tetrahydroisoquinoline-(3S)-carboxamide

IR ν (Nujol) cm⁻¹; 3222, 1652, 1627, 1600.
¹H-NMR (CDCl₃) δ (ppm); 1.66 (6H, br-s), 1.81 (3H, d, J=6.0 Hz), 2.17 (3H, s), 2.8-3.2 (2H, m), 4.2-5.8 (3H, m), 5.9-6.3 (4H, m), 6.9-7.8 (7H, m).

### Example 23: 2-hexanoyl-N-(2-oxo-4-phenyl-1-propyl-1,2-dihydropyridin-3-yl)-1,2,3,4-tetrahydroisoquinoline-(3S)-carboxamide

IR ν (Nujol) cm⁻¹; 1651, 1097, 1217, 1180, 756, 698.
¹H-NMR (CDCl₃) δ (ppm); 0.96 (6H, br-t), 1.0-2.2 (8H, m), 2.2-2.7 (2H, m), 2.6-3.6 (2H, m), 3.89 (2H, t, J=7.2 Hz), 4.4-5.0 (2H, m), 5.0-5.2 (0.2H, m), 5.35 (0.8H, dd, J=5.9, 4.3 Hz), 6.18 (1H, d, J=7.1 Hz), 7.0-7.6 (9H, m, Ph-), 7.11 (1H, d, J=7.1 Hz), 7.8-8.2 (1H, m).

### Example 24: 2-(octane-1-sulfonyl)-N-(2,4,6-trimethylphenyl)-1,2,3,4-tetrahydroisoquinoline-(3S)-carboxamide

IR ν (Nujol) cm⁻¹; 3339, 1674, 1514.
¹H-NMR (CDCl₃) δ (ppm); 0.87 (3H, br-t), 1.02-2.00 (12H, m), 1.91, 2.20 (9H, s, s), 2.89 (2H, t, J=7.7 Hz), 3.08 (1H, dd, J=15.2, 5.5 Hz), 3.48 (1H, dd, J=15.2, 5.5 Hz), 4.46-4.78 (3H, m), 6.79 (2H, s), 7.06-7.38 (4H, m), 7.81 (1H, br-s).

### Example 25: 2-methanesulfonyl-N-(2,4,6-trimethylphenyl)-1,2,3,4-tetrahydroisoquinoline-(3S)-carboxamide

IR ν (Nujol) cm⁻¹; 3321, 1650.
¹H-NMR (DMSO-d₆) δ (ppm); 1.83 (6H, s), 2.17 (3H, s), 2.98 (3H, s), 3.2-3.4 (2H, m), 4.4-4.8 (3H, m), 6.77 (2H, s), 7.22 (4H, br-s), 9.09 (1H, br-s).

### Example 26: 2-octanoyl-N-(2,4,6-trimethylphenyl)-1,2,3,4-tetrahydroisoquinoline-(3R)-carboxamide

IR ν (Nujol) cm⁻¹; 3286, 3235, 1652, 1609, 1501.
¹H-NMR (CDCl₃) δ (ppm); 0.88 (3H, br-t), 1.10-2.00 (10H, m), 1.66, 1.91, 2.19 (9H, s, s, s), 2.30-2.66 (2H, m), 2.84-3.68 (2H, m), 4.40-5.09 (2H, m), 5.31 (1H, t, J=5.8 Hz), 6.77 (2H, s), 7.00-7.32 (4H, m), 7.32-7.66 (1H, br).

### Example 27: 2-benzyl-N-(2,4-difluorophenyl)-1,2,3,4-tetrahydroisoquinoline-3-carboxamide

### (1) 2-tert-butoxycarbonyl-1,2,3,4-tetrahydroisoquinoline-3-carboxylic acid

1,2,3,4-Tetrahydroisoquinoline-3-carboxylic acid hydrochloride was suspended in methanol (100 mL), triethylamine (16.3 mL) and a solution of di-tert-butyl bicarbonate (10.2 g) in methanol (50 mL) was added under ice-cooling, and the mixture was stirred at room temperature for 16 hr. After methanol was evaporated under reduced pressure, ethyl acetate (100 mL) was added to the residue, and the mixture was extracted with 1 M aqueous sodium hydroxide solution (100 mL). The aqueous layer was acidified with 6 M hydrochloric acid (12.5 mL) under ice-cooling and extracted with ethyl acetate (150 mL). The ethyl acetate layer was washed successively with 5% aqueous citric acid solution and saturated brine, and dried over sodium sulfate. Then, ethyl acetate was evaporated under reduced pressure to give the title compound as a powder (12.0 g).

### (2) 2-tert-butoxycarbonyl-N-(2,4-difluorophenyl)-1,2,3,4-tetrahydroisoquinoline-3-carboxamide

According to Example 2 (3), the title compound was obtained as a powder (2.13 g) from the compound (2.00 g) of (1) and 2,4-difluoroaniline (0.81 mL).
IR ν (Nujol) cm⁻¹; 1701, 1663, 1410, 1096, 966.
¹H-NMR (CDCl₃) δ (ppm); 1.51 (9H, s), 3.09 (1H, dd, J=15.3, 6.1 Hz), 3.39 (1H, dd, J=15.3, 4.1 Hz), 4.3-4.7 (2H, m), 4.7-5.1 (1H, m), 6.6-6.9 (2H, m), 7.1-7.4 (4H, m), 7.6-8.6 (2H, m).

### (3) N-(2,4-difluorophenyl)-1,2,3,4-tetrahydroisoquinoline-3-carboxamide

The compound (2.11 g) of (2) was dissolved in formic acid (10 mL), 8.8 M hydrogen chloride - isopropanol solution (1.2 mL) was added under ice-cooling, and the mixture was stirred at room temperature for 1 hr. The reaction mixture was alkalified with 5.5 M aqueous sodium hydroxide solution (50 mL) under ice-cooling and extracted with ethyl acetate (50 mL). The ethyl acetate layer was washed successively with saturated aqueous sodium hydrogen carbonate and saturated brine, and dried over sodium sulfate. Then, ethyl acetate was evaporated under reduced pressure to give the title compound as a powder (1.52 g).
IR ν (Nujol) cm⁻¹; 1692, 1533, 1429, 1285, 1196, 1140, 1096, 955, 853, 820, 748.
¹H-NMR (CDCl₃) δ (ppm); 1.75 (1H, br-s), 2.89 (1H, dd, J=16.3, 10.3 Hz), 3.33 (1H, dd, J=16.3, 5.5 Hz), 3.71 (1H, dd, J=10.3, 5.5 Hz), 4.04 (2H, s), 6.7-7.4 (6H, m), 8.2-8.6 (1H, m), 9.56 (1H, br-s).

### (4) 2-benzyl-N-(2,4-difluorophenyl)-1,2,3,4-tetrahydroisoquinoline-3-carboxamide

The compound (400 mg) of (3) was dissolved in N,N-dimethylformamide (4 mL), benzyl bromide (0.20 mL) and triethylamine (0.23 mL) were added, and the mixture was stirred at room temperature for 16.5 hr. 5% Aqueous citric acid solution was added to the reaction mixture, and the mixture was extracted with ethyl acetate (20 mL). The ethyl acetate layer was washed successively with 5% aqueous citric acid solution, saturated aqueous sodium hydrogen carbonate and saturated brine, and dried over sodium sulfate. Then, ethyl acetate was evaporated under reduced pressure. The residue was purified by column chromatography to give the title compound as a viscous oil (422 mg).
IR ν (neat) cm⁻¹; 1695, 1607, 1526, 1429, 1258, 1138, 1096, 961, 748, 698.
¹H-NMR (CDCl₃) δ (ppm); 3.20 (2H, d, J=6.8 Hz), 3.5-4.0 (3H, m), 3.74 (2H, s), 6.6-7.6 (11H, m), 8.1-8.4 (1H, m), 9.68 (1H, br-s).

### Example 28: 2-benzyl-N-(2,6-diisopropylphenyl)-1,2,3,4-tetrahydroisoquinoline-3-carboxamide

The title compound was synthesized according to Example 27.
IR ν (Nujol) cm⁻¹; 1654, 1531, 1224, 699.
¹H-NMR (DMSO-d₆) δ (ppm); 0.8-1.2 (12H, m), 2.4-3.0 (2H, m), 3.0-3.5 (2H, m), 3.5-4.2 (5H, m), 7.0-7.7 (12H, m), 9.31 (1H, br-s).

### Example 29: N-(2,4-difluorophenyl)-2-octyl-1,2,3,4-tetrahydroisoquinoline-3-carboxamide

The compound (500 mg) obtained in Example 27(3), octyl iodide (624 mg) and potassium carbonate (478 mg) were suspended in N,N-dimethylformamide (5 mL), and the solution was stirred at room temperature for 15 hr. Water was added to the reaction mixture, and the mixture was extracted with ethyl acetate (25 mL). The ethyl acetate layer was washed successively with water and saturated brine, and dried over sodium sulfate. Then, ethyl acetate was evaporated under reduced pressure. The residue was purified by column chromatography to give the title compound as a viscous oil (590 mg).
IR ν (neat) cm⁻¹; 1695, 1607, 1528, 1431, 1258, 1408, 1096, 962, 847, 746.
¹H-NMR (CDCl₃) δ (ppm); 0.86 (3H, br-t), 1.0-1.8 (12H, m), 2.59 (2H, br-t), 3.13 (2H, d, J=6.4 Hz), 3.51 (1H, t, J=6.4 Hz), 3.70, 3.99 (2H, AB-q, J=14.7 Hz), 6.6-7.0 (2H, m), 7.1-7.4 (4H, m), 8.1-8.5 (1H, m), 9.64 (1H, br-s).

### Example 30: N-(3-methylpyridin-2-yl)-2-octanoyl-1,2,3,4-tetrahydroisoquinoline-(3S)-carboxamide

### (1) 2-tert-butoxycarbonyl-N-(3-methylpyridin-2-yl)-1,2,3,4-tetrahydroisoquinoline-(3S)-carboxamide

2-tert-Butoxycarbonyl-1,2,3,4-tetrahydroisoquinoline-(3S)-carboxylic acid (1.00 g) was dissolved in tetrahydrofuran (10 mL), 1,1'-carbonyldiimidazole (928 mg) was added under ice-cooling, and the mixture was stirred at the same temperature for 1 hr. 2-Amino-3-picoline (0.44 mL) was added to the reaction mixture, and the mixture was further stirred at room temperature for 18 hr. After tetrahydrofuran was evaporated, ethyl acetate (20 mL) was added, and the mixture was washed with saturated brine and dried over sodium sulfate. Then, ethyl acetate was evaporated under reduced pressure. The residue was purified by column chromatography to give the title compound as an oil (482 mg).
IR ν (neat) cm⁻¹; 3269, 2976, 2930, 2868, 1693, 1582.
¹H-NMR (CDCl₃) δ (ppm); 1.52 (9H, s), 1.94 (3H, s), 3.13 (1H, dd, J=15.0, 5.9 Hz), 3.41 (1H, dd, J=15.0, 4.3 Hz), 4.40-4.66 (2H, m), 4.66-5.10 (1H, br), 6.90-7.34 (6H, m), 7.34-7.56 (1H, m), 8.10-8.30 (1H, m).

### (2) N-(3-methylpyridin-2-yl)-1,2,3,4-tetrahydroisoquinoline-(3S)-carboxamide

According to Example 27(3), the title compound was obtained as a powder (306 mg) from the compound (469 mg) of (1).
IR ν (Nujol) cm⁻¹; 3321, 3229, 3177, 3113, 1684, 1657, 1603, 1578, 1533.
¹H-NMR (CDCl₃) δ (ppm); 2.23 (3H, s), 2.72-3.48 (3H, m), 3.76 (1H, dd, J=9.8, 5.4 Hz), 4.06 (2H, s), 6.80-7.30 (6H, m), 7.53 (1H, d, J=7.2 Hz), 8.27 (1H, d, J=4.9 Hz).

### (3) N-(3-methylpyridin-2-yl)-2-octanoyl-1,2,3,4-tetrahydroisoquinoline-(3S)-carboxamide

The compound (289 mg) of (2) was dissolved in methylene chloride (3.0 mL), n-octanoyl chloride (0.20 mL) was added under ice-cooling, and the mixture was stirred at the same temperature for 1 hr. The reaction mixture was washed successively with saturated aqueous sodium hydrogen carbonate and saturated brine, and dried over sodium sulfate. Then, methylene chloride was evaporated under reduced pressure. The residue was purified by column chromatography to give the title compound as an oil (191 mg).
IR ν (neat) cm⁻¹; 3250, 2955, 2928, 2855, 1682, 1643, 1582.
¹H-NMR (CDCl₃) δ (ppm); 0.87 (3H, br-t), 1.09-2.30 (10H, m), 2.09 (3H, s), 2.30-2.68 (2H, m), 3.06 (1H, dd, J=15.6, 6.6 Hz), 3.51 (1H, dd, J=15.6, 5.0 Hz), 4.40-5.06 (2H, m), 5.37 (1H, dd, J=6.6, 5.0 Hz), 6.85-7.35 (5H, m), 7.46 (1H, d, J=7.0 Hz), 8.21 (1H, d, J=4.1 Hz), 8.53-8.87 (1H, br).

The compounds of Example 31 and Example 32 were synthesized according to Example 30.

### Example 31: 2-decanoyl-N-(3-methylpyridin-2-yl)-1,2,3,4-tetrahydroisoquinoline-(3S)-carboxamide

IR ν (neat) cm⁻¹; 3254, 2955, 2926, 2854, 1682, 1645, 1582.
¹H-NMR (CDCl₃) δ (ppm); 0.87 (3H, br-t), 1.03-1.91 (14H, m), 2.09 (3H, s), 2.30-2.68 (2H, m), 3.07 (1H, dd, J=17.1, 6.3 Hz), 3.51 (1H, dd, J=17.1, 5.0 Hz), 4.50-5.02 (2H, m), 5.37 (1H, br-t), 6.91-7.35 (5H, m), 7.46 (1H, d, J=4.4 Hz), 8.21 (1H, d, J=4.2 Hz), 8.60-8.89 (1H, br).

### Example 32: 2-pentanoylsulfamoyl-N-(2,4,6-trimethylphenyl)-1,2,3,4-tetrahydroisoquinoline-(3S)-carboxamide

IR ν (Nujol) cm⁻¹; 3389, 3344, 3136, 1717, 1663, 1510.
¹H-NMR (CDCl₃) δ (ppm); 0.83 (3H, br-t), 1.00-1.60 (4H, m), 1.79 (6H, s), 2.18 (3H, s), 1.90-2.32 (2H, m), 3.13 (1H, dd, J=15.2, 5.7 Hz), 3.52 (1H, dd, J=15.2, 3.2 Hz), 4.55 (2H, s), 5.10 (1H, dd, J=5.7, 3.2 Hz), 6.75 (2H, s), 7.07-7.39 (4H, m), 7.89 (1H, br-s), 9.45 (1H, br-s).

### Example 33: N-(2,6-diisopropylphenyl)-2-(2,2-dimethylpropyl)-1,2,3,4-tetrahydroisoquinoline-(3S)-carboxamide

### (1) N-(2,6-diisopropylphenyl)-1,2,3,4-tetrahydroisoquinoline-(3S)-carboxamide

According to Example 27 (2)-(3), the title compound was synthesized using 2,6-diisopropylaniline.
IR ν (Nujol) cm⁻¹; 1652, 1498, 1456, 802, 745.
¹H-NMR (CDCl₃) δ (ppm); 1.15 (12H, d, J=6.8 Hz), 1.84 (1H, br-s), 2.89 (2H, septet, J=6.8 Hz), 2.9-3.5 (2H, m), 3.83 (1H, dd, J=8.8, 5.8 Hz), 4.08 (2H, s), 7.0-7.5 (7H, m), 8.73 (1H, br-s).

### (2) N-(2,6-diisopropylphenyl)-2-(2,2-dimethylpropyl)-1,2,3,4-tetrahydroisoquinoline-(3S)-carboxamide

The compound (950 mg) of (1) was dissolved in methanol (10 mL), trimethylacetoaldehyde (3.2 mL) was added, and the mixture was stirred at room temperature for 1 hr. Sodium cyanoborohydride (394 mg) was added, and the mixture was further stirred at room temperature for 16 hr. After methanol was evaporated under reduced pressure, 1 M aqueous sodium hydroxide solution (25 mL) was added to the residue, and the mixture was extracted twice with ethyl acetate (25 mL). The ethyl acetate layers were combined, washed successively with 1 M aqueous sodium hydroxide solution and saturated brine, and dried over sodium sulfate. Then, ethyl acetate was evaporated under reduced pressure. The residue was purified by column chromatography to give the title compound as a viscous oil (802 mg).
IR ν (neat) cm⁻¹; 1738, 1668, 1487, 1256, 1084, 935, 799, 741.
¹H-NMR (CDCl₃) δ (ppm); 0.8-1.4 (21H, m), 2.55 (2H, s), 2.5-2.8 (2H, m), 3.0-3.5 (2H, m), 3.6-4.4 (3H, m), 7.0-7.4 (7H, m), 8.83 (1H, br-s).

### Example 34: N-(2,6-diisopropylphenyl)-2-dimethylaminoacetyl-1,2,3,4-tetrahydroisoquinoline-(3S)-carboxamide

### (1) 2-chloroacetyl-N-(2,6-diisopropylphenyl)-1,2,3,4-tetrahydroisoquinoline-(3S)-carboxamide

According to Example 30 (3), the title compound was obtained using the compound of Example 33 (1) and chloroacetyl chloride (0.10 mL).
IR ν (Nujol) cm⁻¹; 3248, 3033, 1659, 1642, 1588, 1533.
¹H-NMR (CDCl₃) δ (ppm); 1.01 (12H, d, J=6.8 Hz), 2.07-2.80 (2H, m), 2.90-3.73 (2H, m), 4.24 (2H, AB-q, J=11.8 Hz), 4.55-6.08 (2H, m), 5.30 (1H, br-t), 6.81-7.50 (8H, m).

### (2) N-(2,6-diisopropylphenyl)-2-dimethylaminoacetyl-1,2,3,4-tetrahydroisoquinoline-(3S)-carboxamide

The compound (620 mg) of (1) was suspended in ethanol (13 mL), 50% aqueous dimethylamine solution (203 mg) and potassium carbonate (622 mg) were added, and the mixture was stirred at room temperature for 2.5 hr. After ethanol was evaporated under reduced pressure, water was added, and the mixture was extracted with chloroform (20 mL). The chloroform layer was washed with saturated brine and dried over sodium sulfate. Chloroform was evaporated under reduced pressure to give the title compound as a powder (208 mg).
IR ν (Nujol) cm⁻¹; 3256, 3067, 2768, 1659, 1638, 1526.
¹H-NMR (CDCl₃) δ (ppm); 0.80-1.20 (12H, m), 2.20-2.80 (2H, m), 2.36 (6H, s), 2.89-3.71 (4H, m), 4.50-5.17 (2H, m), 5.38 (1H, br-t), 6.92-7.46 (8H, m).

### Example 35: N-(2,6-diisopropylphenyl)-2-(2-oxobutyryl)-1,2,3,4-tetrahydroisoquinoline-(3S)-carboxamide

The compound (700 mg) of Example 33 (1) and 2-oxobutyric acid (276 mg) were dissolved in methylene chloride (7 mL), 1-ethyl-3-(3-dimethylaminopropyl)carbodiimide hydrochloride (518 mg) was added under ice-cooling, and the mixture was stirred at the same temperature for 2 hr. After methylene chloride was evaporated under reduced pressure, 5% aqueous citric acid solution was added to the residue, and the mixture was extracted with ethyl acetate (15 mL), washed successively with 5% aqueous citric acid solution, saturated aqueous sodium hydrogen carbonate and saturated brine, and dried over sodium sulfate. Ethyl acetate was evaporated under reduced pressure. The obtained crystalline residue was dissolved by heating in ethyl acetate (3.5 mL), and the mixture was subjected to recrystallization from n-hexane (10.5 mL) to give the title compound as white crystals (450 mg).
IR ν (Nujol) cm⁻¹; 1719, 1647, 1516, 1429, 1105, 1022, 750, 741. ¹H-NMR (CDCl₃) δ (ppm); 0.8-1.5 (15H, m), 2.4-3.8 (6H, m), 4.2-5.2 (2H, m), 5.27(1H, br-t), 6.9-7.5 (8H, m).

### Example 36: 2-[(2S)-amino-3-phenylpropionyl]-N-(2,4,6-trimethylphenyl)-1,2,3,4-tetrahydroisoquinoline-(3S)-carboxamide

### (1) N-(2,4,6-trimethylphenyl)-1,2,3,4-tetrahydroisoquinoline-(3S)-carboxamide

According to Example 27 (2)-(3), the title compound was synthesized using 2,4,6-trimethylaniline.
IR ν (Nujol) cm⁻¹; 3317, 3254, 1659, 1610, 1533.
¹H-NMR (CDCl₃) δ (ppm); 1.81 (1H, s), 2.11 (6H, s), 2.25 (3H, s), 2.97 (1H, dd, J=15.4, 9.3 Hz), 3.31 (1H, dd, J=15.4, 5.5 Hz), 3.77 (1H, dd, J=9.3, 5.5 Hz), 4.06 (2H, s), 6.86 (2H, s), 7.00-7.37 (4H, m), 8.50-8.75 (1H, br).

### (2) tert-butyl {1-benzyl-2-oxo-2-[(3S)-(2,4,6-trimethylphenylcarbamoyl)-1,2,3,4-tetrahydroisoquinolin-2-yl]ethyl}carbamate

According to Example 2 (3), the title compound was obtained as a powder (1.81 g) from the compound (990 mg) of (1) and Boc-L-phenylalanine (981 mg).
IR ν (Nujol) cm⁻¹; 3279, 1682, 1639, 1514.
¹H-NMR (CDCl₃) δ (ppm); 1.36, 1.45 (9H, s, s), 1.64 (6H, s), 1.74 (3H, s), 2.19 (2H, d, J=4.8 Hz), 2.95 (1H, dd, J=15.3, 6.3 Hz), 3.14 (1H, t, J=2.7 Hz), 3.47 (1H, dd, J=15.3, 2.7 Hz), 4.08-4.87 (2H, m), 4.87-5.52 (2H, m), 6.65-6.82 (2H, m), 6.82-7.40 (10H, m).

### (3) 2-[(2S)-amino-3-phenylpropionyl]-N-(2,4,6-trimethylphenyl)-1,2,3,4-tetrahydroisoquinoline-(3S)-carboxamide

The compound (1.79 g) of (2) was dissolved in chloroform (18 mL), 8.8M hydrogen chloride - isopropanol solution (3.76 mL) was added under ice-cooling, and the mixture was stirred at room temperature for 1 hr. The reaction mixture was neutralized with saturated aqueous sodium hydrogen carbonate and extracted with chloroform (40 mL). The chloroform layer was washed with saturated brine and dried over sodium sulfate. Then, chloroform was evaporated under reduced pressure. The residue was purified by column chromatography to give the title compound as crystals (608 mg).
IR ν (Nujol) cm⁻¹; 3379, 3223, 3184, 1641, 1609, 1537.
¹H-NMR (CDCl₃) δ (ppm); 1.69 (6H, s), 1.73 (3H, s), 2.19 (2H, d, J=4.4 Hz), 2.77-3.63 (3H, m), 3.89-4.80 (4H, m), 5.25 (1H, dd, J=5.4, 3.6 Hz), 6.65-6.80 (2H, m), 6.80-7.46 (10H, m).

### Example 37: 2-[(2S)-amino-(3S)-methylpentanoyl]-N-(2,4,6-trimethylphenyl)-1,2,3,4-tetrahydroisoquinoline-(3S)-carboxamide

The title compound was synthesized according to Example 36.
IR ν (Nujol) cm⁻¹; 3269, 1645, 1589.
¹H-NMR (CDCl₃) δ (ppm); 0.70-1.20 (6H, m), 1.20-1.90 (5H, m), 1.77 (3H, s), 1.94 (3H, s), 2.19 (3H, s), 3.08 (1H, dd, J=14.6, 5.9 Hz), 3.36-3.82 (2H, m), 4.60-4.92 (2H, m), 4.92-5.10 (0.5H, m), 5.30 (0.5H, t, J=7.2 Hz), 6.76 (1H, s), 6.78 (1H, s), 7.01-7.45 (4H, m), 7.90-8.20 (1H, br).

### Example 38: 4-{{2-[(2E),(4E)-hexadienoyl]-1,2,3,4-tetrahydroisoquinoline-(3S)-carbonyl}amino}-3-methylbenzoic acid

### (1) methyl 4-{{2-[(2E),(4E)-hexadienoyl]-1,2,3,4-tetrahydroisoquinoline-(3S)-carbonyl}amino}-3-methylbenzoate

The title compound was synthesized according to Example 30.
¹H-NMR (CDCl₃) δ (ppm); 1.89 (3H, d, J=6.7 Hz), 2.24 (3H, s), 2.95-3.10 (0.8H, m), 3.20-3.30 (0.2H, m), 3.40-3.60 (1H, m), 3.86 (3H, s), 4.45-4.65, 4.55 (1H, m, d, J=15.0 Hz), 4.80 (0.8H, d, J=15.0 Hz), 4.85-4.95 (0.2H, m), 5.00-5.10 (0.2H, m), 5.45 (0.8H, br-t), 6.10-6.40 (2H, m), 6.39 (1H, d, J=14.5 Hz), 7.13 (1H, d, J=7.1 Hz), 7.15-7.35 (3H, m), 7.42 (1H, dd, J=14.5, 10.5Hz), 7.70-7.85 (2H, m), 8.14 (1H, d, J=8.3 Hz), 9.08 (1H, br-s).

### (2) 4-{{2-[(2E),(4E)-hexadienoyl]-1,2,3,4-tetrahydroisoquinoline-(3S)-carbonyl}amino}-3-methylbenzoic acid

The compound (162 mg) of (1) was dissolved in a mixed solution of tetrahydrofuran-methanol (3:1, 2.5 mL), 1 M aqueous lithium hydroxide solution (0.98 mL) was added at room temperature, and the mixture was stirred at the same temperature for 30 min, and then 40°C for 3.5 hr. After acidifying with 10% aqueous citric acid solution, tetrahydrofuran - methanol was evaporated under reduced pressure. Diethyl ether (10 mL) was added, and the mixture was stirred at room temperature for 30 min. The precipitate was collected by filtration to give the title compound as a powder (104 mg).
IR ν (Nujol) cm⁻¹; 1699, 1651, 1418, 1001, 970, 770, 743.
¹H-NMR (CDCl₃+DMSO-d₆) δ (ppm); 1.86 (3H, d, J=6.1 Hz), 2.18 (3H, s), 2.95-3.05 (0.2H, m), 3.15-3.25 (0.8H, m), 3.40-3.60 (1H, m), 4.45-4.65, 4.54 (1H, m, d, J=15.0 Hz), 4.78 (0.8H, d, J=15.0 Hz), 4.85-4.95 (0.2H, m), 4.95-5.05 (0.2H, m), 5.42 (0.8H, br-t), 6.10-6.40 (2H, m), 6.36 (1H, d, J=14.8 Hz), 7.10 (1H, d, J=7.1 Hz), 7.15-7.35 (3H, m), 7.38 (1H, dd, J=14.8, 10.2 Hz), 7.70-7.85 (2H, m), 8.06 (1H, d, J=8.3 Hz), 9.00 (1H, br-s).

The compounds of Example 39-Example 45 were synthesized according to Example 38.

### Example 39: 4-{{2-[(2E),(4E)-octadienoyl]-1,2,3,4-tetrahydroisoquinoline-(3S)-carbonyl}amino}-3-methylbenzoic acid

IR ν (Nujol) cm⁻¹; 1670, 1651, 1587, 1526, 1196, 1001, 746.
¹H-NMR (CDCl₃+DMSO-d₆) δ (ppm); 0.94 (3H, br-t), 1.40-1.55 (2H, m), 2.10-2.25 (2H, m), 2.22 (3H, s), 2.95-3.10 (0.8H, m), 3.20-3.30 (0.2H, m), 3.40-3.60 (1H, m), 4.50-4.65, 4.58 (1H, m, d, J=15.3 Hz), 4.81 (0.8H, d, J=15.3 Hz), 4.90-5.00 (0.2H, m), 5.00-5.10 (0.2H, m), 5.43 (0.8H, br-t), 6.10-6.35 (2H, m), 6.42 (1H, d, J=14.4 Hz), 7.14 (1H, d, J=6.6 Hz), 7.15-7.35 (3H, m), 7.41 (1H, dd, J=14.4, 10.6 Hz), 7.70-7.85 (2H, m), 8.08 (1H, d, J=8.1 Hz), 9.04 (1H, br-s).

### Example 40: 3,5-diisopropyl-4-{[2-(2,2-dimethylbutyryl)-1,2,3,4-tetrahydroisoquinoline-(3S)-carbonyl]amino}benzoic acid

IR ν (Nujol) cm⁻¹; 3296, 1697, 1664, 1594, 1583.
¹H-NMR (CDCl₃) δ (ppm); 0.88 (3H, t, J=7.6 Hz), 0.95-1.15 (12H, m), 1.20-1.40 (6H, m), 1.80 (2H, t, J=7.6 Hz), 2.60-3.00 (2H, m), 3.12 (1H, dd, J=15.6, 6.4 Hz), 4.47 (1H, dd, J=15.6, 5.4 Hz), 4.67, 5.00 (2H, AB-q, J=15.6 Hz), 5.28 (1H, dd, J=6.4, 5.4 Hz), 7.10-7.30 (4H, m), 7.75 (2H, s), 7.94 (1H, s).

### Example 41: 4-{[2-(2,2-dimethylpropionyl)-1,2,3,4-tetrahydroisoquinoline-(3S)-carbonyl]amino}-3-methylbenzoic acid

IR ν (Nujol) cm⁻¹; 1682, 1589, 1184, 741.
¹H-NMR (CDCl₃) δ (ppm); 1.38 (9H, s), 2.20 (3H, s), 3.08 (1H, dd, J=15.9, 6.9 Hz), 3.54 (1H, dd, J=15.9, 5.8 Hz), 4.50, 5.00 (2H, AB-q, J=15.5 Hz), 5.0-7.0 (1H, br), 5.29 (1H, dd, J=6.9, 5.8 Hz), 7.0-7.4 (4H, m), 7.7-8.0 (2H, m), 8.1-8.3 (1H, m), 8.60 (1H, s).

### Example 42: {3-{[2-(2,2-dimethylpropionyl)-1,2,3,4-tetrahydroisoquinoline-(3S)-carbonyl]amino}-2,4,6-trimethylphenyl}acetic acid

IR ν (Nujol) cm⁻¹; 1759, 1666, 1582, 1537, 1418, 1354, 1171, 754.
¹H-NMR (CDCl₃+DMSO-d₆) δ (ppm); 1.39 (9H, s), 1.96 (6H, br-s), 2.24 (3H, s), 3.16 (1H, dd, J=15.7, 6.5 Hz), 3.47 (1H, dd, J=15.7, 5.0 Hz), 3.58 (2H, s), 4.68, 4.95 (1H, AB-q, J=16.2 Hz), 5.20 (1H, dd, J=6.5, 5.0 Hz), 6.85 (1H, s), 7.15-7.30 (4H, m), 7.80 (1H, br-s).

### Example 43: {3-{[2-(2,2-dimethylbutyryl)-1,2,3,4-tetrahydroisoquinoline-(3S)-carbonyl]amino}-2,4,6-trimethylphenyl}acetic acid

IR ν (Nujol) cm⁻¹; 1728, 1661, 1589, 1408, 1232, 1171, 754.
¹H-NMR (CDCl₃) δ (ppm); 0.86 (3H, t, J=7.4 Hz), 1.32 (6H, s), 1.70-1.90 (2H, m), 1.93, 1.97 (6H, br-s, br-s), 2.21 (3H, s), 3.11 (1H, dd, J=15.6, 6.4 Hz), 3.35-3.50 (1H, m), 3.59 (2H, s), 4.62, 5.00 (2H, AB-q, J=15.6 Hz), 5.00-5.20 (1H, m), 6.82 (1H, s), 7.10-7.30 (4H, m), 8.00 (1H, br-s).

### Example 44: 2,4,6-trimethyl-3-{[2-(2,2-dimethylpropionyl)-1,2,3,4-tetrahydroisoquinoline-(3S)-carbonyl]amino}benzoic acid

IR ν (Nujol) cm⁻¹; 3256, 1720, 1664, 1589, 1533.
¹H-NMR (DMSO-d₆) δ (ppm); 1.29 (9H, s), 1.88 (3H, s), 1.93 (3H, s), 2.17 (3H, s), 3.10-3.30 (2H, m), 3.50-5.50 (1H, br), 4.58, 4.95 (2H, AB-q, J=16.3 Hz), 4.90-5.10 (1H, m), 6.87 (1H, s), 7.22 (4H, br-s), 9.15 (1H, s).

### Example 45: 3-{[2-(2,2-dimethylbutyryl)-1,2,3,4-tetrahydroisoquinoline-(3S)-carbonyl]amino}-2,4,6-trimethylbenzoic acid

IR ν (Nujol) cm-¹ ; 3280, 1718, 1668, 1587.
¹H-NMR (DMSO-d₆) δ (ppm); 0.79 (3H, t, J=7.3 Hz), 1.24 (6H, s), 1.72 (2H, q, J=7.3 Hz), 1.90 (3H, s), 1.94 (3H, s), 2.16 (3H, s), 3.10-3.30 (2H, m), 3.50-6.60 (1H, br), 4.58, 4.95 (2H, AB-q, J=15.8 Hz), 4.90-5.20 (1H, m), 6.84 (1H, s), 7.22 (4H, br-s), 9.18 (1H, s).

### Example 46: 4-{[2-(2,2-dimethylbutyryl)-1,2,3,4-tetrahydroisoquinoline-(3S)-carbonyl]amino}-3,5-diisopropyl-N,N-dimethylbenzamide

Dimethylamine hydrochloride (102 mg) was dissolved in methylene chloride (3 mL), triethylamine (0.13 mL) was added under ice-cooling, and the mixture was stirred at room temperature for 15 min. The compound (300 mg) of Example 40 and 1-ethyl-3-(3-dimethylaminopropyl)carbodiimide hydrochloride (156 mg) were added, and the mixture was stirred at the same temperature for 1 hr. Ethyl acetate was added to the reaction solution, and the mixture was washed successively with 5% aqueous citric acid solution, 5% sodium bicarbonate water and saturated brine, and dried over sodium sulfate. Ethyl acetate was evaporated under reduced pressure, and the obtained residue was purified by column chromatography to give the title compound (310 mg).
IR ν (Nujol) cm⁻¹; 3249, 1687, 1637, 1595, 1516.
¹H-NMR (CDCl₃) δ (ppm); 0.87 (3H, t, J=7.6 Hz), 0.95-1.15 (12H, m), 1.35, 1.38 (6H, s, s), 1.70-1.90 (2H, m), 2.60-2.90 (2H, br), 2.93 (3H, s), 3.08 (3H, s), 3.05-3.20 (1H, m), 3.54 (1H, dd, J=15.6, 5.4 Hz), 4.67, 4.94 (2H, AB-q, J=15.4 Hz), 5.26 (1H, t, J=5.6 Hz), 7.10-7.30 (6H, m), 7.45 (1H, s).

### Example 47: N-(3,5-di-tert-butyl-4-hydroxyphenyl)-2-(2,2-dimethylpropyl)-1,2,3,4-tetrahydroisoquinoline-(3S)-carboxamide

### (1) methyl 2-(2,2-dimethylpropyl)-1,2,3,4-tetrahydroisoquinoline-(3S)-carboxylate

To 0.45 M of borane-tetrahydrofuran solution (100 mL) was added a solution of the compound (6.00 g) of Example 2 (1) in tetrahydrofuran (24 mL), and the mixture was stirred at 50-52°C for 0.5 hr. 6M hydrochloric acid (50 mL) was added, and the mixture was stirred for 0.5 hr, neutralized with saturated aqueous sodium hydrogen carbonate, and extracted with ethyl acetate (300 mL). The ethyl acetate layer was washed with water and dried over sodium sulfate. Then, ethyl acetate was evaporated under reduced pressure. The residue was purified by column chromatography to give the title compound as an oil (3.40 g).
IR ν (neat) cm⁻¹; 2950, 1737.
¹H-NMR (CDCl₃) δ (ppm); 0.89 (9H, s), 2.40, 2.55 (2H, AB-q, J=13.8 Hz), 3.1-3.3 (2H, m), 3.5-3.7 (1H, m), 3.61 (3H, s), 3.89, 4.23 (2H, AB-q, J=16.3 Hz), 5.7-6.2 (4H, m).

### (2) 2-(2,2-dimethylpropyl)-1,2,3,4-tetrahydroisoquinoline-(3S)-carboxylic acid

The compound (4.40 g) of (1) was dissolved in methanol (50 mL), a solution of sodium hydroxide (1.04 g) in water (20 mL) was added, and the mixture was stirred at 60°C for 1 hr. Methanol was evaporated, and the residue was neutralized with 10% aqueous citric acid solution and extracted with chloroform (100 mL). The chloroform layer was washed with water and dried over sodium sulfate. Then, chloroform was evaporated under reduced pressure. The residue was purified by column chromatography to give the title compound as an oil (2.20 g).
IR ν (neat) cm⁻¹; 2864, 1714, 1622.
¹H-NMR (CDCl₃) δ (ppm); 0.98 (9H, s), 2.48, 2.73 (2H, AB-q, J=13.7 Hz), 3.1-3.4 (2H, m), 3.5-3.8 (1H, m), 3.93, 4.21 (2H, AB-q, J=15.0 Hz), 5.7-6.2 (4H, m), 7.70 (1H, br-s).

### (3) N-(3,5-di-tert-butyl-4-hydroxyphenyl)-2-(2,2-dimethylpropyl)-1,2,3,4-tetrahydroisoquinoline-(3S)-carboxamide

The compound (1.00 g) of Reference Example 1 was dissolved in toluene (20 mL), 10% palladium carbon (100 mg) was added, and the mixture was subjected to catalytic hydrogenation at room temperature for 3 hr under 3kgf/cm². Separately, to a solution of the compound (984 mg) of (2) in methylene chloride (30 mL) was added 1-ethyl-3-(3-dimethylaminopropyl)carbodiimide hydrochloride (839 mg) under ice-cooling, and the mixture was stirred at the same temperature for 1 hr. The earlier reaction mixture was added under ice-cooling while removing palladium carbon by filtration, and the mixture was stirred at the same temperature for 1 hr. The reaction mixture was washed with water and dried over sodium sulfate, and solvent was evaporated under reduced pressure. The residue was purified by column chromatography to give the title compound as an oil (650 mg).
IR ν (neat) cm⁻¹; 3639, 3315, 1733.
¹H-NMR (CDCl₃) δ (ppm); 0.99 (9H, s), 1.42 (18H, s), 2.39 (2H, s), 3.18 (2H, d, J=6.0 Hz), 3.57 (1H, t, J=6.0 Hz), 3.70, 4.07 (2H, AB-q, J=15.4 Hz), 5.01 (1H, s), 5.9-6.4 (6H, m), 9.27 (1H, br-s).

### Example 48: 2-(2,2-dimethylpropyl)-N-(4-hydroxy-2,3,5-trimethylphenyl)-1,2,3,4-tetrahydroisoquinoline-(3S)-carboxamide hydrochloride

### (1) N-(4-acetoxy-2,3,5-trimethylphenyl)-2-(2,2-dimethylpropyl)-1,2,3,4-tetrahydroisoquinoline-(3S)-carboxamide

According to Example 2 (3), the title compound was obtained as a powder (289 mg) from the compound (370 mg) of Example 47 (2) and the compound (348 mg) of Reference Example 15.
¹H-NMR (CDCl₃) δ (ppm); 0.98 (9H, s), 2.00, 2.04, 2.08 (9H, s, s, s), 2.31 (3H, s), 2.48 (2H, s), 3.0-3.4 (2H, m), 3.5-4.3 (3H, m), 6.9-7.3 (4H, m), 7.57 (1H, s), 9.27 (1H, br-s).

### (2) 2-(2,2-dimethylpropyl)-N-(4-hydroxy-2,3,5-trimethylphenyl)-1,2,3,4-tetrahydroisoquinoline-(3S)-carboxamide hydrochloride

The compound (278 mg) of (1) was dissolved in a mixed solution of tetrahydrofuran - methanol (3:1, 8 mL), 1 M aqueous lithium hydroxide solution (2.0 mL) was added at room temperature, and the mixture was stirred at the same temperature for 1.5 hr. After neutralization with 10% aqueous citric acid solution, tetrahydrofuran - methanol was evaporated under reduced pressure, and the residue was extracted twice with diethyl ether (10 mL). The diethyl ether layers were combined, washed successively with saturated aqueous sodium hydrogen carbonate and saturated brine, and dried over sodium sulfate. Then, diethyl ether was evaporated under reduced pressure.

The residue (0.25 g) was suspended in methanol (1.0 mL), 10M hydrogen chloride-isopropanol solution (0.1 mL) was added under ice-cooling, and the mixture was stirred at the same temperature for 10 min. Diethyl ether (10 mL) was added to the reaction mixture, and the precipitate was collected by filtration to give the title compound as a powder (223 mg).
IR ν (Nujol) cm⁻¹; 1668, 1545, 1221, 1202, 1099, 976, 756.
¹H-NMR (DMSO-d₆) δ (ppm); 1.11 (9H, s), 2.02 (3H, s), 2.11 (6H, s), 2.7-4.0 (5H, m), 4.4-5.0 (3H, m), 6.82 (1H, s), 7.1-7.5 (4H, m), 10.0-11.0 (1H, br).

The compounds of Example 49 and Example 50 were synthesized according to Example 48.

### Example 49: N-(4-hydroxy-2,3,5-trimethylphenyl)-2-octanoyl-1,2,3,4-tetrahydroisoquinoline-(3S)-carboxamide

IR ν (Nujol) cm⁻¹; 1647, 1522, 1225, 1097, 748.
¹H-NMR (CDCl₃) δ (ppm); 0.87 (3H, br-t), 0.9-2.2 (13H, m), 2.04 (6H, s), 2.51 (2H, br-t), 2.8-3.4 (1H, m), 3.51 (1H, dd, J=15.4, 4.1 Hz), 4.3-5.4 (3H, m), 6.46 (1H, br-s), 6.95 (1H, s), 7.0-7.4 (4H, m), 7.86 (1H, br-s).

### Example 50: N-(4-hydroxy-2,3,5-trimethylphenyl)-2-isobutyryl-1,2,3,4-tetrahydroisoquinoline-(3S)-carboxamide

IR ν (Nujol) cm⁻¹; 3300, 1670, 1634, 124,4, 1207, 1088.
¹H-NMR (CDCl₃) δ (ppm); 1.10-1.30 (6H, m), 1.40-1.70 (1H, m), 1.90-2.20 (8H, m), 2.80-2.90 (0.3H, m), 2.95-3.10 (1.4H, m), 3.20 (0.3H, br-dd), 3.52 (1H, dd, J=15.5, 4.0 Hz), 4.52, 4.99 (0.6H, AB-q, J=16.2 Hz), 4.55, 4.75 (1.4H, AB-q, J=15.0 Hz), 4.60-4.90 (1H, m), 4.80-4.90 (0.3H, m), 5.31 (0.7H, br-t), 6.51 (0.3H, br-d), 6.95-7.30 (5H, m), 7.93 (0.7H, br-s).

### Example 51: N-(3,5-di-tert-butyl-4-hydroxyphenyl)-2-pentyl-1,2,3,4-tetrahydroisoquinoline-(3S)-carboxamide

The title compound was synthesized according to Example 47.
IR ν (neat) cm⁻¹; 3639, 3308, 3003, 2957, 2872, 1734, 1668, 1603, 1531.
¹H-NMR (CDCl₃) δ (ppm); 0.90 (3H, br-t), 1.08-1.80 (6H, m), 1.42 (18H, s), 2.58 (2H, t, J=7.4 Hz), 3.12 (2H, d, J=6.6 Hz), 3.52 (1H, br-t), 3.97, 4.03 (2H, AB-q, 11.4 Hz), 5.01 (1H, s), 7.00-7.29 (4H, m), 7.31 (2H, s), 9.00-9.20 (1H, br).

### Example 52: N-(4-amino-2,6-diisopropylphenyl)-2-octanoyl-1,2,3,4-tetrahydroisoquinoline-(3S)-carboxamide hydrochloride

### (1) 2-octanoyl-1,2,3,4-tetrahydroisoquinoline-(3S)-carboxylic acid

The title compound was synthesized according to Example 2 (1)-(2).
IR ν (neat) cm⁻¹; 3029, 2927, 2856, 2576, 1951, 1914, 1733, 1652, 1607, 1587.
¹H-NMR (CDCl₃) δ (ppm); 0.88 (3H, br-t), 1.10-1.85 (10H, m), 2.20-2.60 (2H, m), 2.90-3.50 (2H, m), 4.30-5.05 (2H, m), 5.40 (1H, t, J=5.2 Hz), 6.98-7.42 (4H, m), 8.80 (1H, br-s).

### (2) N-(2,6-diisopropyl-4-nitrophenyl)-2-octanoyl-1,2,3,4-tetrahydroisoquinoline-(3S)-carboxamide

The compound (600 mg) of (1) and 2,6-diisopropyl-4-nitroaniline [synthesized according to the method described in Chem. Europ. J., 13, 2847-2859 (2002)] (396 mg) were dissolved in methylene chloride (6.0 mL), pyridine (0.48 mL) and phosphorus oxychloride (0.24 mL) were added under ice-cooling, and the mixture was stirred at the same temperature for 3 hr. The reaction mixture was washed successively with 10% aqueous citric acid solution, saturated aqueous sodium hydrogen carbonate and saturated brine, and dried over sodium sulfate. Then, methylene chloride was evaporated under reduced pressure. The residue was purified by column chromatography to give the title compound as a powder (812 mg).
IR ν (Nujol) cm⁻¹; 3377, 3263, 2727, 1738, 1622, 1589, 1504. ¹H-NMR (CDCl₃) δ (ppm); 0.68-1.90 (13H, m), 1.06 (12H, d, J=6.6 Hz), 2.40-3.73 (6H, m), 4.43-5.45 (3H, m), 7.08-7.41 (4H, m), 7.70 (1H, s), 7.92 (2H, s).

### (3) N-(4-amino-2,6-diisopropylphenyl)-2-octanoyl-1,2,3,4-tetrahydroisoquinoline-(3S)-carboxamide hydrochloride

The compound (795 mg) of (2) was dissolved in methanol (10 mL), 10% palladium carbon (160 mg) was added, and the mixture was subjected to catalytic hydrogenation at 40°C for 2.5 hr under 3.0 kgf/cm². After removing palladium carbon by filtration, methanol was evaporated under reduced pressure. The residue was dissolved in ethyl acetate (20 mL), and the solution was washed successively with saturated aqueous sodium hydrogen carbonate and saturated brine, and dried over sodium sulfate. Then, ethyl acetate was evaporated under reduced pressure. The residue was purified by column chromatography.

The residue was dissolved in methanol (2.6 mL), 10M hydrogen chloride-isopropanol solution (0.13 mL) was added dropwise, and methanol was evaporated under reduced pressure. Diethyl ether was added, and then the precipitate was collected by filtration to give the title compound as a powder (453 mg).
IR ν (Nujol) cm⁻¹; 3420, 3177, 2613, 1636, 1609, 1585, 1558, 1539.
¹H-NMR (DMSO-d₆) δ (ppm); 0.35-1.80 (25H, m), 2.10-4.00 (6H, m), 4.38-5.32 (3H, m), 7.03 (2H, s), 7.10-7.50 (4H, m), 8.91 (1H, s), 8.60-11.5 (3H, m).

### Example 53: 2-(2,2-dimethylpropionyl)-N-(3-sulfamoylamino-2,4,6-trimethylphenyl)-1,2,3,4-tetrahydroisoquinoline-(3S)-carboxamide

### (1) N-(3-tert-butoxycarbonylsulfamoylamino-2,4,6-trimethylphenyl)-2-(2,2-dimethylpropionyl)-1,2,3,4-tetrahydroisoquinoline-(3S)-carboxamide

According to Example 2, the title compound was synthesized from the compound of Reference Example 6.
IR ν (Nujol) cm⁻¹; 3369, 1718, 1656, 1614, 1236, 1150.
¹H-NMR (DMSO-d₆) δ (ppm); 1.29 (9H, s), 1.43 (9H, s), 1.91 (3H, s), 1.93 (3H, s), 2.22 (3H, s), 3.00-3.60 (2H, m), 4.57, 4.96 (2H, AB-q, J=18.0 Hz), 4.70-5.20 (1H, m), 6.88 (1H, s), 7.00-7.40 (4H, m), 9.14 (1H, s), 9.29 (1H, s), 10.86 (1H, s).

### (2) 2-(2,2-dimethylpropionyl)-N-(3-sulfamoylamino-2,4,6-trimethylphenyl)-1,2,3,4-tetrahydroisoquinoline-(3S)-carboxamide

The compound (580 mg) of (1) was dissolved in formic acid (5.0 mL), 8.7M hydrogen chloride - isopropanol solution (0.17 mL) was added dropwise under ice-cooling, and the mixture was stirred at the same temperature for 10 min. Diethyl ether was added to the reaction solution, and the precipitate was removed by filtration. The solvent of the filtrate was evaporated under reduced pressure, and the obtained residue was purified by column chromatography. Then, the obtained residue was washed with isopropanol to give the title compound as a powder (142 mg).
IR ν (Nujol) cm⁻¹; 3263, 1726, 1666, 1614, 1330, 1159.
¹H-NMR (DMSO-d₆) δ (ppm); 1.32 (9H, s), 1.88 (3H, s), 2.01 (3H, s), 2.28 (3H, s), 3.20-3.40 (2H, m), 4.59, 4.93 (2H, AB-q, J=15.9 Hz), 4.80-5.20 (1H, m), 6.69 (2H, s), 6.84 (1H, s), 7.15-7.25 (4H, m), 8.21 (1H, s), 9.12 (1H, s).

### Example 54: N-(4-hydroxy-2,3,5-trimethylphenyl)-2-pentyl-1,2,3,4-tetrahydroisoquinoline-(3S)-carboxamide hydrochloride

### (1) N-(4-acetoxy-2,3,5-trimethylphenyl)-2-pentyl-1,2,3,4-tetrahydroisoquinoline-(3S)-carboxamide

The title compound was obtained according to Example 29. IR ν (Nujol) cm⁻¹; 1749, 1661, 1528, 1231, 1120, 1082.
¹H-NMR (CDCl₃) δ (ppm); 0.89 (3H, br-t), 0.9-2.0 (6H, m), 1.84 (3H, s), 2.01, 2.08 (6H, s, s), 2.30 (3H, s), 2.68 (2H, br-t), 3.14 (2H, d, J=6.1 Hz), 3.4-4.1 (3H, m), 7.0-7.4 (4H, m), 7.52 (1H, s), 8.23 (1H, br-s).

### (2) N-(4-hydroxy-2,3,5-trimethylphenyl)-2-pentyl-1,2,3,4-tetrahydroisoquinoline-(3S)-carboxamide hydrochloride

The compound (930 mg) of (1) was dissolved in a mixed solution of tetrahydrofuran-methanol (1:1, 30 mL), 1 M aqueous lithium hydroxide solution (6.6 mL) was added, and the mixture was stirred at room temperature for 30 min. After the reaction mixture was neutralized with 10% aqueous citric acid solution, tetrahydrofuran - methanol was evaporated under reduced pressure, and the residue was extracted with ethyl acetate (20 mL). The ethyl acetate layer was washed successively with saturated aqueous sodium hydrogen carbonate and saturated brine, and dried over sodium sulfate. Then, ethyl acetate was evaporated under reduced pressure.

The obtained oily residue was dissolved in methanol (2 mL), 10M hydrogen chloride - isopropanol solution (0.30 mL) was added, and the mixture was stirred for 10 min. Then, diethyl ether (30 mL) was added to the reaction mixture, and the precipitate was collected by filtration to give the title compound as a powder (830 mg).
IR ν (Nujol) cm⁻¹; 1672, 1549, 1219, 1196, 1097, 984, 748.
¹H-NMR (DMSO-d₆) δ (ppm); 0.90 (3H, br-t), 1.0-2.2 (6H, m), 2.03 (3H, s), 2.11 (6H, s), 3.0-3.8 (4H, m), 4.3-4.8 (3H, m), 6.78 (1H, s), 7.1-7.5 (4H, m), 7.8-8.4 (1H, br), 10.0-10.8 (1H, m).

### Example 55: 2-(3-aminobenzyl)-N-(4-hydroxy-2,3,5-trimethylphenyl)-1,2,3,4-tetrahydroisoquinoline-(3S)-carboxamide dihydrochloride

### (1) N-(4-acetoxy-2,3,5-trimethylphenyl)-2-(3-nitrobenzyl)-1,2,3,4-tetrahydroisoquinoline-(3S)-carboxamide

The title compound was obtained according to Example 27. IR ν (Nujol) cm⁻¹; 1757, 1686, 1528, 1348, 1194, 1080.
¹H-NMR (CDCl₃) δ (ppm); 1.92 (3H, s), 2.03, 2.08 (6H, s, s), 2.31 (3H, s), 3.24 (2H, d, J=6.4 Hz), 3.6-4.0 (5H, m), 7.0-7.4 (4H, m), 7.4-7.8 (2H, m), 7.51 (1H, s), 8.1-8.4 (2H, m), 8.92 (1H, br-s).

### (2) N-(4-hydroxy-2,3,5-trimethylphenyl)-2-(3-nitrobenzyl)-1,2,3,4-tetrahydroisoquinoline-(3S)-carboxamide

The compound (1.25 g) of (1) was dissolved in a mixed solution of tetrahydrofuran - methanol (1:1, 50 mL), 1 M aqueous lithium hydroxide solution (7.7 mL) was added, and the mixture was stirred at room temperature for 2.5 hr. After the reaction mixture was neutralized with 10% aqueous citric acid solution, tetrahydrofuran - methanol was evaporated under reduced pressure, and the precipitate was collected by filtration. The obtained powder was dissolved in chloroform, and the solution was washed with saturated brine and dried over sodium sulfate. Then, chloroform was evaporated under reduced pressure. The obtained residue was washed with diisopropyl ether, and the precipitate was collected by filtration to give the title compound as a powder (900 mg).
IR ν (Nujol) cm⁻¹; 1649, 1533, 1510, 1352, 1229, 1096.
¹H-NMR (CDCl₃+DMSO-d₆) δ (ppm); 1.88 (3H, s), 2.13 (6H, s), 3.01 (1H, br-s), 3.22 (2H, d, J=5.9 Hz), 3.6-4.2 (3H, m), 3.97 (2H, s), 6.93 (1H, s), 7.0-7.3 (4H, m), 7.4-7.8 (2H, m), 8.15 (1H, d, J=7.9 Hz), 8.30 (1H, s), 8.90 (1H, br-s).

### (3) 2-(3-aminobenzyl)-N-(4-hydroxy-2,3,5-trimethylphenyl)-1,2,3,4-tetrahydroisoquinoline-(3S)-carboxamide dihydrochloride

10% Palladium carbon (120 mg) was suspended in methanol (18 mL), the compound (600 mg) of (2) was added to thereto, and the mixture was subjected to catalytic hydrogenation at 40°C for 3.5 hr under 4 kgf/cm². After palladium carbon was removed by filtration, methanol was evaporated under reduced pressure. Diisopropyl ether was added to the residue, and the precipitate was collected by filtration. The obtained powder (410 mg) was purified by column chromatography.

The obtained residue was dissolved in methanol (3 mL), 10M hydrogen chloride-isopropanol solution (0.30 mL) was added, and the mixture was stirred for 10 min. After methanol was evaporated under reduced pressure, the residue was washed with diethyl ether, and the precipitate was collected by filtration to give the title compound as a powder (385 mg).
IR ν (Nujol) cm⁻¹; 1674, 1539, 1244, 1096, 754.
¹H-NMR (DMSO-d₆) δ (ppm); 1.94 (3H, s), 2.01, 2.12 (6H, s, s), 3.2-3.8 (2H, m), 4.3-4.8 (5H, m), 6.72 (1H, s), 7.0-7.8 (8H, m), 10.38 (1H, br-s).

### Example 56: 2-[(4-amino-2,6-diisopropylphenyl)amino]carbonyl-N-pentyl-1,2,3,4-tetrahydroisoquinoline-(3S)-carboxamide

### (1) 2-[(2,6-diisopropyl-4-nitrophenyl)amino]carbonyl-N-pentyl-1,2,3,4-tetrahydroisoquinoline-(3S)-carboxamide

The compound (6.27 g) of Example 27 (1) was dissolved in methylene chloride (50 mL), 1-ethyl-3-(3-dimethylaminopropyl)carbodiimide hydrochloride (6.48 g) and n-pentylamine (3.1 mL) were successively added under ice-cooling, and the mixture was stirred at the same temperature for 1.5 hr. After the reaction mixture was washed successively with 10% aqueous citric acid solution and saturated brine, and dried over sodium sulfate. Then, methylene chloride was evaporated under reduced pressure.

The obtained residue was dissolved in formic acid (20 mL), 10M hydrogen chloride - isopropanol solution (6.8 mL) was added under ice-cooling, and the mixture was stirred at the same temperature for 15 min. The reaction mixture was neutralized with saturated aqueous sodium hydrogen carbonate, extracted with ethyl acetate (50 mL), washed with saturated brine, and dried over sodium sulfate. Then, ethyl acetate was evaporated under reduced pressure. The obtained residue was washed with n-hexane, and the precipitate was collected by filtration to give a powder (2.58 g).

The obtained powder (300 mg) was dissolved in toluene (3 mL), trichloromethyl chloroformate (0.082 mL) was added, and the mixture was refluxed for 2.5 hr. After allowing to cool, 2,6-diisopropyl-4-nitroaniline (340 mg) and pyridine (0.16 mL) were successively added, and the mixture was stirred at room temperature for 30 min. 10% Aqueous citric acid solution was added to the reaction mixture, and the mixture was extracted with ethyl acetate (30 mL). The ethyl acetate layer was washed with saturated brine and dried over sodium sulfate. Then, ethyl acetate was evaporated under reduced pressure. The obtained residue was washed with diisopropyl ether, and the precipitate was collected by filtration to give the title compound as a powder (420 mg).
IR ν (Nujol) cm⁻¹; 1647, 1628, 1514, 1352, 1252, 745.
¹H-NMR (CDCl₃) δ (ppm); 0.81 (3H, br-t), 0.9-1.4 (6H, m), 1.23 (12H, d, J=6.8 Hz), 2.8-3.4 (6H, m), 4.53, 4.80 (2H, AB-q, J=14.9 Hz), 4.86 (1H, br-t), 5.92 (1H, br-t), 6.75 (1H, br-s), 7.0-7.4 (4H, m), 8.03 (2H, s).

### (2) 2-[(4-amino-2,6-diisopropylphenyl)amino]carbonyl-N-pentyl-1,2,3,4-tetrahydroisoquinoline-(3S)-carboxamide

10% Palladium carbon (100 mg) was suspended in methanol (10 mL), the compound (475 mg) of (1) was added, and the mixture was subjected to catalytic hydrogenation at 40°C for 1.5 hr under 4 kgf/cm². After palladium carbon was removed by filtration, methanol was evaporated under reduced pressure. The residue was dissolved in ethyl acetate (5 mL), and 10M hydrogen chloride - isopropanol solution (0.11 mL) was added. Ethyl acetate was evaporated under reduced pressure, diethyl ether was added, and the precipitate was collected by filtration. Ethyl acetate (20 mL) was added to the obtained powder, and the mixture was washed successively with saturated aqueous sodium hydrogen carbonate and saturated brine, and dried over sodium sulfate. Then, ethyl acetate was evaporated under reduced pressure. The obtained residue was washed with diisopropyl ether, and then the precipitate was collected by filtration to give the title compound as a powder (310 mg).
IR ν (Nujol) cm⁻¹; 1641, 1626, 1516, 1342, 745.
¹H-NMR (DMSO-d₆) δ (ppm); 0.6-1.4 (21H, m), 2.6-3.4 (6H, m), 4.53, 4.80 (2H, AB-q, J=14.9 Hz), 4.86 (1H, br-t), 5.92 (1H, br-t), 6.75 (1H, br-s), 7.0-7.4 (4H, m), 8.03 (2H, s).

### Example 57: 2-[(4-hydroxy-2,3,5-trimethylphenyl)amino]carbonyl-N-pentyl-1,2,3,4-tetrahydroisoquinoline-(3S)-carboxamide

The title compound was synthesized according to Example 56.
IR ν (Nujol) cm⁻¹; 1634, 1514, 1219, 1090, 743.
¹H-NMR (DMSO-d₆) δ (ppm); 0.78 (3H, br-t), 0.8-1.4 (6H, m), 1.99 (3H, s), 2.10 (6H, s), 2.8-3.3 (4H, m), 4.5-5.0 (3H, m), 6.73 (1H, s), 7.0-7.4 (4H, m), 7.56 (1H, br-t), 7.87 (2H, br-s).

### Example 58: 2-(2,2-dimethylpropionyl)-N-(4-methanesulfonylamino-2,6-dimethylphenyl)-1,2,3,4-tetrahydroisoquinoline-(3S)-carboxamide

### (1) N-(2,6-dimethyl-4-nitrophenyl)-2-(2,2-dimethylpropionyl)-1,2,3,4-tetrahydroisoquinoline-(3S)-carboxamide

The title compound was obtained according to Example 52 (2).
IR ν (Nujol) cm⁻¹; 1662, 1652, 1622, 1348, 1171, 745.
¹H-NMR (CDCl₃) δ (ppm); 1.40 (9H, s), 2.15 (6H, s), 3.10 (1H, dd, J=15.8, 6.5 Hz), 3.54 (1H, dd, J=15.8, 6.2 Hz), 4.58, 4.98 (2H, AB-q, J=15.3 Hz), 5.17 (1H, dd, J=6.5, 6.2 Hz), 7.1-7.5 (4H, m), 7.80-8.00 (1H, br), 7.88 (2H, s).

### (2) N-(4-amino-2,6-dimethylphenyl)-2-(2,2-dimethylpropionyl)-1,2,3,4-tetrahydroisoquinoline-(3S)-carboxamide

According to Example 56 (2), the title compound was obtained as a powder (274 mg) from the compound (395 mg) of (1).
¹H-NMR (CDCl₃) δ (ppm); 1.33 (9H, s), 1.91 (6H, br-s), 3.10 (1H, dd, J=15.6, 6.5 Hz), 3.45-3.55 (2H, br), 3.54 (1H, dd, J=15.6, 5.4 Hz), 4.62, 4.96 (2H, AB-q, J=15.9 Hz), 5.22 (1H, dd, J=6.5, 5.4 Hz), 6.33 (2H, s), 7.10-7.30 (5H, m).

### (3) 2-(2,2-dimethylpropionyl)-N-(4-methanesulfonylamino-2,6-dimethylphenyl)-1,2,3,4-tetrahydroisoquinoline-(3S)-carboxamide

The compound (264 mg) of (2) was dissolved in chloroform (5 mL), pyridine (0.17 mL) and methanesulfonyl chloride (0.11 mL) were successively added, and the mixture was stirred at room temperature for 3 hr. Chloroform (20 mL) was added, and the mixture was washed successively with 2 M hydrochloric acid (10 mL) and saturated brine, and dried over sodium sulfate. Then, chloroform was evaporated under reduced pressure. The residue was dissolved by heating in a mixture of chloroform - methanol (5:1, 10 mL). After allowing to cool, diethyl ether (30 mL) was added with stirring. The precipitate was collected by filtration to give the title compound as a powder (142 mg). IR ν (Nujol) cm⁻¹; 1645, 1615, 1410, 1316, 1147, 978, 850, 757, 519.
¹H-NMR (DMSO-d₆) δ (ppm); 1.29 (9H, s), 1.93 (6H, br-s), 2.92 (3H, s), 3.15-3.40 (2H, m), 4.59, 4.93 (2H, AB-q, J=16.5 Hz), 4.85-5.05 (1H, m), 6.84 (2H, s), 7.10-7.40 (4H, m), 9.11, 9.52 (2H, br-s, br-s).

The compounds of Example 59-Example 61 were synthesized according to Example 58.

### Example 59: 2-(2,2-dimethylpropionyl)-N-(5-methanesulfonylamino-2-methoxy-4-methylphenyl)-1,2,3,4-tetrahydroisoquinoline-(3S)-carboxamide

IR ν (Nujol) cm⁻¹; 1682, 1582, 1323, 1150, 1022, 976, 773, 505. ¹H-NMR (CDCl₃) δ (ppm) ; 1.39 (9H, s), 2.35 (3H, s), 3.03 (3H, s), 3.12 (1H, dd, J=16.0, 6.8 Hz), 3.44 (1H, dd, J=16.0, 4.9 Hz), 3.83 (3H, s), 4.55, 5.02 (2H, AB-q, J=15.9 Hz), 5.30 (1H, dd, J=6.8, 4.9 Hz), 5.90 (1H, s), 6.70 (1H, s), 7.11 (1H, d, J=6.8 Hz), 7.15-7.25 (3H, m), 8.25 (1H, s), 8.37 (1H, br-s).

### Example 60: 2-(2,2-dimethylpropionyl)-N-(2-isopropyl-5-methanesulfonylaminophenyl)-1,2,3,4-tetrahydroisoquinoline-(3S)-carboxamide

IR ν (Nujol) cm⁻¹; 3193, 1674, 1612, 1529.
¹H-NMR (CDCl₃) δ (ppm); 1.05-1.20 (6H, m), 1.37 (9H, s), 2.60-2.80 (1H, m), 2.86 (3H, s), 3.09 (1H, dd, J=16.1, 7.1 Hz), 3.52 (1H, dd, J=16.1, 5.4 Hz), 4.55, 4.95 (2H, AB-q, J=15.6 Hz), 5.26 (1H, dd, J=7.1, 5.4 Hz), 6.80-7.30 (7H, m), 7.60-7.70 (1H, m), 8.38 (1H, s).

### Example 61: 2-(2,2-dimethylbutyryl)-N-(2-isopropyl-5-methanesulfonylaminophenyl)-1,2,3,4-tetrahydroisoquinoline-(3S)-carboxamide

IR ν (Nujol) cm⁻¹; 3197, 1674, 1602, 1529.
¹H-NMR (CDCl₃) δ (ppm); 0.81 (3H, t, J=7.3 Hz), 1.13 (6H, d, J=6.1 Hz), 1.31, 1.34 (6H, s, s), 1.76 (2H, q, J=7.3 Hz), 2.60-2.80 (1H, m), 2.86 (3H, s), 3.08 (1H, dd, J=16.1, 7.1 Hz), 3.50 (1H, dd, J=16.1, 5.4 Hz), 4.54, 4.95 (2H, AB-q, J=15.6 Hz), 5.26 (1H, dd, J=7.1, 5.4 Hz), 6.80-7.30 (7H, m), 7.60-7.65 (1H, m), 8.42 (1H, s).

The compounds of Example 62-Example 67 were synthesized according to Example 2.

### Example 62: 2-(2,2-dimethylpropionyl)-N-(2-methoxy-6-methylphenyl)-1,2,3,4-tetrahydroisoquinoline-(3S)-carboxamide IR ν (Nujol) cm⁻¹; 1668, 1634, 1531, 1335, 1273, 1084, 739.

¹H-NMR (CDCl₃) δ (ppm); 1.41 (9H, s), 3.10 (1H, dd, J=15.9, 6.4 Hz), 3.55 (1H, dd, J=15.9, 4.2 Hz), 3.71 (3H, s), 4.66, 5.01 (2H, AB-q, J=16.3 Hz), 5.38 (1H, dd, J=6.4, 4.2 Hz), 6.67 (1H, d, J=8.3 Hz), 6.74 (1H, d, J=7.6 Hz), 7.06 (1H, dd, J=8.3, 7.6 Hz), 7.13 (1H, d, J=7.0 Hz), 7.15-7.25 (3H, m), 7.47 (1H, br-s).

### Example 63: N-(2,4-dimethylphenyl)-2-(2,2-dimethylpropionyl)-1,2,3,4-tetrahydroisoquinoline-(3S)-carboxamide

IR ν (Nujol) cm⁻¹; 1699, 1684, 1652, 1155, 1034, 928, 745.
¹H-NMR (CDCl₃) δ (ppm) ; 1.37 (9H, s), 3.08 (1H, dd, J=16.0, 7.0 Hz), 3.55 (1H, dd, J=16.0, 5.0 Hz), 4.52, 4.97 (2H, AB-q, J=15.7 Hz), 5.29 (1H, dd, J=7.0, 5.0 Hz), 6.90-7.00 (2H, m), 7.13 (1H, d, J=7.3 Hz), 7.15-7.25 (3H, m), 7.63 (1H, d, J=8.1 Hz), 8.01 (1H, br-s).

### Example 64: N-(2,6-dimethylphenyl)-2-(2,2-dimethylpropionyl)-1,2,3,4-tetrahydroisoquinoline-(3S)-carboxamide

IR ν (Nujol) cm⁻¹; 1666, 1614, 1410, 1196, 986, 783, 748.
¹H-NMR (CDCl₃) δ (ppm); 1.40 (9H, s), 3.12 (1H, dd, J=15.8, 6.7 Hz), 3.55 (1H, dd, J=15.8, 5.5 Hz), 4.62, 4.97 (2H, AB-q, J=15.6 Hz), 5.24 (1H, dd, J=6.7, 5.5 Hz), 6.95-7.10 (3H, m), 7.16 (1H, d, J=7.1 Hz), 7.20-7.30 (3H, m), 7.53 (1H, br-s).

### Example 65: 2-(2,2-dimethylpropionyl)-N-(2-methoxy-4-methylphenyl)-1,2,3,4-tetrahydroisoquinoline-(3S)-carboxamide

IR ν (Nujol) cm⁻¹; 1699, 1684, 1652, 1155, 1034, 928, 745.
¹H-NMR (CDCl₃) δ (ppm); 1.39 (9H, s), 3.11 (1H, dd, J=16.0, 6.6 Hz), 3.48 (1H, dd, J=16.0, 4.3 Hz), 3.81 (3H, s), 4.55, 5.03 (2H, AB-q, J=16.2 Hz), 5.37 (1H, dd, J=6.6, 4.3 Hz), 6.64 (1H, s), 6.70 (1H, d, J=8.0 Hz), 7.08 (1H, d, J=6.8 Hz), 7.10-7.25 (3H, m), 8.11 (1H, d, J=8.0 Hz), 8.31 (1H, br-s).

### Example 66: 2-(2,2-dimethylpropionyl)-N-(3-methanesulfonylamino-2,4-dimethylphenyl)-1,2,3,4-tetrahydroisoquinoline-(3S)-carboxamide

IR ν (Nujol) cm⁻¹; 1682, 1591, 1539, 1319, 1294, 1148, 978.
¹H-NMR (CDCl₃) δ (ppm); 1.39 (9H, s), 2.09 (3H, s), 2.31 (3H, s), 3.00 (3H, s), 3.10 (1H, dd, J=15.8, 6.6 Hz), 3.51 (1H, dd, J=15.8, 5.6 Hz), 4.56, 4.95 (2H, AB-q, J=15.6 Hz), 5.23 (1H, dd, J=6.6, 5.6 Hz), 6.15 (1H, s), 7.02 (1H, d, J=8.3 Hz), 7.15 (1H, d, J=7.1 Hz), 7.15-7.30 (3H, m), 7.52 (1H, d, J=8.3 Hz), 8.01 (1H, br-s).

### Example 67: N-(1,2-dimethyl-6-oxo-1H-pyridin-3-yl)-2-(2,2-dimethylpropionyl)-1,2,3,4-tetrahydroisoquinoline-(3S)-carboxamide

The compound of Reference Example 16 was used. IR ν (Nujol) cm⁻¹; 1418, 1286, 1171, 1113, 826, 754.
¹H-NMR (CDCl₃) δ (ppm); 1.38 (9H, s), 2.04 (3H, s), 3.10 (1H, dd, J=15.6, 6.6 Hz), 3.46 (1H, dd, J=15.6, 6.1 Hz), 3.48 (3H, s), 4.59, 4.91 (2H, AB-q, J=15.4 Hz), 5.07 (1H, dd, J=6.6, 6.1 Hz), 6.40 (1H, d, J=9.5 Hz), 7.00 (1H, d, J=9.5 Hz), 7.18 (1H, d, J=6.8 Hz), 7.20-7.30 (3H, m), 7.57(1H, br-s).

### Example 68: 2-(2,2-dimethylpropionyl)-N-(6-hydroxy-2-methylpyridin-3-yl)-1,2,3,4-tetrahydroisoquinoline-(3S)-carboxamide

### (1) N-(1-tert-butoxycarbonyl-2-methyl-6-oxo-1H-pyridin-3-yl)-2-(2,2-dimethylpropionyl)-1,2,3,4-tetrahydroisoquinoline-(3S)-carboxamide

According to Example 2, the title compound was synthesized from the compound of Reference Example 11.
¹H-NMR (CDCl₃) δ (ppm); 1.38 (9H, s), 1.58 (9H, s), 2.35 (3H, s), 3.08 (1H, dd, J=16.2, 7.1 Hz), 3.46 (1H, dd, J=16.2, 5.7 Hz), 4.47, 4.97 (2H, AB-q, J=15.5 Hz), 5.25 (1H, dd, J=7.1, 5.7 Hz), 6.93 (1H, d, J=8.8 Hz), 7.15 (1H, d, J=7.3 Hz), 7.20-7.30 (3H, m), 8.37 (1H, d, J=9.5 Hz), 8.48 (1H, br-s).

### (2) 2-(2,2-dimethylpropionyl)-N-(6-hydroxy-2-methylpyridin-3-yl)-1,2,3,4-tetrahydroisoquinoline-(3S)-carboxamide

The compound (602 mg) of (1) was dissolved in formic acid (1.2 mL), 8.7 M hydrogen chloride - isopropanol solution (0.30 mL) was added under ice-cooling, and the mixture was stirred at room temperature for 30 min. Diethyl ether (25 mL) was added to the reaction mixture, and the precipitate was collected by filtration. The obtained powder (520 mg) was suspended in methanol (5 mL), and the suspension was neutralized with saturated aqueous sodium hydrogen carbonate. Then, methanol was evaporated under reduced pressure, and the residue was extracted twice with chloroform (20 mL). The chloroform layer was washed successively with saturated aqueous sodium hydrogen carbonate (5 mL) and saturated brine, and dried over sodium sulfate. Then, chloroform was evaporated under reduced pressure. The residue was washed with diethyl ether (15 mL), and then the precipitate was collected by filtration to give the title compound as a powder (200 mg).
IR ν (Nujol) cm⁻¹; 1668, 1616, 1410, 1190, 1111, 750.
¹H-NMR (CDCl₃) δ (ppm); 1.37 (9H, s), 1.40-2.20 (1H, br), 2.05 (3H, s), 3.09 (1H, dd, J=14.9, 6.3 Hz), 3.46 (1H, dd, J=14.9, 6.2 Hz), 4.56, 4.92 (2H, AB-q, J=15.4 Hz), 5.10 (1H, dd, J=6.3, 6.2 Hz), 6.33 (1H, d, J=9.5 Hz), 7.16 (1H, d, J=6.8 Hz), 7.15-7.30 (3H, m), 7.33 (1H, d, J=9.5 Hz), 7.92 (1H, br-s).

### Example 69: 7-dimethylamino-2-(2,2-dimethylbutyryl)-N-(4-hydroxy-2,3,5-trimethylphenyl)-1,2,3,4-tetrahydroisoquinoline-(3S)-carboxamide hydrochloride

### (1) methyl 2-tert-butoxycarbonyl-7-nitro-1,2,3,4-tetrahydroisoquinoline-(3S)-carboxylate

Methyl 7-nitro-1,2,3,4-tetrahydroisoquinoline-(3S)-carboxylate (1.64 g) was dissolved in chloroform (30 mL), di-tert-butyl bicarbonate (1.89 g) was added, and the mixture was stirred at room temperature for 14 hr. Chloroform was evaporated under reduced pressure, and the residue was purified by column chromatography to give the title compound as a powder (2.43 g).
IR ν (Nujol) cm⁻¹; 1744, 1688, 1520, 1414, 1348, 1211, 1163, 1011, 772.
¹H-NMR (CDCl₃) δ (ppm); 1.51 (9H, s), 3.2-3.4 (2H, m), 3.64 (3H, s), 4.55, 4.85 (2H, AB-q, J=17.1 Hz), 5.0-5.4 (1H, m), 7.2-7.4 (1H, m), 7.9-8.2 (2H, m).

### (2) methyl 2-tert-butoxycarbonyl-7-dimethylamino-1,2,3,4-tetrahydroisoquinoline-(3S)-carboxylate

10% Palladium carbon (0.25 g) was suspended in methanol (50 mL), the compound (3.57 g) of (1) was added thereto, and the mixture was subjected to catalytic hydrogenation at room temperature for 1.5 hr under 3 kgf/cm². Then, formalin (2.4 mL) and 2 M hydrochloric acid (1.05 mL) were added, and the mixture was again subjected to catalytic hydrogenation at room temperature for 3 hr under 3 kgf/cm². After palladium carbon was removed by filtration, methanol was evaporated under reduced pressure. Saturated aqueous sodium hydrogen carbonate (30 mL) was added, and the mixture was extracted twice with ethyl acetate (30 mL). The ethyl acetate layers were combined, washed with saturated brine, and dried over sodium sulfate. Then, ethyl acetate was evaporated under reduced pressure. The residue was dissolved by heating in ethyl acetate (5 mL), n-hexane (30 mL) was added with stirring, and the mixture was stirred for 30 min. The precipitate was collected by filtration to give the title compound as a powder (2.33 g). IR ν (Nujol) cm⁻¹; 1747, 1688, 1620, 1520, 1402, 1246, 1202, 1124, 1016, 897, 808.
¹H-NMR (CDCl₃) δ (ppm); 1.45, 1.52 (9H, s, s), 2.8-3.3 (2H, m), 2.91 (6H, s), 3.63 (3H, s), 4.2-5.2 (3H, m), 6.4-6.7 (2H, m), 7.00 (1H, d, J=8.4 Hz).

### (3) N-(4-acetoxy-2,3,5-trimethylphenyl)-7-dimethylamino-1,2,3,4-tetrahydroisoquinoline-(3S)-carboxamide

The compound (1.79 g) of (2) was dissolved in a mixed solution of tetrahydrofuran - methanol (3:1, 30 mL), 1 M aqueous lithium hydroxide solution (10.7 mL) was added under ice-cooling, and the mixture was stirred at room temperature for 2 hr. After acidifying with 5% aqueous citric acid solution, tetrahydrofuran-methanol was evaporated under reduced pressure, and the residue was extracted twice with ethyl acetate (30 mL). The ethyl acetate layers were combined, washed with saturated brine, and dried over sodium sulfate. Then, ethyl acetate was evaporated under reduced pressure to give 1.76 g of a powder.

According to Example 27 (2)-(3), the title compound was obtained as a powder (1.66 g) from the powder. IR ν (Nujol) cm-¹; 1744, 1666, 1537, 1236, 1202, 1086, 814.
¹H-NMR (CDCl₃) δ (ppm); 1.71 (3H, br-s), 1.9-2.1 (1H, br), 2.12 (6H, s), 2.32 (3H, s), 2.6-3.5 (2H, m), 2.91 (6H, s), 3.5-4.0 (1H, m), 4.01 (2H, s), 6.47 (1H, d, J=1.6 Hz), 6.62 (1H, dd, J=8.5, 1.6 Hz), 7.08 (1H, d, J=8.5 Hz), 7.67 (1H, s), 9.23 (1H, br-s).

### (4) N-(4-acetoxy-2,3,5-trimethylphenyl)-7-dimethylamino-2-(2,2-dimethylbutyryl)-1,2,3,4-tetrahydroisoquinoline-(3S)-carboxamide

The compound (350 mg) of (3) was dissolved in methylene chloride (4 mL), triethylamine (0.25 mL) and 2,2-dimethylbutyryl chloride (153 mg) were successively added under ice-cooling, and the mixture was stirred at the same temperature for 40 min. Methylene chloride (20 mL) was added to the reaction mixture, and the mixture was washed successively with 5% aqueous citric acid solution (20 mL) and saturated brine, and dried over sodium sulfate. Then, methylene chloride was evaporated under reduced pressure. The residue was purified by column chromatography to give the title compound as a powder (326 mg).
IR ν (Nujol) cm⁻¹; 1751, 1666, 1616, 1516, 1408, 1192, 1082, 984, 905, 802.
¹H-NMR (CDCl₃) δ (ppm); 0.86 (3H, br-t), 1.33, 1.34 (6H, s, s), 1.77 (2H, br-q), 1.97 (3H, s), 2.03, 2.06 (6H, s, s), 2.30 (3H, s), 2.92 (6H, s), 2.98 (1H, dd, J=15.3, 6.8 Hz), 3.40 (1H, dd, J=15.3, 4.9 Hz), 4.51, 4.91 (2H, AB-q, J=15.6 Hz), 5.23 (1H, dd, J=6.8, 4.9 Hz), 6.51 (1H, d, J=2.5 Hz), 6.65 (1H, dd, J=8.4, 2.5 Hz), 7.10 (1H, d, J=8.4 Hz), 7.38 (1H, s), 7.83 (1H, br-s).

### (5) 7-dimethylamino-2-(2,2-dimethylbutyryl)-N-(4-hydroxy-2,3,5-trimethylphenyl)-1,2,3,4-tetrahydroisoquinoline-(3S)-carboxamide hydrochloride

The compound (316 mg) of (4) was dissolved in a mixed solution of tetrahydrofuran - methanol (3:1, 5.7 mL), 1 M aqueous lithium hydroxide solution (1.9 mL) was added under ice-cooling, and the mixture was stirred at room temperature for 1 hr. After neutralization with 10% aqueous citric acid solution, tetrahydrofuran - methanol was evaporated under reduced pressure, and pH of the residue was adjusted to 8 with saturated aqueous sodium hydrogen carbonate. The mixture was extracted twice with ethyl acetate (20 mL). The ethyl acetate layers were combined, washed with saturated brine, and dried over sodium sulfate. Then, ethyl acetate was evaporated under reduced pressure.

The obtained powder (316 mg) was dissolved in methanol (1 mL), 10 M hydrogen chloride - isopropanol solution (0.13 mL) was added under ice-cooling, and the mixture was stirred at the same temperature for 5 min. Diethyl ether (15 mL) was added, and the mixture was stirred at the same temperature for 30 min. Then, the precipitate was collected by filtration to give the title compound as a powder (284 mg).
IR ν (Nujol) cm⁻¹; 1614, 1178, 1097, 986, 930, 866.
¹H-NMR (DMSO-d₆) δ (ppm); 0.80 (3H, br-t), 1.25 (6H, s), 1.6-2.0 (2H, m), 1.81 (3H, s), 2.06 (6H, s), 2.9-3.6 (2H, m), 3.05 (6H, s), 4.4-6.4 (1H, br), 4.62, 4.99 (2H, AB-q, J=16.3 Hz), 5.11 (1H, br-t), 6.61 (1H, s), 7.2-7.7 (3H, m), 9.19 (1H, br-s).

The compounds of Example 70-Example 79 were synthesized according to Example 69.

### Example 70: 7-dimethylamino-2-[(2E,4E)-hexadienoyl]-N-(4-hydroxy-2,3,5-trimethylphenyl)-1,2,3,4-tetrahydroisoquinoline-(3S)-carboxamide hydrochloride

IR ν (Nujol) cm⁻¹; 1651, 1593, 1207, 997.
¹H-NMR (DMSO-d₆) δ (ppm); 1.67 (3H, s), 1.83 (3H, d, J=4.7 Hz), 2.03, 2.05 (6H, s, s), 2.9-3.6 (2H, m), 3.05 (6H, s), 4.4-7.0 (1H, br), 4.5-5.4 (3H, m), 6.0-6.8 (3H, m), 6.52 (1H, s), 7.0-7.7 (4H, m), 9.0-9.4 (1H, br).

### Example 71: 2-butyryl-7-dimethylamino-N-(4-hydroxy-2,3,5-trimethylphenyl)-1,2,3,4-tetrahydroisoquinoline-(3S)-carboxamide hydrochloride

IR ν (Nujol) cm⁻¹; 1622, 1207, 1096, 995.
¹H-NMR (DMSO-d₆) δ (ppm); 0.86 (3H, br-t), 1.6-2.0 (5H, m), 2.03, 2.05 (6H, s, s), 2.0-2.6 (2H, m), 2.9-3.6 (2H, m), 3.05 (6H, s), 4.4-6.4 (1H, br), 4.5-5.4 (3H, m), 6.50 (1H, s), 7.2-7.7 (3H, m), 8.96, 9.30 (1H, br-s, br-s).

### Example 72: 7-dimethylamino-N-(4-hydroxy-2,3,5-trimethylphenyl)-2-isobutyryl-1,2,3,4-tetrahydroisoquinoline-(3S)-carboxamide hydrochloride

IR ν (Nujol) cm⁻¹; 1622, 1207, 1090, 993.
¹H-NMR (DMSO-d₆) δ (ppm); 0.9-1.4 (6H, m), 1.71 (3H, s), 2.04 (6H, s), 2.8-3.5 (3H, m), 3.05 (6H, s), 4.0-6.0 (1H, br), 4.6-5.3 (3H, m), 6.53 (1H, s), 7.2-7.7 (3H, m), 9.01, 9.32 (1H, br-s, br-s).

### Example 73: 7-dimethylamino-2-(2,2-dimethylpropionyl)-N-(4-hydroxy-2,3,5-trimethylphenyl)-1,2,3,4-tetrahydroisoquinoline-(3S)-carboxamide hydrochloride

IR ν (Nujol) cm⁻¹; 1614, 1412, 1180, 1097, 986.
¹H-NMR (DMSO-d₆) δ (ppm); 1.29 (9H, m), 1.79 (3H, s), 2.05 (6H, s), 2.9-3.4 (2H, m), 3.05 (6H, s), 4.0-6.0 (1H, br), 4.5-5.3 (3H, m), 6.60 (1H, s), 7.2-7.7 (3H, m), 9.18 (1H, br-s).

### Example 74: 7-dimethylamino-2-hexanoyl-N-(4-hydroxy-2,3,5-trimethylphenyl)-1,2,3,4-tetrahydroisoquinoline-(3R)-carboxamide hydrochloride

IR ν (Nujol) cm⁻¹; 1622, 1207, 1097, 995.
¹H-NMR (DMSO-d₆) δ (ppm); 0.87 (3H, br-t), 1.2-2.0 (9H, m), 2.02 (6H, s), 2.1-2.6 (2H, m), 2.9-3.6 (2H, m), 3.04 (6H, s), 3.8-6.0 (1H, br), 4.5-5.4 (3H, m), 6.48 (1H, s), 7.2-7.7 (3H, m), 8.95, 9.31 (1H, br-s, br-s).

### Example 75: 7-dimethylamino-2-(2,2-dimethylbutyryl)-N-(4-hydroxy-2,3,5-trimethylphenyl)-1,2,3,4-tetrahydroisoquinoline-(3R)-carboxamide hydrochloride

IR ν (Nujol) cm⁻¹; 1614, 1242, 1178, 1097.
¹H-NMR (DMSO-d₆) δ (ppm); 0.80 (3H, br-t), 1.25 (6H, s), 1.6-2.0 (2H, m), 1.81 (3H, s), 2.06 (6H, s), 2.9-3.6 (2H, m), 3.05 (6H, s), 3.8-6.0 (1H, br), 4.61, 4.98 (2H, AB-q, J=16.0 Hz), 5.12 (1H, br-t), 6.61 (1H, s), 7.2-7.7 (3H, m), 9.19 (1H, br-s).

### Example 76: 7-dimethylamino-2-octanoyl-N-(2,4,6-trimethylphenyl)-1,2,3,4-tetrahydroisoquinoline-(3S)-carboxamide

IR ν (Nujol) cm⁻¹; 1661, 1614, 1288, 812.
¹H-NMR (CDCl₃) δ (ppm); 0.88 (3H, br-t), 1.0-2.0 (10H, m), 1.72 (3H, s), 1.93 (3H, s), 2.19 (3H, s), 2.51 (2H, t, J=7.1 Hz), 2.91 (6H, s), 2.8-3.6 (2H, m), 4.4-5.0 (2H, m), 5.25 (1H, t, J=5.0 Hz), 6.5-6.9 (2H, m), 6.77 (2H, s), 6.9-7.2 (1H, m), 7.34 (1H, br-s).

### Example 77: 7-dimethylamino-2-(2,2-dimethylpropionyl)-N-(3-methanesulfonylamino-2,4,6-trimethylphenyl)-1,2,3,4-tetrahydroisoquinoline-(3S)-carboxamide hydrochloride

IR ν (Nujol) cm⁻¹; 3429, 3163, 2446, 1674, 1622, 1313, 1150.
¹H-NMR (CDCl₃) δ (ppm); 1.40 (9H, s), 1.93 (3H, s), 2.00 (3H, s), 2.20 (3H, s), 2.94 (3H, s), 3.00-3.20 (2H, m), 3.12 (6H, s), 3.53 (1H, d, J=13.8 Hz), 4.71, 5.06 (2H, AB-q, J=16.4 Hz), 5.40-5.50 (1H, m), 6.54 (1H, s), 6.85 (1H, s), 7.40 (1H, br-d), 7.40-7.50 (1H, m), 7.60-7.80 (1H, br), 7.80-7.90 (1H, br).

### Example 78: 7-dimethylamino-2-(2,2-dimethylpropionyl)-N-(2,4,6-trimethylphenyl)-1,2,3,4-tetrahydroisoquinoline-(3S)-carboxamide

IR ν (Nujol) cm⁻¹; 1683, 1615, 1241, 1200, 1190, 981, 853, 830, 797.
¹H-NMR (CDCl₃) δ (ppm); 1.40 (9H, s), 1.98 (6H, s), 2.21 (3H, s), 2.9-3.2 (1H, m), 2.91 (6H, s), 3.42 (1H, dd, J=15.3, 5.1 Hz), 4.58, 4.91 (2H, AB-q, J=15.6 Hz), 5.17 (1H, dd, J=6.3, 5.1), 6.4-6.7 (2H, m), 6.80 (2H, s), 7.08 (1H, d, J=8.2 Hz), 7.29 (1H, br-s).

### Example 79: 4-{[7-dimethylamino-2-(2,2-dimethylpropionyl)-1,2,3,4-tetrahydroisoquinoline-(3S)-carbonyl]amino}-3-methylbenzoic acid

IR ν (Nujol) cm⁻¹; 1703, 1684, 1605, 1516, 1273, 1186, 1171, 1126, 770.
¹H-NMR (CDCl₃) δ (ppm); 1.39 (9H, s), 2.18 (3H, s), 2.93 (6H, s), 3.02 (1H, dd, J=15.2, 6.2 Hz), 3.41 (1H, dd, J=15.2, 5.5 Hz), 4.50, 4.93 (2H, AB-q, J=15.8 Hz), 5.25 (1H, dd, J=6.2, 5.5 Hz), 6.40-6.70 (2H, m), 7.12 (1H, d, J=8.8 Hz), 7.83 (1H, s), 7.88 (1H, d, J=8.8 Hz), 8.20 (1H, d, J=8.8 Hz), 8.42 (1H, s).

### Example 80: 7-amino-2-hexanoyl-N-(4-hydroxy-2,3,5-trimethylphenyl)-1,2,3,4-tetrahydroisoquinoline-(3S)-carboxamide hydrochloride

### (1) N-(4-acetoxy-2,3,5-trimethylphenyl)-2-tert-butoxycarbonyl-7-nitro-1,2,3,4-tetrahydroisoquinoline-(3S)-carboxamide

According to Example 2 (2)-(3), the title compound was obtained from the compound of Example 69 (1).
IR ν (Nujol) cm⁻¹; 1759, 1699, 1529, 1346, 1227, 1196, 1163, 1132, 1082.
¹H-NMR (CDCl₃) δ (ppm); 1.55 (9H, s), 1.95, 2.03 (9H, s, s), 2.30 (3H, s), 3.04 (1H, dd, J=15.8, 6.3 Hz), 3.4-4.4 (1H, m), 4.4-4.9 (2H, m), 5.0-5.2 (1H, m), 7.2-7.6 (2H, m), 7.9-8.4 (3H, m).

### (2) N-(4-acetoxy-2,3,5-trimethylphenyl)-7-nitro-1,2,3,4-tetrahydroisoquinoline-(3S)-carboxamide

The compound (2.54 g) of (1) was dissolved in trifluoroacetic acid (13 mL) under ice-cooling, and the solution was stirred at the same temperature for 1 hr. TFA was evaporated under reduced pressure, the crystalline residue was dissolved in diethyl ether (30 mL), and the solution was neutralized with saturated aqueous sodium hydrogen carbonate. The precipitate was collected by filtration to give the title compound as a powder (1.77 g).
IR ν (Nujol) cm⁻¹; 1763, 1682, 1587, 1221, 1088, 905, 829, 743. ¹H-NMR (CDCl₃) δ (ppm); 1.88 (1H, br-s), 2.07 (3H, s), 2.12 (6H, s), 2.33 (3H, s), 2.8-3.6 (2H, m), 3.79 (1H, dd, J=9.2, 5.7 Hz), 4.13 (2H, s), 7.34 (1H, d, J=8.4 Hz), 7.61 (1H, s), 7.9-8.1 (2H, m), 9.10 (1H, br-s).

### (3) N-(4-acetoxy-2,3,5-trimethylphenyl)-2-hexanoyl-7-nitro-1,2,3,4-tetrahydroisoquinoline-(3S)-carboxamide

According to Example 30 (3), the title compound was obtained as a powder (752 mg) using the compound (1.00 g) of (2) and hexanoyl chloride (0.39 mL).
IR ν (Nujol) cm⁻¹; 1747, 1651, 1339, 1200, 1084, 908, 810. ¹H-NMR (CDCl₃) δ (ppm); 0.92 (3H, br-t), 1.2-1.8 (6H, m), 2.05 (9H, s), 2.30 (3H, s), 2.52 (2H, br-t), 2.8-3.8 (2H, m), 4.4-5.6 (3H, m), 7.3-7.6 (2H, m), 8.0-8.2 (2H, m), 8.33 (1H, br-s).

### (4) 7-amino-2-hexanoyl-N-(4-hydroxy-2,3,5-trimethylphenyl)-1,2,3,4-tetrahydroisoquinoline-(3S)-carboxamide hydrochloride

The compound (750 mg) of (3) was dissolved in a mixed solution of tetrahydrofuran - methanol (3:1, 18 mL), 1 M aqueous lithium hydroxide solution (4.5 mL) was added under ice-cooling, and the mixture was stirred at the same temperature for 1.5 hr. After the reaction mixture was neutralized with 10% aqueous citric acid solution, tetrahydrofuran - methanol was evaporated under reduced pressure, and the residue was extracted twice with ethyl acetate (30 mL). The ethyl acetate layers were combined, washed successively with saturated aqueous sodium hydrogen carbonate and saturated brine, and dried over sodium sulfate. Then, ethyl acetate was evaporated under reduced pressure.

10% Palladium carbon (60 mg) was suspended in methanol (10 mL), the obtained powder (650 mg) was added thereto, and the mixture was subjected to catalytic hydrogenation at 30°C for 2.5 hr under 3 kgf/cm². Methylene chloride was added to dissolve the precipitate, and palladium carbon was removed by filtration. Then, solvent was evaporated under reduced pressure, and the residue was purified by column chromatography.

The obtained residue (489 mg) was dissolved in methanol (2 mL), 10 M hydrogen chloride - isopropanol solution (0.23 mL) was added under ice-cooling, and the mixture was stirred at the same temperature for 15 min. Diethyl ether (20 mL) was added, and the precipitate was collected by filtration to give the title compound as a powder (462 mg).
IR ν (Nujol) cm⁻¹; 3600-3100, 1653, 1622, 1545, 1092.
¹H-NMR (DMSO-d₆) δ (ppm); 0.88 (3H, br-t), 1.0-1.8 (6H, m), 1.70 (3H, s), 2.03, 2.05 (6H, s, s), 2.2-2.7 (2H, m), 3.0-3.6 (2H, m), 4.4-5.3 (3H, m), 6.50 (1H, s), 7.0-7.5 (3H, m), 8.99, 9.34 (1H, br-s, br-s).

The compounds of Example 81-Example 82 were synthesized according to Example 80.

### Example 81: 7-amino-N-(2,4-difluorophenyl)-2-(3,3-dimethylbutyryl)-1,2,3,4-tetrahydroisoquinoline-(3S)-carboxamide hydrochloride

IR ν (Nujol) cm⁻¹; 1652, 1635, 1259, 1142, 1097, 965.
¹H-NMR (DMSO-d₆) δ (ppm); 1.03 (9H, s), 2.35 (2H, s), 3.0-5.3 (5H, m), 6.8-7.8 (6H, m), 9.73 (1H, br-s).

### Example 82: 7-amino-2-(3,3-dimethylbutyryl)-N-(2,4,6-trimethoxyphenyl)-1,2,3,4-tetrahydroisoquinoline-(3S)-carboxamide

IR ν (Nujol) cm⁻¹; 1684, 1595, 1507, 1228, 1205, 1154, 1130, 811.
¹H-NMR (CDCl₃) δ (ppm); 1.10 (9H, s), 2.40 (2H, s), 2.6-3.6 (4H, m), 3.62, 3.69, 3.75 (9H, s, s, s), 4.5-4.9 (2.2H, m), 5.6-5.8 (0.8H, m), 6.04, 6.07 (2H, s, s), 6.4-7.4 (1H, br), 6.4-6.8 (2H, m), 6.99 (1H, d, J=8.4 Hz).

### Example 83: 7-dimethylamino-2-hexanoyl-N-(4-hydroxy-2,3,5-trimethylphenyl)-1,2,3,4-tetrahydroisoquinoline-(3S)-carboxamide hydrochloride

10% Palladium carbon (25 mg) was suspended in methanol (5 mL), the compound (250 mg) of Example 80 and formalin (0.20 mL) were successively added, and the mixture was subjected to catalytic hydrogenation at 40°C for 2 hr under 4 kgf/cm². After palladium carbon was removed by filtration, methanol was evaporated under reduced pressure. To the residue (0.8 g) was added diethyl ether (15 mL), and the mixture was stirred at room temperature for 1.5 hr. The precipitate was collected by filtration to give the title compound as a powder (220 mg).
IR ν (Nujol) cm⁻¹; 3600-3100, 1651, 1506, 1097, 995, 928, 868.
¹H-NMR (DMSO-d₆) δ (ppm); 0.88 (3H, br-t), 1.0-1.8 (6H, m), 1.66 (3H, s), 2.03 (6H, s), 2.2-2.7 (2H, m), 3.0-3.6 (2H, m), 3.05 (6H, s), 4.4-5.3 (3H, m), 6.49 (1H, s), 7.2-7.7 (3H, m), 8.96, 9.33 (1H, br-s, br-s).

### Example 84: 7-dimethylamino-N-(4-hydroxy-2,3,5-trimethylphenyl)-2-propyl-1,2,3,4-tetrahydroisoquinoline-(3S)-carboxamide

### (1)N-(4-acetoxy-2,3,5-trimethylphenyl)-7-nitro-2-propyl-1,2,3,4-tetrahydroisoquinoline-(3S)-carboxamide

The title compound was obtained from the compound of Example 80 (2) according to Example 29.
IR ν (Nujol) cm⁻¹; 1749, 1663, 1526, 1348, 1232, 1202, 1086, 820, 741.
¹H-NMR (CDCl₃) δ (ppm); 0.95 (3H, br-t), 1.4-2.0 (2H, m), 1.92 (3H, s), 2.03, 2.09 (6H, s, s), 2.31 (3H, s), 2.65(2H, br-t), 2.9-3.2 (2H, m), 3.63 (1H, dd, J=7.0, 5.0 Hz), 3.78, 4.10 (2H, AB-q, J=14.7 Hz), 7.36 (1H, d, J=8.1 Hz), 7.54 (1H, s), 8.0-8.3 (2H, m), 9.15 (1H, br-s).

### (2) N-(4-acetoxy-2,3,5-trimethylphenyl)-7-dimethylamino-2-propyl-1,2,3,4-tetrahydroisoquinoline-(3S)-carboxamide

10% Palladium carbon (30 mg) was suspended in methanol (7 mL), the compound (329 mg) of (1) was added, and the mixture was subjected to catalytic hydrogenation at room temperature for 2.5 hr under 3 kgf/cm² Formalin (0.34 mL) and 2 M hydrochloric acid (0.15 mL) were successively added, and the mixture was again subjected to catalytic hydrogenation at room temperature for 4 hr under 3 kgf/cm². After palladium carbon was removed by filtration, methanol was evaporated under reduced pressure. Saturated aqueous sodium hydrogen carbonate (10 mL) was added, and the mixture was extracted twice with ethyl acetate (10 mL). The ethyl acetate layers were combined, washed with saturated brine, and dried over sodium sulfate. Then, ethyl acetate was evaporated under reduced pressure. The residue was purified by column chromatography to give the title compound as a viscous oil (230 mg).
¹H-NMR (CDCl₃) δ (ppm) ; 0.92 (3H, br-t), 1.4-2.0 (2H, m), 1.88(3H, s), 2.02, 2.08 (6H, s, s), 2.30 (3H, s), 2.64 (2H, br-t), 2.8-3.2 (2H, m), 2.91 (6H, s), 3.49 (1H, br-t), 3.65, 3.92 (2H, AB-q, J=14.5 Hz), 6.5-6.7 (2H, m), 7.03 (1H, d, J=7.8 Hz), 7.55 (1H, s), 9.25 (1H, br-s).

### (3) 7-dimethylamino-N-(4-hydroxy-2,3,5-trimethylphenyl)-2-propyl-1,2,3,4-tetrahydroisoquinoline-(3S)-carboxamide

According to Example 48 (2), the title compound was obtained as a powder (122 mg) from the compound (220 mg) of (2).
IR ν (Nujol) cm⁻¹; 1651, 1514, 1240, 1099.
¹H-NMR (CDCl₃) δ (ppm); 0.93 (3H, br-t), 1.4-2.0 (2H, m), 1.83(3H, s), 2.06, 2.11 (6H, s, s), 2.65 (2H, br-t), 2.9-3.2 (2H, m), 2.91 (6H, s), 3.49 (1H, br-t), 3.65, 3.92 (2H, AB-q, J=13.9 Hz), 5.0-5.4 (1H, br), 6.5-6.8 (2H, m), 6.9-7.2 (2H, m), 9.00 (1H, br-s). Example 85: 7-amino-2-(2,2-dimethylpropionyl)-N-(4-hydroxy-2,3,5-trimethylphenyl)-1,2,3,4-tetrahydroisoquinoline-(3S)-carboxamide hydrochloride

### (1) N-(4-acetoxy-2,3,5-trimethylphenyl)-2-(2,2-dimethylpropionyl)-7-nitro-1,2,3,4-tetrahydroisoquinoline-(3S)-carboxamide

According to Example 30 (3), the title compound was obtained as a powder from the compound of Example 80 (2).
IR ν (Nujol) cm⁻¹; 1747, 1668, 1634, 1531, 1344, 1194, 1082, 903, 741, 660.
¹H-NMR (CDCl₃) δ (ppm); 1.39 (9H, s), 2.06 (9H, s), 2.31 (3H, s), 3.10 (1H, dd, J=16.8, 6.6 Hz), 3.63 (1H, dd, J=16.8, 4.3 Hz), 4.53, 5.16 (2H, AB-q, J=16.2 Hz), 5.48 (1H, dd, J=6.6, 4.3), 7.3-7.5 (1H, m), 7.46 (1H, s), 8.0-8.3 (3H, m).

### (2) 7-amino-2-(2,2-dimethylpropionyl)-N-(4-hydroxy-2,3,5-trimethylphenyl)-1,2,3,4-tetrahydroisoquinoline-(3S)-carboxamide hydrochloride

10% Palladium carbon (30 mg) was suspended in methanol (10 mL), the compound (300 mg) of (1) was added, and the mixture was subjected to catalytic hydrogenation at room temperature for 5 hr under 3 kgf/cm². Chloroform was added to dissolve the precipitate, palladium carbon was removed by filtration, and then solvent was evaporated under reduced pressure.

According to Example 69 (5), the title compound was obtained as a powder (147 mg) from the obtained powder.
IR ν (Nujol) cm⁻¹; 1651, 1180, 1096.
¹H-NMR (DMSO-d₆) δ (ppm); 1.28 (9H, s), 1.80 (3H, s), 2.05 (6H, s), 2.8-4.0 (3H, m), 4.57, 4.95 (2H, AB-q, J=17.3 Hz), 5.06 (1H, br-t), 6.59 (1H, s), 7.0-7.4 (3H, m), 9.18 (1H, br-s).

### Example 86: 2-(2,2-dimethylpropionyl)-N-(4-hydroxy-2,3,5-trimethylphenyl)-7-methanesulfonylamino-1,2,3,4-tetrahydroisoquinoline-(3S)-carboxamide

The free form (282 mg) of Example 85 was dissolved in chloroform (6 mL), pyridine (0.084 mL) and methanesulfonyl chloride (95 mg) were successively added at room temperature, and the mixture was stirred at the same temperature for 1 hr. Pyridine (0.084 mL) and methanesulfonyl chloride (64 mg) were further added, and the mixture was stirred for 14 hr. 2 M hydrochloric acid (20 mL) was added to the reaction mixture, and chloroform was evaporated under reduced pressure. Ethyl acetate (10 mL) was added to the residue was added, and the precipitate was collected by filtration. The ethyl acetate layer of the filtrate was separated, washed with saturated brine, and combined with a solution of the obtained powder dissolved in chloroform. The mixture was dried over sodium sulfate, and solvent was evaporated under reduced pressure. The residue was purified by column chromatography, and then the obtained powder was washed with a mixed solution of chloroform - methanol (20:1, 5 mL), and collected by filtration to give the title compound as a powder (184 mg).
IR ν (Nujol) cm⁻¹; 1653, 1605, 1506, 1319, 1225, 1177, 1144, 976, 839, 530, 515.
¹H-NMR (DMSO-d₆) δ (ppm); 1.28 (9H, s), 1.79 (3H, s), 2.05 (6H, s), 2.95 (3H, s), 3.0-3.6 (2H, m), 4.4-5.2 (3H, m), 6.59 (1H, s), 6.9-7.3 (3H, m), 7.8-8.3 (1H, br-s), 9.05 (1H, br-s), 9.59 (1H, br-s).

### Example 87: 7-(2-aminoacetylamino)-2-hexanoyl-N-(2,4,6-trimethylphenyl)-1,2,3,4-tetrahydroisoquinoline-(3S)-carboxamide

### (1) methyl 2-hexanoyl-7-nitro-1,2,3,4-tetrahydroisoquinoline-(3S)-carboxylate

Methyl 7-nitro-1,2,3,4-tetrahydroisoquinoline-(3S)-carboxylate (2.00 g) was dissolved in chloroform (20 mL), triethylamine (1.3 mL) and hexanoyl chloride (1.3 mL) were added under ice-cooling, and the mixture was stirred at the same temperature for 30 min. The reaction mixture was washed successively with 5% aqueous citric acid solution and saturated brine, and dried over sodium sulfate. Then, chloroform was evaporated under reduced pressure. The residue was purified by column chromatography to give the title compound as an oil (2.92 g).
¹H-NMR (CDCl₃) δ (ppm); 0.92 (3H, br-t), 1.0-2.0 (6H, m), 2.2-2.7 (2H, m), 3.0-3.7 (2H, m), 3.63 (3H, s), 4.3-5.3 (2H, m), 5.64 (1H, dd, J=5.7, 3.2 Hz), 7.34 (1H, d, J=9.5 Hz), 8.0-8.2 (2H, m).

### (2) methyl 7-amino-2-hexanoyl-1,2,3,4-tetrahydroisoquinoline-(3S)-carboxylate

10% Palladium carbon (290 mg) was suspended in methanol (30 mL), the compound (2.90 g) of (1) was added, and the mixture was subjected to hydrogenation at room temperature for 3.5 hr under 3 kgf/cm². After palladium carbon was removed by filtration, methanol was evaporated under reduced pressure, and the residue was purified by column chromatography to give the title compound as an oil (2.29 g).
¹H-NMR (CDCl₃) δ (ppm); 0.91 (3H, br-t), 1.0-2.0 (6H, m), 2.2-2.6 (2H, m), 2.9-3.4 (2H, m), 3.4-4.0 (2H, br), 3.61 (3H, s), 4.4-5.0 (2H, m), 5.43 (1H, br-t), 6.4-6.7 (2H, m), 6.93 (1H, d, J=7.9 Hz).

### (3) methyl 7-(tert-butoxycarbonylamino)acetylamino-2-hexanoyl-1,2,3,4-tetrahydroisoquinoline-(3S)-carboxylate

The compound (1.93 g) of (2) and N-Boc-glycine (1.22 g) were dissolved in methylene chloride (20 mL), 1-ethyl-3-(3-dimethylaminopropyl)carbodiimide hydrochloride (1.34 g) was added under ice-cooling, and the mixture was stirred at room temperature for 15 hr. After methylene chloride was evaporated under reduced pressure, 1 M hydrochloric acid (20 mL) was added to the residue, and the mixture was extracted with ethyl acetate (40 mL). The ethyl acetate layer was washed successively with 1 M hydrochloric acid (20 mL), saturated aqueous sodium hydrogen carbonate and saturated brine, and dried over sodium sulfate. Then, ethyl acetate was evaporated under reduced pressure. The residue was purified by column chromatography to give the title compound as a powder (2.91 g). IR ν (Nujol) cm⁻¹; 1657, 1533, 1256, 947, 908, 750, 694.
¹H-NMR (CDCl₃) δ (ppm); 0.91 (3H, br-t), 1.2-2.0 (6H, m), 1.47 (9H, s), 2.46 (2H, br-t), 3.0-3.3 (2H, m), 3.60 (3H, s), 3.81 (2H, d, J=5.9 Hz), 4.5-4.9 (2H, m), 5.3-5.6 (2H, m), 7.0-7.5 (2H, m), 7.54 (1H, s), 8.35 (1H, br-s).

### (4) 7-(tert-butoxycarbonylamino)acetylamino-2-hexanoyl-1,2,3,4-tetrahydroisoquinoline-(3S)-carboxylic acid

According to Example 2 (2), the title compound was obtained as a powder (2.72 g) from the compound (2.91 g) of (3).
IR ν (Nujol) cm⁻¹; 1728, 1605, 1317, 1209, 746, 694.
¹H-NMR (CDCl₃) δ (ppm); 0.94 (3H, br-t), 1.0-2.0 (6H, m), 1.44 (9H, s), 2.2-2.6 (2H, m), 2.8-3.5 (2H, m), 3.84 (2H, br-d), 4.4-5.0 (2H, m), 5.0-7.6 (1H, br), 5.2-5.5 (1H, m), 5.5-6.0 (1H, br), 6.8-7.4 (2H, m), 7.44 (1H, s), 8.71 (1H, br-s).

### (5) 7-(tert-butoxycarbonylamino)acetylamino-2-hexanoyl-N-(2,4,6-trimethylphenyl)-1,2,3,4-tetrahydroisoquinoline-(3S)-carboxamide

According to Example 2 (3), the title compound was obtained as a powder (637 mg) from the compound (1.50 g) of (4) and 2,4,6-trimethylaniline (499 mg).
IR ν (Nujol) cm⁻¹; 1705, 1682, 1663, 1632, 1514, 1418, 1244, 1180, 1057.
¹H-NMR (CDCl₃) δ (ppm); 0.91 (3H, br-t), 1.0-2.0 (6H, m), 1.46 (9H, s), 1.71, 1.77, 1.92 (6H, s, s, s), 2.18 (3H, s), 2.50 (2H, br-t), 2.8-3.7 (2H, m), 3.82 (2H, d, J=5.7 Hz), 4.5-5.0 (2H, m), 5.2-5.6 (2H, m), 6.75 (2H, s), 7.0-7.8 (4H, m), 8.47 (1H, br-s).

### (6) 7-(2-aminoacetylamino)-2-hexanoyl-N-(2,4,6-trimethylphenyl)-1,2,3,4-tetrahydroisoquinoline-(3S)-carboxamide

The compound (620 mg) of (5) was dissolved in formic acid (3 mL), 8.8 M hydrogen chloride - isopropanol solution (0.38 mL) was added under ice-cooling, and the mixture was stirred at the same temperature for 2 hr. The reaction mixture was alkalified with 5 M aqueous sodium hydroxide solution (16 mL) under ice-cooling, and extracted with a mixed solvent (40 mL) of ethyl acetate-methanol (20:1). The organic layer was washed with saturated brine and dried over sodium sulfate. Then, solvent was evaporated under reduced pressure. Ethyl acetate (15 mL) was added to the obtained residue, and the mixture was stirred for 30 min. Then, the precipitate was collected by filtration to give the title compound as a powder (130 mg).
IR ν (Nujol) cm⁻¹; 1662, 1652, 1622, 1609, 1505, 1238, 827.
¹H-NMR (DMSO-d₆) δ (ppm); 0.89 (3H, br-t), 1.0-2.0 (6H, m), 1.76 (6H, s), 2.16 (3H, s), 2.0-5.4 (11H, m), 6.76 (2H, s), 6.8-7.8 (4H, m), 8.5-9.4 (1H, br).

### Example 88: 2-hexanoyl-N-(4-hydroxy-2,3,5-trimethylphenyl)-7-(morpholin-4-yl)-1,2,3,4-tetrahydroisoquinoline-(3S)-carboxamide hydrochloride

### (1) methyl 2-hexanoyl-7-(morpholin-4-yl)-1,2,3,4-tetrahydroisoquinoline-(3S)-carboxylate

The compound (700 mg) of Example 87 (2) was dissolved in N-methyl-2-pyrrolidinone (3.5 mL), bis(2-chloroethyl)ether (329 mg), potassium carbonate (636 mg) and potassium iodide (191 mg) were successively added, and the mixture was stirred at 100°C for 5 hr. After allowing to cool, acetyl chloride (0.03 mL) was added to the reaction mixture, and the mixture was stirred for 10 min. Water (20 mL) was added, and the mixture was extracted twice with ethyl acetate (20 mL). The ethyl acetate layers were combined, washed with saturated brine, and dried over sodium sulfate. Then, ethyl acetate was evaporated under reduced pressure. The residue was purified by column chromatography to give the title compound as a viscous oil (626 mg).
¹H-NMR (CDCl₃) δ (ppm); 0.91 (3H, br-t), 1.1-1.8 (6H, m), 2.35 (2H, br-t), 2.8-3.5 (2H, m), 3.11 (4H, br-t), 3.61 (3H, s), 3.85 (4H, br-t), 4.4-5.6 (3H, m), 6.6-6.9 (2H, m), 7.07 (1H, d, J=8.3 Hz).

### (2) 2-hexanoyl-7-(morpholin-4-yl)-1,2,3,4-tetrahydroisoquinoline-(3S)-carboxylic acid

According to Example 2 (2), the title compound was obtained as a powder (691 mg) from the compound (685 mg) of (1).
¹H-NMR (CDCl₃+DMSO-d₆) δ (ppm); 0.90 (3H, br-t), 1.1-1.8 (6H, m), 2.38 (2H, br-t), 2.8-3.5 (2H, m), 3.11 (4H, br-t), 3.85 (4H, br-t), 4.4-5.6 (3H, m), 6.6-7.0 (2H, m), 6.6-8.0 (1H, br), 7.07 (1H, d, J=8.3 Hz).

### (3) 2-hexanoyl-N-(4-hydroxy-2,3,5-trimethylphenyl)-7-(morpholin-4-yl)-1,2,3,4-tetrahydroisoquinoline-(3S)-carboxamide hydrochloride

According to Example 48, the title compound was obtained as a powder (241 mg) from the compound (624 mg) of (2).
IR ν (Nujol) cm⁻¹; 1634, 1205, 1126, 1097, 1065, 876.
¹H-NMR (DMSO-d₆) δ (ppm); 0.88 (3H, br-t), 1.0-2.0 (6H, m), 1.65 (3H, s), 2.04 (6H, s), 2.2-2.6 (2H, m), 2.9-3.5 (6H, m), 3.7-4.2 (4H, m), 4.2-5.4 (4H, m), 6.51 (1H, s), 7.2-7.6 (3H, m), 8.94 (1H, br-s).

The compounds of Example 89-Example 93 were synthesized according to Example 88.

### Example 89: N-(4-hydroxy-2,3,5-trimethylphenyl)-2-pentanoyl-7-(pyrrolidin-1-yl)-1,2,3,4-tetrahydroisoquinoline-(3S)-carboxamide hydrochloride

IR ν (Nujol) cm⁻¹; 3246, 1734, 1637.
¹H-NMR (DMSO-d₆) δ (ppm); 0.70-1.20 (3H, m), 1.20-2.00 (8H, m), 2.04 (9H, s), 2.20-2.80 (2H, m), 2.90-3.50 (6H, m), 4.40-5.30 (4H, m), 6.51 (1H, s), 6.80-7.30 (3H, m), 8.91, 9.25 (1H, s, s).

### Example 90: 2-(3,3-dimethylbutyryl)-N-(4-hydroxy-2,3,5-trimethylphenyl)-7-(pyrrolidin-1-yl)-1,2,3,4-tetrahydroisoquinoline-(3S)-carboxamide hydrochloride

IR ν (Nujol) cm⁻¹; 3246, 1647.
¹H-NMR (DMSO-d₆) δ (ppm); 1.04 (9H, s), 1.60-1.90 (4H, m), 2.04 (9H, s), 2.50 (2H, s), 2.90-3.60 (6H, m), 4.40-5.30 (4H, m), 6.80-7.30 (3H, m), 8.95, 9.25 (1H, s, s).

### Example 91: N-(4-hydroxy-2,3,5-trimethylphenyl)-2-propionyl-7-(pyrrolidin-1-yl)-1,2,3,4-tetrahydroisoquinoline-(3S)-carboxamide hydrochloride

IR ν (Nujol) cm⁻¹; 3241, 2559, 2457, 1645.
¹H-NMR (DMSO-d₆) δ (ppm); 1.04 (3H, t, J=7.0 Hz), 1.50-1.90 (4H, m), 2.03 (9H, s), 2.10-2.80 (2H, m), 2.90-3.70 (6H, m), 3.60-4.40 (1H, br), 4.40-5.20 (3H, m), 6.51 (1H, s), 6.80-7.30 (3H, m), 9.58, 10.25 (1H, s, s).

### Example 92: 2-acetyl-N-(4-hydroxy-2,3,5-trimethylphenyl)-7-(pyrrolidin-1-yl)-1,2,3,4-tetrahydroisoquinoline-(3S)-carboxamide hydrochloride

IR ν (Nujol) cm⁻¹; 3242, 2563, 2457, 1633.
¹H-NMR (DMSO-d₆) δ (ppm); 1.50-1.80 (4H, m), 2.05 (9H, s), 2.19 (3H, s), 2.90-3.60 (6H, m), 3.97 (1H, br-s), 4.40-5.20 (3H, m), 6.48, 6.55 (1H, s, s), 6.80-7.30 (3H, m), 8.94, 9.25 (1H, s, s).

### Example 93: N-(4-hydroxy-2,3,5-trimethylphenyl)-2-isobutyryl-7-(pyrrolidin-1-yl)-1,2,3,4-tetrahydroisoquinoline-(3S)-carboxamide hydrochloride

IR ν (Nujol) cm⁻¹; 3242, 1639.
¹H-NMR (DMSO-d₆) δ (ppm); 1.00-1.10 (6H, m), 1.72, 1.76 (3H, s, s), 2.00-2.10 (10H, m), 2.75-3.40 (7H, m), 4.50-4.80 (3H, m), 4.90-5.00 (0.32H, m), 5.20-5.25 (0.68H, m), 6.51, 6.56 (1H, m), 6.80-7.20 (3H, m), 8.98, 9.27 (1H, s, s).

### Example 94: N-(4-acetoxy-2,3,5-trimethylphenyl)-7-amino-2-hexanoyl-1,2,3,4-tetrahydroisoquinoline-(3S)-carboxamide hydrochloride

### (1) methyl 2-benzyloxycarbonyl-7-nitro-1,2,3,4-tetrahydroisoquinoline-(3S)-carboxylate

Methyl 7-nitro-1,2,3,4-tetrahydroisoquinoline-(3S)-carboxylate (8.00 g) was suspended in acetone (80 mL), magnesium oxide (2.05 g) and benzyl chloroformate (5.3 mL) were successively added under ice-cooling, and the mixture was stirred at room temperature for 16 hr. After magnesium oxide was removed by filtration, diethyl ether (50 mL) was added to the filtrate, and the mixture was again subjected to filtration. Then, solvent was evaporated under reduced pressure. n-Hexane (20 mL) was added to the oily residue (10.3 g), and the mixture was stirred for 30 min. The supernatant solution was removed, and the residue was dried under reduced pressure to give the title compound as an oil (9.30 g).
¹H-NMR (CDCl₃) δ (ppm); 3.28 (2H, br-d), 3.57, 3.63 (3H, s, s), 4.5-5.4 (3H, m), 5.24 (2H, s), 7.2-7.6 (6H, m), 7.9-8.2 (2H, m).

### (2) 2-benzyloxycarbonyl-7-tert-butoxycarbonylamino-1,2,3,4-tetrahydroisoquinoline-(3S)-carboxylic acid

The compound (9.30 g) of (1) was suspended in 65% methanol (260 mL), concentrated hydrochloric acid (2.6 mL) was added, and the mixture was heated to reflux. Under refluxing, iron powder (5.61 g) was added in portions over 10 min, and then the mixture was stirred for 1.5 hr. Additional concentrated hydrochloric acid (2.6 mL) was added, and the mixture was stirred for 2 hr. Additional iron powder (2.80 g) was added, and the mixture was stirred for 1 hr. After allowing to cool, the reaction mixture was neutralized with sodium bicarbonate (15 g). Insoluble material was removed by filtration, and methanol was evaporated under reduced pressure. The residue was twice extracted with ethyl acetate (100 mL), and the ethyl acetate layers were combined, washed with saturated brine, and dried over sodium sulfate. Then, ethyl acetate was evaporated under reduced pressure.

The obtained residue was dissolved in tetrahydrofuran (40 mL), di-tert-butyl bicarbonate (5.48 g) was added, and the mixture was stirred at 45°C for 16 hr. Tetrahydrofuran was evaporated under reduced pressure, and the residue was purified by column chromatography.

The obtained residue was dissolved in a mixed solution of tetrahydrofuran-methanol (3:1, 100 mL), 1 M aqueous lithium hydroxide solution (33 mL) was added dropwise over 10 min at room temperature, and the mixture was stirred at the same temperature for 1 hr. After the reaction mixture was neutralized with 10% aqueous citric acid solution, tetrahydrofuran-methanol was evaporated under reduced pressure, and the residue was twice extracted with ethyl acetate (100 mL). The ethyl acetate layers were combined, washed with saturated brine, and dried over sodium sulfate. Then, ethyl acetate was evaporated under reduced pressure to give the title compound as a powder (6.91 g).
¹H-NMR (CDCl₃) δ (ppm); 1.50 (9H, s), 3.0-3.4 (2H, m), 4.3-5.3 (3H, m), 5.19 (2H, s), 5.4-6.6 (1H, br), 6.57 (1H, br-s), 6.8-7.6 (8H, m).

### (3) N-(4-acetoxy-2,3,5-trimethylphenyl)-2-benzyloxycarbonyl-7-tert-butoxycarbonylamino-1,2,3,4-tetrahydroisoquinoline-(3S)-carboxamide

The compound (6.91 g) of (2) and the compound (3.13 g) of Reference Example 15 were dissolved in methylene chloride (70 mL), 1-ethyl-3-(3-dimethylaminopropyl)carbodiimide hydrochloride (3.72 g) was added under ice-cooling, and the mixture was stirred at room temperature for 1 hr. After methylene chloride was evaporated under reduced pressure, 5% aqueous citric acid solution (50 mL) was added to the residue, and the mixture was twice extracted with ethyl acetate (50 mL). The ethyl acetate layers were combined, washed successively with saturated aqueous sodium hydrogen carbonate and saturated brine, and dried over sodium sulfate. Then, ethyl acetate was evaporated under reduced pressure. The residue was purified by column chromatography to give the title compound as a powder (8.83 g).
IR ν (Nujol) cm⁻¹; 1759, 1699, 1531, 1234, 1196, 1161, 1082. ¹H-NMR (CDCl₃) δ (ppm); 1.50 (9H, s), 1.70 (3H, br-s), 1.97, 2.03 (6H, s, s), 2.29 (3H, s), 3.04 (1H, dd, J=15.4, 5.9 Hz), 3.37 (1H, dd, J=15.4, 3.3 Hz), 4.63 (2H, br-s), 4.92 (1H, br-t), 5.23 (2H, s), 6.48 (1H, br-s), 6.9-7.5 (10H, m).

### (4) N-(4-acetoxy-2,3,5-trimethylphenyl)-7-tert-butoxycarbonylamino-1,2,3,4-tetrahydroisoquinoline-(3S)-carboxamide

10% Palladium carbon (400 mg) was suspended in methanol (160 mL), the compound (8.43 g) of (3) was added, and the mixture was subjected to catalytic hydrogenation at 30°C for 2.5 hr under 3 kgf/cm². Chloroform was added to dissolve the precipitate, palladium carbon was removed by filtration, and then solvent was evaporated under reduced pressure. The residue was washed with diethyl ether (40 mL) and collected by filtration to give the title compound as a powder (4.42 g).
IR ν (Nujol) cm⁻¹; 1742, 1726, 1666, 1531, 1232, 1201, 1165.
¹H-NMR (CDCl₃) δ (ppm); 1.51 (9H, s), 1.86 (1H, br-s), 2.07 (3H, s), 2.12 (6H, s), 2.29 (3H, s), 2.85 (1H, dd, J=16.0, 9.5 Hz), 3.27 (1H, dd, J=16.0, 5.5 Hz), 3.70 (1H, dd, J=9.5, 5.5 Hz), 4.01 (2H, s), 6.47 (1H, br-s), 6.9-7.2 (2H, m), 7.2-7.4 (1H, m), 7.64 (1H, s), 9.21 (1H, br-s).

### (5) N-(4-acetoxy-2,3,5-trimethylphenyl)-7-tert-butoxycarbonylamino-2-hexanoyl-1,2,3,4-tetrahydroisoquinoline-(3S)-carboxamide

The compound (800 mg) of (4) was dissolved in methylene chloride (8 mL), triethylamine (0.29 mL) and hexanoyl chloride (0.26 mL) were successively added at room temperature, and the mixture was stirred at the same temperature for 40 min. Methylene chloride (20 mL) was added to the reaction mixture, and the mixture was washed successively with water, 5% aqueous citric acid solution and saturated brine, and dried over sodium sulfate. Then, methylene chloride was evaporated under reduced pressure. The residue was purified by column chromatography to give the title compound as a powder (960 mg).
IR ν (Nujol) cm⁻¹; 1759, 1726, 1634, 1537, 1196, 1161, 1082, 1055.
¹H-NMR (CDCl₃) δ (ppm); 0.91 (3H, br-t), 1.2-1.8 (6H, m), 1.51 (9H, s), 2.03 (9H, s), 2.29 (3H, s), 2.93 (1H, dd, J=15.7, 6.3 Hz), 3.43 (1H, dd, J=15.7, 3.8 Hz), 4.3-5.5 (3H, m), 6.51 (1H, br-s), 6.8-7.6 (4H, m), 8.16 (1H, br-s).

### (6) N-(4-acetoxy-2,3,5-trimethylphenyl)-7-amino-2-hexanoyl-1,2,3,4-tetrahydroisoquinoline-(3S)-carboxamide hydrochloride

The compound (929 mg) of (5) was dissolved in formic acid (2.5 mL), 10 M hydrogen chloride - isopropanol solution (0.25 mL) was added under ice-cooling, and the mixture was stirred at the same temperature for 40 min. Diethyl ether (25 mL) was added, and the precipitate was collected by filtration to give the title compound as a powder (766 mg).
IR ν (Nujol) cm⁻¹; 1755, 1657, 1539, 1229, 1198, 1082.
¹H-NMR (DMSO-d₆) δ (ppm); 0.89 (3H, br-t), 1.2-2.0 (6H, m), 1.75 (3H, s), 1.94, 1.98 (6H, s, s), 2.30 (3H, s), 2.3-2.7 (2H, m), 3.0-3.6 (2H, m), 4.4-5.3 (5H, m), 6.74 (1H, s), 7.0-7.5 (3H, m), 8.14, 9.22, 9.57 (1H, s, s, s).

### Example 95: 2-hexanoyl-N-(4-hydroxy-2,3,5-trimethylphenyl)-7-methanesulfonylamino-1,2,3,4-tetrahydroisoquinoline-(3S)-carboxamide

The compound (350 mg) of Example 80 was dissolved in chloroform (3.5 mL), pyridine (0.12 mL) and methanesulfonyl chloride (0.06 mL) were added under ice-cooling, and the mixture was stirred at room temperature for 1 hr. 10% Aqueous citric acid solution was added to the reaction solution, and the precipitate was collected by filtration and washed with water to give the title compound as a powder (297 mg).
IR ν (Nujol) cm⁻¹; 3219, 1649, 1634.
¹H-NMR (DMSO-d₆) δ (ppm); 0.70-1.10 (3H, br-t), 1.10-1.80 (6H, m), 2.03 (9H, s), 2.10-2.70 (2H, m), 2.70-3.50 (2H, m), 2.94 (3H, s), 4.40-5.20 (3H, m), 6.45 (1H, s), 6.80-7.30 (3H, m), 7.60-8.20 (1H, br), 8.88, 9.15 (1H, s, s), 9.62 (1H, s).

### Example 96: 7-acetylamino-2-hexanoyl-N-(4-hydroxy-2,3,5-trimethylphenyl)-1,2,3,4-tetrahydroisoquinoline-(3S)-carboxamide

The title compound was synthesized according to Example 95.
IR ν (Nujol) cm⁻¹; 1651, 1537, 1246, 1207, 1099.
¹H-NMR (CDCl₃+DMSO-d₆) δ (ppm); 0.90 (3H, br-t), 1.1-1.8 (6H, m), 1.85 (3H, s), 2.10 (9H, s), 2.51 (2H, br-t), 2.8-3.6 (3H, m), 4.3-5.5 (3H, m), 6.86 (1H, br-s), 6.5-8.1 (4H, m), 8.94 (1H, br-s).

### Example 97: 7-acetoimidoylamino-2-hexanoyl-N-(4-hydroxy-2,3,5-trimethylphenyl)-1,2,3,4-tetrahydroisoquinoline-(3S)-carboxamide hydrochloride

### (1) N-(4-acetoxy-2,3,5-trimethylphenyl)-7-acetoimidoylamino-2-hexanoyl-1,2,3,4-tetrahydroisoquinoline-(3S)-carboxamide

The compound (0.66 g) of Example 94 was dissolved in ethanol (6.6 mL), benzylthioacetoimidate hydrobromide [synthesized according to the method described in Tetrahedron Lett.,38 (2), 179-182 (1997)] (0.52 g) was added, and the mixture was stirred at room temperature for 1 hr. Water (50 mL) was added to the reaction solution, and the mixture was washed with diethyl ether. After pH of the diethyl ether layer was adjusted to 9 with 2 M aqueous sodium hydroxide solution, the mixture was twice extracted with chloroform (50 mL). The chloroform layers were combined and dried over sodium sulfate, and then chloroform was evaporated under reduced pressure. The obtained residue was washed with diisopropyl ether to give the title compound as a powder (0.58 g).
IR ν (Nujol) cm⁻¹; 3201, 1757, 1645, 1608.
¹H-NMR (CDCl₃) δ (ppm); 0.70-1.10 (3H, br-t), 1.20-1.90 (6H, m), 2.01 (9H, s), 2.20-2.60 (2H, m), 2.30 (6H, s), 2.80-3.80 (2H, m), 4.00-5.40 (3H, m), 6.50-7.40 (5H, m), 7.43 (1H, s), 8.14 (1H, s).

### (2) 7-acetoimidoylamino-2-hexanoyl-N-(4-hydroxy-2,3,5-trimethylphenyl)-1,2,3,4-tetrahydroisoquinoline-(3S)-carboxamide hydrochloride

The compound (454 mg) of (1) was dissolved in methanol (5.0 mL), 1 M lithium hydroxide (2.69 mL) was added, and the mixture was stirred at room temperature for 0.5 hr. Chloroform was added to the reaction solution, and the aqueous layer was separated and dried over sodium sulfate. Then, chloroform was evaporated under reduced pressure.

The obtained residue was dissolved in methanol (5.0 mL), 10 M hydrogen chloride-isopropanol solution (0.27 mL) was added under ice-cooling, and the mixture was stirred at the same temperature for 10 min. Water (40 mL) was added, and the mixture was concentrated under reduced pressure and purified by reversed-phase column chromatography. The obtained residue was washed with diethyl ether to give the title compound as a powder (360 mg).
IR ν (Nujol) cm⁻¹; 3234, 1672, 1629.
¹H-NMR (DMSO-d₆) δ (ppm); 0.70-1.10 (3H, br-t), 1.00-2.00 (6H, m), 2.04, 2.06 (9H, s, s), 2.30-2.70 (2H, m), 2.37 (3H, s), 2.80-3.50 (2H, m), 4.30-5.20 (3H, m), 6.53 (1H, s), 7.00-7.50 (3H, m), 7.98 (1H, br-s), 8.50 (1H, br-s), 9.01, 9.35 (1H, s, s), 9.63 (1H, br-s), 11.20-11.90 (1H, br).

### Example 98: 7-amino-2-[(2E),(4E)-hexadienoyl]-N-(4-hydroxy-2,3,5-trimethylphenyl)-1,2,3,4-tetrahydroisoquinoline-(3S)-carboxamide hydrochloride

### (1) 7-tert-butoxycarbonylamino-2-[(2E),(4E)-hexadienoyl]-N-(4-hydroxy-2,3,5-trimethylphenyl)-1,2,3,4-tetrahydroisoquinoline-(3S)-carboxamide

The compound (400 mg) of Example 94(4) was dissolved in methylene chloride (4 mL), triethylamine (0.14 mL) and sorbyl chloride (124 mg) were successively added under ice-cooling, and the mixture was stirred at the same temperature for 1 hr. The reaction mixture was washed successively with 5% aqueous citric acid solution (10 mL) and saturated brine, and dried over sodium sulfate. Then, methylene chloride was evaporated under reduced pressure.

The obtained residue was dissolved in a mixed solution of tetrahydrofuran - methanol (3:1, 7 mL), 1 M aqueous lithium hydroxide solution (3.1 mL) was added at room temperature, and the mixture was stirred at the same temperature for 3 hr. After the reaction mixture was neutralized with 10% aqueous citric acid solution, tetrahydrofuran-methanol was evaporated under reduced pressure, and the residue was twice extracted with ethyl acetate (20 mL). The ethyl acetate layers were combined, washed successively with saturated aqueous sodium hydrogen carbonate and saturated brine, and dried over sodium sulfate. Then, ethyl acetate was evaporated under reduced pressure. The residue was purified by column chromatography to give the title compound as a powder (400 mg).
IR ν (Nujol) cm⁻¹; 3600-3100, 1651, 1622, 1236, 1159, 997.
¹H-NMR (CDCl₃) δ (ppm); 1.45 (9H, s), 1.7-2.0 (6H, m), 2.07 (6H, s), 2.8-3.6 (2H, m), 4.5-5.6 (3H, m), 6.0-6.7 (3H, m), 6.52 (1H, br-s), 6.8-7.5 (5H, m), 7.8-8.0 (1H, m).

### (2) 7-amino-2-[(2E),(4E)-hexadienoyl]-N-(4-hydroxy-2,3,5-trimethylphenyl)-1,2,3,4-tetrahydroisoquinoline-(3S)-carboxamide hydrochloride

According to Example 94 (6), the title compound was obtained as a powder (192 mg) from the compound (327 mg) of (1).
IR ν (Nujol) cm⁻¹; 3600-3100, 1651, 1589, 1242, 1211, 1094, 1005.
¹H-NMR (DMSO-d₆) δ (ppm); 1.70 (3H, s), 1.83 (3H, d, J=4.6 Hz), 2.03, 2.14 (6H, s, s), 3.0-3.6 (2H, m), 4.5-5.4 (4H, m), 6.0-6.8 (3H, m), 6.52 (1H, s), 7.0-7.5 (4H,m), 9.0-9.5 (1H, m).

The compounds of Example 99-Example 105 were synthesized according to Example 98.

### Example 99: 7-amino-N-(4-hydroxy-2,3,5-trimethylphenyl)-2-[5-methyl-(2E)-hexenoyl]-1,2,3,4-tetrahydroisoquinoline-(3S)-carboxamide hydrochloride

IR ν (Nujol) cm⁻¹; 1651, 1589, 1207, 1096, 980.
¹H-NMR (DMSO-d₆) δ (ppm); 0.91 (6H, d, J=6.1 Hz), 1.7-2.3 (3H, m), 1.94, 2.03 (9H, s, s), 3.0-3.6 (2H, m), 4.5-5.4 (3H, m), 6.2-6.8 (3H, m), 7.0-7.5 (3H, m), 8.9-9.4 (1H, m).

### Example 100: 7-amino-2-(5-hexenoyl)-N-(4-hydroxy-2,3,5-trimethylphenyl)-1,2,3,4-tetrahydroisoquinoline-(3S)-carboxamide hydrochloride

IR ν (Nujol) cm⁻¹; 1653, 1622, 1599, 1545, 1211, 1090.
¹H-NMR (DMSO-d₆) δ (ppm); 1.5-2.7 (6H, m), 1.70 (3H, s), 2.03, 2.05 (6H, s, s), 2.9-3.5 (2H, m), 4.6-5.3 (5H, m), 5.6-6.2 (1H, m), 6.49 (1H, s), 7.0-7.5 (3H, m), 8.99, 9.34 (1H, br-s, br-s).

### Example 101: 7-amino-2-butyryl-N-(4-hydroxy-2,3,5-trimethylphenyl)-1,2,3,4-tetrahydroisoquinoline-(3S)-carboxamide hydrochloride

IR ν (Nujol) cm⁻¹; 3600-3100, 1622, 1506, 1242, 1096.
¹H-NMR (DMSO-d₆) δ (ppm); 0.94 (3H, br-t), 1.4-2.0 (2H, m), 1.67 (3H, br-s), 2.05 (6H, s), 2.1-2.7 (2H, m), 2.9-3.6 (2H, m), 4.4-5.3 (3H, m), 6.50 (1H, s), 7.0-7.5 (3H, m), 9.00 (1H, br-s).

### Example 102: 7-amino-N-(4-hydroxy-2,3,5-trimethylphenyl)-2-(4-methylpentanoyl)-1,2,3,4-tetrahydroisoquinoline-(3S)-carboxamide hydrochloride

IR ν (Nujol) cm⁻¹; 3600-3100, 1628, 1242, 1097.
¹H-NMR (DMSO-d₆) δ (ppm); 0.91 (6H, d, J=5.0 Hz), 1.4-1.9 (3H, m), 1.66 (3H, s), 2.05 (6H, s), 2.1-2.6 (2H, m), 3.0-3.6 (2H, m), 4.4-5.3 (3H, m), 6.49 (1H, s), 7.1-7.6 (3H, m), 8.99, 9.36 (1H, br-s, br-s).

### Example 103: 7-amino-N-(4-hydroxy-2,3,5-trimethylphenyl)-2-pentanoyl-1,2,3,4-tetrahydroisoquinoline-(3S)-carboxamide hydrochloride

IR ν (Nujol) cm⁻¹; 3600-3100, 1622, 1096.
¹H-NMR (DMSO-d₆) δ (ppm); 0.91 (3H, br-t), 1.2-1.8 (4H, m), 1.66, 1.71 (3H, s, s), 2.03, 2.05 (6H, s, s), 2.3-2.7 (2H, m), 2.9-3.6 (2H, m), 4.4-5.3 (3H, m), 7.0-7.5 (4H, m), 8.99 (1H, br-s).

### Example 104: 7-amino-2-(2,2-dimethylbutyryl)-N-(4-hydroxy-2,3,5-trimethylphenyl)-1,2,3,4-tetrahydroisoquinoline-(3S)-carboxamide hydrochloride

IR ν (Nujol) cm⁻¹; 3600-3100, 1655, 1506, 1479, 1412, 1244, 1175.
¹H-NMR (DMSO-d₆) δ (ppm); 0.79 (3H, br-t), 1.24 (6H, s), 1.5-1.9 (2H, m), 1.83 (3H, s), 2.06 (6H, s), 3.0-3.6 (2H, m), 4.3-5.2 (3H, m), 6.60 (1H, s), 7.0-7.5 (3H, m), 9.17 (1H, br-s).

### Example 105: 7-amino-2-(3,3-dimethylbutyryl)-N-(4-hydroxy-2,3,5-trimethylphenyl)-1,2,3,4-tetrahydroisoquinoline-(3S)-carboxamide hydrochloride

IR ν (Nujol) cm⁻¹; 1622, 1504, 1229, 1096.
¹H-NMR (DMSO-d₆) δ (ppm); 1.04 (9H, s), 1.74 (3H, s), 2.05 (6H, s), 2.4-2.6 (2H, m), 3.0-3.4 (2H, m), 4.4-5.3 (3H, m), 6.53 (1H, s), 7.0-7.4 (3H, m), 9.04 (1H, br-s).

### Example 106: 2-(3,3-dimethylbutyryl)-N-(4-hydroxy-2,3,5-trimethylphenyl)-7-methanesulfonylamino-1,2,3,4-tetrahydroisoquinoline-(3S)-carboxamide

The title compound was synthesized using the compound of Example 105 according to Example 86.
IR ν (Nujol) cm⁻¹; 1622, 1506, 1321, 1229, 1151, 976, 521.
¹H-NMR (DMSO-d₆) δ (ppm); 1.04 (9H, s), 1.72 (3H, s), 2.04 (6H, s, s), 2.4-2.7 (2H, m), 2.9-3.6 (2H, m), 2.94 (3H, s), 4.4-5.2 (3H, m), 6.49 (1H, s), 6.9-7.3 (3H, m), 7.8-8.2 (1H, br), 8.92 (1H, br-s), 9.61 (1H, br-s).

### Example 107: 7-amino-2-(3,3-dimethylbutyryl)-N-(4-methoxy-2,3,5-trimethylphenyl)-1,2,3,4-tetrahydroisoquinoline-(3S)-carboxamide hydrochloride

### (1) 2-(3,3-dimethylbutyryl)-N-(4-hydroxy-2,3,5-trimethylphenyl)-7-nitro-1,2,3,4-tetrahydroisoquinoline-(3S)-carboxamide

The title compound was synthesized according to Example 80.
¹H-NMR (CDCl₃) δ (ppm); 1.12 (9H, s), 2.02 (3H, s), 2.13 (6H, s), 2.43, 2.50 (2H, AB-q, J=14.5 Hz), 3.07, 3.15-3.30 (1H, dd, m, J=16.4, 6.2 Hz), 3.58, 3.65-3.75 (1H, dd, m, J=16.4, 3.8 Hz), 4.60-5.10, 4.62, 4.87 (3.2H, m, AB-q, J=15.8 Hz), 5.54 (0.8H, dd, J=6.2, 3.8 Hz), 7.11 (1H, s), 7.46 (1H, d, J=8.3
Hz), 8.05 (1H, d, J=1.8 Hz), 8.08 (1H, br-s), 8.12 (1H, dd, J=8.3, 1.8 Hz).

### (2) 2-(3,3-dimethylbutyryl)-N-(4-methoxy-2,3,5-trimethylphenyl)-7-nitro-1,2,3,4-tetrahydroisoquinoline-(3S)-carboxamide

The compound (495 mg) of (1) was suspended in acetone (30 mL), methyl iodide (0.34 mL) and potassium carbonate (452 mg) were successively added, and the mixture was stirred at room temperature for 15 hr, and further refluxed for 2 hr. Methyl iodide (0.34 mL) was added, and the mixture was refluxed for 7 hr. Additional methyl iodide (0.34 mL) was added, and the mixture was refluxed for 12 hr. After allowing to cool, potassium carbonate was removed by filtration, and acetone was evaporated under reduced pressure. Water (20 mL) was added to the residue, and the mixture was extracted with chloroform (30 mL). The chloroform layer was washed with saturated brine and dried over sodium sulfate. Then, chloroform was evaporated under reduced pressure. The obtained powdery residue was washed with diethyl ether (10 mL) and collected by filtration to give the title compound as a powder (410 mg).
IR ν (Nujol) cm⁻¹; 1668, 1652, 1403, 1344, 1250, 1088, 997, 888, 874.
¹H-NMR (CDCl₃) δ (ppm); 1.12 (9H, s), 2.03 (3H, s), 2.18 (6H, s), 2.45 (2H, s), 2.8-3.8 (2H, m), 3.62 (3H, s), 4.3-5.0 (2H, m), 5.55 (1H, dd, J=6.4, 4.4 Hz), 7.26 (1H, s), 7.45 (1H, d, J=8.1 Hz), 8.0-8.4 (3H, m).

### (3) 7-amino-2-(3,3-dimethylbutyryl)-N-(4-methoxy-2,3,5-trimethylphenyl)-1,2,3,4-tetrahydroisoquinoline-(3S)-carboxamide hydrochloride

10% Palladium carbon (40 mg) was suspended in methanol (10 mL), the compound (400 mg) of (2) was added thereto, and the mixture was subjected to catalytic hydrogenation at 30°C for 2.5 hr under 3 kgf/cm². After palladium carbon was removed by filtration, methanol was evaporated under reduced pressure. The residue was purified by column chromatography.

The residue (190 mg) was suspended in methanol (0.5 mL), 10 M hydrogen chloride-isopropanol solution (0.07 mL) was added under ice-cooling, and the mixture was stirred at the same temperature for 10 min. Diethyl ether (10 mL) was added, and the precipitate was collected by filtration to give the title compound as a powder (168 mg).
IR ν (Nujol) cm⁻¹; 1616, 1506, 1231, 1097, 1003.
¹H-NMR (DMSO-d₆) δ (ppm); 1.04 (9H, s), 1.79 (3H, s), 2.08, 2.12 (6H, s, s), 2.4-2.7 (2H, m), 3.1-3.6 (2H, m), 3.56 (3H, s), 4.4-5.0 (2H, m), 5.10 (1H, br-t), 6.69 (1H, s), 7.1-7.4 (3H, m), 9.18 (1H, br-s).

### Example 108: 7-acetylsulfamoylamino-N-(4-hydroxy-2,3,5-trimethylphenyl)-2-isobutyryl-1,2,3,4-tetrahydroisoquinoline-(3S)-carboxamide

N-(4-acetoxy-2,3,5-trimethylphenyl)-7-amino-2-isobutyryl-1,2,3,4-tetrahydroisoquinoline-(3S)-carboxamide (1.00 g) synthesized according to Example 80 was suspended in methylene chloride (30 mL), pyridine (0.24 mL) was added at room temperature. Then, the compound (470 mg) of Reference Example 12 was added in portions over 20 min, and the mixture was further stirred at the same temperature for 30 min. Water (20 mL) and diethyl ether (20 mL) were added to the reaction mixture, and the mixture was stirred for 30 min, and then the precipitate was collected by filtration.

The obtained powder (1.7 g) was suspended in methanol (9 mL), 1 M aqueous lithium hydroxide solution (3.0 mL) was added under ice-cooling, and the mixture was stirred at room temperature for 2 hr. After the reaction mixture was acidified with 10% aqueous citric acid solution, methanol was evaporated under reduced pressure, and the residue was twice extracted with ethyl acetate (20 mL). The ethyl acetate layers were combined, washed successively with 10% aqueous citric acid solution and saturated brine, and dried over sodium sulfate. Then, ethyl acetate was evaporated under reduced pressure. The residue was washed with ethyl acetate (20 mL) and collected by filtration to give the title compound as a powder (617 mg).
IR ν (Nujol) cm⁻¹; 1699, 1614, 1240, 1165, 1088, 914, 536.
¹H-NMR (DMSO-d₆) δ (ppm); 1.00-1.15 (6H, m), 1.65 (1H, s), 1.70 (2H, s), 1.86 (3H, s), 2.03, 2.06 (6H, s, s), 2.75-2.85 (0.35H, m), 3.00-3.35 (2.65H, m), 4.46 (0.35H, d, J=16.8 Hz), 4.60-4.80 (1.65H, m), 4.93 (0.35H, br-t), 5.02 (0.65H, t, J=5.4 Hz), 6.49 (0.35H, s), 6.54 (0.65H, s), 6.95-7.05 (2H, m), 7.10-7.20 (1H, m), 7.97, 8.00 (1H, s, s), 8.98 (0.65H, s), 9.21 (0.35H, s), 10.34 (1H, s), 11.62, 11.65 (1H, s, s).

### Example 109: 7-acetylsulfamoylamino-N-(4-hydroxy-2,3,5-trimethylphenyl)-2-(2,2-dimethylpropionyl)-1,2,3,4-tetrahydroisoquinoline-(3S)-carboxamide

The title compound was synthesized according to Example 108.
IR ν (Nujol) cm⁻¹; 3180, 1703, 1668, 1618, 1510.
¹H-NMR (DMSO-d₆) δ (ppm); 1.27 (9H, s), 1.77, 1.86, 2.04, 2.07 (12H, s, s, s, s), 3.00-3.40 (2H, m), 4.52, 4.85 (2H, AB-q, J=16.0 Hz), 4.90-5.10 (1H, br), 6.59 (1H, s), 6.90-7.20 (3H, m), 7.98 (1H, s), 9.10 (1H, s), 10.31, 11.62 (2H, s, s).

### Example 110: 2-hexanoyl-7-hydroxy-N-(2,4,6-trimethylphenyl)-1,2,3,4-tetrahydroisoquinoline-(3S)-carboxamide

### (1) 7-benzyloxy-2-hexanoyl-6,8-diiodo-N-(2,4,6-trimethylphenyl)-1,2,3,4-tetrahydroisoquinoline-(3S)-carboxamide

According to Example 2, the title compound was obtained as a powder (2.53 g) from methyl 7-benzyloxy-6,8-diiodo-1,2,3,4-tetrahydroisoquinoline-(3S)-carboxylate (3.00 g).
IR ν (Nujol) cm-¹; 1657, 1533, 1256, 947, 908, 750, 694.
¹H-NMR (CDCl₃) δ (ppm); 0.91 (3H, br-t), 1.0-2.0 (6H, m), 2.02 (6H, s), 2.22 (3H, s), 2.59 (2H, br-t), 2.97 (1H, dd, J=15.6, 6.3 Hz), 3.45 (1H, dd, J=15.6, 4.2 Hz), 4.4-5.2 (2H, m), 4.98 (2H, s), 5.38 (1H, dd, J=6.3, 4.2 Hz), 6.82 (2H, s), 7.2-8.0 (7H, m).

### (2) 7-benzyloxy-2-hexanoyl-N-(2,4,6-trimethylphenyl)-1,2,3,4-tetrahydroisoquinoline-(3S)-carboxamide

10% Palladium carbon (250 mg) was suspended in methanol (50 mL), the compound (2.52 g) of (1) and triethylamine (1.2 mL) were added thereto, and the mixture was subjected to hydrogenation at 40°C for 3.5 hr under 3.5 kgf/cm². After palladium carbon was removed by filtration, methanol was evaporated under reduced pressure. 5% Aqueous citric acid solution was added to the residue, and the mixture was extracted with ethyl acetate (30 mL). The ethyl acetate layer was washed successively with 5% aqueous citric acid solution and saturated brine, and dried over sodium sulfate. Then, ethyl acetate was evaporated under reduced pressure, and the residue was purified by column chromatography. After the obtained residue was dissolved by heating in chloroform (2 mL), diethyl ether (40 mL) was added, and the mixture was stirred for 30 min. The precipitate was collected by filtration to give the title compound as a powder (1.12 g).
¹H-NMR (CDCl₃) δ (ppm); 0.91 (3H, br-t), 1.0-2.0 (6H, m), 1.68, 1.92 (6H, s, s), 2.20 (3H, s), 2.50 (2H, br-t), 2.8-3.7 (2H, m), 4.4-5.0 (2H, m), 5.04 (2H, s), 5.29 (1H, br-t), 6.7-7.2 (4H, m), 7.2-7.8 (7H, m).

### (3) 2-hexanoyl-7-hydroxy-N-(2,4,6-trimethylphenyl)-1,2,3,4-tetrahydroisoquinoline-(3S)-carboxamide

20% Palladium hydroxide carbon (110 mg) was suspended in methanol (25 mL), the compound (1.12 g) of (2) was added thereto, and the mixture was subjected to hydrogenation at room temperature for 3 days under 3.5 kgf/cm². After palladium hydroxide was removed by filtration, methanol was evaporated under reduced pressure, and the residue was purified by column chromatography. The obtained residue was dissolved by heating in a mixed solvent (10 mL) of chloroform-methanol (20:1), diethyl ether (50 mL) was added, and the mixture was stirred for 1 hr. The precipitate was collected by filtration to give the title compound as a powder (758 mg).
IR ν (Nujol) cm⁻¹; 3600-3100, 1651, 1614, 1504, 1227, 852.
¹H-NMR (DMSO-d₆) δ (ppm); 0.88 (3H, br-t), 1.0-2.0 (6H, m), 1.77 (6H, s), 2.0-2.6 (2H, m), 2.17 (3H, s), 2.8-3.4 (2H, m), 4.4-4.7 (2H, m), 4.7-5.2 (1H, m), 6.5-6.9 (2H, m), 6.76 (2H, s), 6.97 (1H, d, J=7.7 Hz), 8.72, 9.08, 9.17 (2H, br-s, br-s, br-s).

The compounds of Example 111-Example 113 were synthesized according to Example 110.

### Example 111: N-(2,6-diisopropylphenyl)-7-hydroxy-2-octanoyl-1,2,3,4-tetrahydroisoquinoline-(3S)-carboxamide

IR ν (Nujol) cm⁻¹; 1661, 1614, 1288, 812.
¹H-NMR (DMSO-d₆) δ (ppm); 0.6-1.9 (13H, m), 0.90 (12H, d, J=6.4 Hz), 2.0-3.6 (6H, m), 4.4-5.0 (2H, m), 5.08 (1H, br-t), 6.5-6.7 (2H, m), 6.9-7.2 (4H, m), 8.68, 9.21 (2H, br-s, br-s).

### Example 112: N-(2,4-difluorophenyl)-7-hydroxy-2-octanoyl-1,2,3,4-tetrahydroisoquinoline-(3S)-carboxamide

IR ν (Nujol) cm⁻¹; 1670, 1620, 1296, 1261, 1140, 1097, 964, 839. ¹H-NMR (DMSO-d₆) δ (ppm); 0.86 (3H, br-t), 1.0-1.8 (10H, m), 2.2-2.6 (2H, m), 2.8-3.3 (2H, m), 4.2-5.2 (3H, m), 6.4-7.7 (6H, m), 9.21, 9.54 (1H, br-s, br-s).

### Example 113: N-(2,6-diisopropylphenyl)-2-(2,2-dimethylpropionyl)-7-hydroxy-1,2,3,4-tetrahydroisoquinoline-(3S)-carboxamide

IR ν (Nujol) cm⁻¹; 1642, 1623, 1313, 1230, 1106, 857, 802.
¹H-NMR (CDCl₃+CD₃OD) δ (ppm); 1.04, 1.07 (12H, d, d, J=6.6 Hz, 6.6 Hz), 1.40 (9H, s), 2.5-3.0 (2H, m), 2.9-3.8 (2H, m), 4.60, 4.90 (2H, AB-q, J=15.8 Hz), 5.20 (1H, t, J=5.5 Hz), 6.6-6.8 (2H, m), 7.0-7.4 (4H, m), 7.80 (1H, br-s).

### Example 114: N-(4-amino-2,6-diisopropylphenyl)-2-hexanoyl-7-hydroxy-1,2,3,4-tetrahydroisoquinoline-(3S)-carboxamide hydrochloride

### (1) 7-benzyloxy-N-(4-benzyloxycarbonylamino-2,6-diisopropylphenyl)-2-hexanoyl-1,2,3,4-tetrahydroisoquinoline-(3S)-carboxamide

The title compound was synthesized according to Example 110.
IR ν (Nujol) cm⁻¹; 1605, 1219, 1051, 739, 696.
¹H-NMR (CDCl₃) δ (ppm); 0.8-1.2 (15H, m), 1.0-2.0 (6H, m), 2.2-2.8 (2H, m), 2.60 (2H, t, J=7.6 Hz), 2.8-3.7 (2H, m), 4.3-5.4 (3H, m), 5.04 (2H, s), 5.17 (2H, s), 6.64 (1H, br-s), 6.7-7.6 (16H, m).

### (2) N-(4-amino-2,6-diisopropylphenyl)-2-hexanoyl-7-hydroxy-1,2,3,4-tetrahydroisoquinoline-(3S)-carboxamide hydrochloride

20% Palladium hydroxide carbon (60 mg) was suspended in methanol (15 mL), the compound (687 mg) of (1) was added, and the mixture was subjected to catalytic hydrogenation at 25°C for 3 hr under 3 kgf/cm². After palladium carbon was removed by filtration, methanol was evaporated under reduced pressure, and the residue was purified by column chromatography.

The obtained residue was suspended in methanol (0.8 mL), 10 M hydrogen chloride - isopropanol solution (0.2 mL) was added under ice-cooling, and the mixture was stirred at the same temperature for 15 min. Diethyl ether (20 mL) was added, and the precipitate was collected by filtration to give the title compound as a powder (410 mg).
IR ν (Nujol) cm⁻¹; 3600-3100, 1614, 1506, 1104, 955.
¹H-NMR (DMSO-d₆) δ (ppm); 0.6-1.8 (21H, m), 2.1-2.8 (4H, m), 2.8-3.4 (2H, m), 4.3-5.4 (3H, m), 6.61, 6.68 (2H, s, s), 6.8-7.3 (3H, m), 8.84 (1H, br-s).

### Example 115: 7-carboxymethyloxy-2-octanoyl-N-(2,4,6-trimethylphenyl)-1,2,3,4-tetrahydroisoquinoline-(3S)-carboxamide

Compound 7-hydroxy-2-octanoyl-N-(2,4,6-trimethylphenyl)-1,2,3,4-tetrahydroisoquinoline-(3S)-carboxamide (500 mg) synthesized according to Example 110 was dissolved in ethanol (20 mL), ethyl bromoacetate (385 mg) and potassium carbonate (316 mg) were added, and the mixture was stirred at 70°C for 10 hr. Ethanol was evaporated under reduced pressure, and the residue was dissolved in methanol (50 mL). A solution of sodium hydroxide (250 mg) in water (10 mL) was added, and the mixture was stirred at room temperature for 1 hr. After methanol was evaporated under reduced pressure, the residue was neutralized with 1 M hydrochloric acid. The precipitate was recrystallized from methanol, and the crystals were collected by filtration to give the title compound as crystals (300 mg).
IR ν (Nujol) cm⁻¹; 2923, 1666, 1610.
¹H-NMR (DMSO-d₆) δ (ppm); 0.87 (3H, br-t), 1.0-2.3 (12H, m), 1.76 (6H, s), 2.16 (3H, s), 2.9-3.7 (2H, m), 4.60 (2H, s), 4.4-5.2 (3H, m), 6.6-7.3 (5H, m), 8.77 (1H, br-s).

### Example 116: 2-hexanoyl-7-hydroxy-N-(4-hydroxy-2,3,5-trimethylphenyl)-1,2,3,4-tetrahydroisoquinoline-(3S)-carboxamide

### (1) N-(4-acetoxy-2,3,5-trimethylphenyl)-7-benzyloxy-2-hexanoyl-1,2,3,4-tetrahydroisoquinoline-(3S)-carboxamide

The title compound was synthesized according to Example 110 (1)-(2).
IR ν (Nujol) cm⁻¹; 1755, 1651, 1506, 1225, 1198, 1155, 1084.
¹H-NMR (CDCl₃) δ (ppm); 0.90 (3H, br-t), 1.1-2.0 (6H, m), 1.58 (3H, s), 2.04 (6H, s), 2.2-2.6 (2H, m), 2.30 (3H, s), 2.7-3.6 (2H, m), 4.4-5.4 (3H, m), 5.04 (2H, s), 6.7-7.0 (2H, m), 7.0-7.6 (7H, m), 8.13 (1H, br-s).

### (2) 2-hexanoyl-7-hydroxy-N-(4-hydroxy-2,3,5-trimethylphenyl)-1,2,3,4-tetrahydroisoquinoline-(3S)-carboxamide

20% Palladium hydroxide carbon (50 mg) was suspended in methanol (20 mL), the compound (508 mg) of (1) was added, and the mixture was subjected to catalytic hydrogenation at 25°C for 17 hr under 3 kgf/cm². Chloroform was added to dissolve the precipitate, and palladium carbon was removed by filtration. Then, solvent was evaporated under reduced pressure.

The obtained residue was dissolved in a mixed solution of tetrahydrofuran - methanol (3:1, 14 mL), 1 M aqueous lithium hydroxide solution (3.5 mL) was added, and the mixture was stirred at room temperature for 1 hr. After the reaction mixture was neutralized with 10% aqueous citric acid solution, tetrahydrofuran-methanol was evaporated under reduced pressure, and the residue was twice extracted with ethyl acetate (15 mL). The ethyl acetate layers were combined, washed successively with saturated aqueous sodium hydrogen carbonate and saturated brine, and dried over sodium sulfate. Then, ethyl acetate was evaporated under reduced pressure. The residue was purified by column chromatography to give the title compound as a powder (244 mg).
IR ν (Nujol) cm⁻¹; 3600-3100, 1636, 1508, 1223, 1096.
¹H-NMR (DMSO-d₆) δ (ppm); 0.88 (3H, br-t), 1.1-2.0 (6H, m), 1.68 (3H, s), 2.03 (6H, s), 2.2-2.6 (2H, m), 2.7-3.6 (2H, m), 4.3-5.0 (2H, m), 5.01 (1H, br-t), 6.4-6.8 (3H, m), 6.97 (1H, d, J=7.9 Hz), 7.92, 8.84, 9.11, 9. 19 (3H, br-s, br-s, br-s, br-s).

### Example 117: 7-(2-aminoethoxy)-2-hexanoyl-N-(4-hydroxy-2,3,5-trimethylphenyl)-1,2,3,4-tetrahydroisoquinoline-(3S)-carboxamide hydrochloride

### (1) 4-nitrobenzyl N-{[(3S)-(4-acetoxy-2,3,5-trimethylphenyl)carbamoyl-2-hexanoyl-1,2,3,4-tetrahydroisoquinolin-7-yl]oxyethyl}carbamate

According to Example 2, the title compound was obtained as a powder.
IR ν (Nujol) cm⁻¹; 3284, 1757, 1724.
¹H-NMR (CDCl₃) δ (ppm); 0.70-1.10 (3H, m), 1.10-2.00 (6H, m), 2.03 (9H, s), 2.30 (3H, s), 2.20-2.60 (2H, m), 2.70-3.40 (2H, m), 3.40-3.80 (2H, m), 3.90-4.20 (2H, m), 4.40-4.90 (3H, m), 5.20 (2H, s), 5.30 (1H, br-t), 6.60-7.30 (3H, m), 7.44 (1H, br-s), 7.49 (2H, br-d), 8.14 (1H, br-s), 8.19 (2H, br-d).

### (2) N-(4-acetoxy-2,3,5-trimethylphenyl)-7-(2-aminoethoxy)-2-hexanoyl-1,2,3,4-tetrahydroisoquinoline-(3S)-carboxamide

5% Palladium carbon (300 mg) was suspended in methanol (30 mL), the compound (1.50 g) of (1) was added thereto, and the mixture was subjected to catalytic hydrogenation at 40°C for 0.5 hr under 3.0 kgf/cm². After palladium carbon was removed by filtration, methanol was evaporated under reduced pressure. The obtained residue was purified by column chromatography to give the title compound as a powder (0.73 g). IR ν (Nujol) cm⁻¹; 3261, 1755, 1645.
¹H-NMR (CDCl₃) δ (ppm); 0.70-1.10 (3H, m), 1.10-1.90 (6H, m), 2.04 (9H, s), 2.30 (3H, s), 2.20-2.60 (2H, m), 2.70-3.20 (1H, m), 3.06 (2H, t, J=5.2 Hz), 3.43 (1H, dd, J=15.4, 4.1 Hz), 3.96 (2H, t, J=5.2 Hz), 4.30-5.40 (3H, m), 6.50-7.80 (2H, br), 6.60-7.30 (3H, m), 7.45 (1H, br-s), 8.15 (1H, br-s).

### (3) 7-(2-aminoethoxy)-2-hexanoyl-N-(4-hydroxy-2,3,5-trimethylphenyl)-1,2,3,4-tetrahydroisoquinoline-(3S)-carboxamide hydrochloride

The compound (0.72 g) of (2) was dissolved in methanol (7.2 mL), 1 M aqueous lithium hydroxide solution (2.8 mL) was added, and the mixture was stirred at room temperature for 0.5 hr. Chloroform was added to the reaction mixture, and the aqueous layer was separated and dried over sodium sulfate. Then, chloroform was evaporated under reduced pressure.

The obtained residue was dissolved in chloroform (7.6 mL), 10 M hydrogen chloride-isopropanol solution (0.37 mL) was added under ice-cooling, and the mixture was stirred at the same temperature for 10 min. Water (60 mL) was added, and the mixture was concentrated under reduced pressure and purified by reversed-phase column chromatography. The obtained residue was washed with diethyl ether to give the title compound as a powder (436 mg).
IR ν (Nujol) cm⁻¹; 3369, 1670, 1635.
¹H-NMR (DMSO-d₆) δ (ppm); 0.60-1.10 (3H, m), 1.10-1.90 (6H, m), 2.04 (9H, s), 2.20-2.60 (2H, m), 2.80-3.60 (4H, m), 3.80-4.40 (2H, m), 4.40-5.20 (3H, m), 6.52 (1H, s), 6.60-7.40 (3H, m), 7.80-8.60 (4H, br), 8.95, 9.30 (1H, s, s).

### Example 118: 2-hexanoyl-N-(4-hydroxy-2,3,5-trimethylphenyl)-7-[2-(pyrrolidin-1-yl)ethoxy]-1,2,3,4-tetrahydroisoquinoline-(3S)-carboxamide hydrochloride

### (1) N-(4-acetoxy-2,3,5-trimethylphenyl)-2-hexanoyl-7-[2-(pyrrolidin-1-yl)ethoxy]-1,2,3,4-tetrahydroisoquinoline-(3S)-carboxamide

The compound (1.85 g) of Example 117 was dissolved in N,N-dimethylformamide (18 mL), potassium carbonate (1.25 g) and 1,4-dibromobutane (0.48 mL) were added, and the mixture was stirred at 50-60°C for 14 hr. Water was added to the reaction solution, and the mixture was twice extracted with ethyl acetate (150 mL). The ethyl acetate layers were combined, washed successively with water and saturated brine, and dried over sodium sulfate. Then, ethyl acetate was evaporated under reduced pressure. The obtained residue was purified by column chromatography to give the title compound as a powder (1.05 g). IR ν (Nujol) cm⁻¹; 3263, 1757, 1654.
¹H-NMR (CDCl₃) δ (ppm); 0.70-1.10 (3H, m), 1.10-2.10 (10H, m), 2.04 (9H, s), 2.30 (3H, s), 2.30-2.80 (6H, m), 2.80-3.20 (1H, m), 2.88 (2H, t, J=5.9 Hz), 3.43 (1H, dd, J=15.8, 4.1 Hz), 4.08 (2H, t, J=5.9 Hz), 4.30-5.40 (3H, m), 6.60-7.30 (3H, m), 7.45 (1H, br-s), 8.13 (1H, br-s).

### (2) 2-hexanoyl-N-(4-hydroxy-2,3,5-trimethylphenyl)-7-[2-(pyrrolidin-1-yl)ethoxy]-1,2,3,4-tetrahydroisoquinoline-(3S)-carboxamide hydrochloride

According to Example 48 (2), the title compound was obtained as a powder (585 mg) from the compound (1.05 g) of (1).
IR ν (Nujol) cm⁻¹; 3360, 3196, 2611, 2482, 2364, 1622.
¹H-NMR (DMSO-d₆) δ (ppm); 0.60-1.10 (3H, m), 1.10-2.30 (12H, m), 2.04 (9H, s), 2.30-3.80 (8H, m), 4.10-4.40 (2H, m), 4.40-5.20 (3H, m), 6.52 (1H, s), 6.70-7.30 (3H, m), 7.97 (1H, br-s), 8.95, 9.30 (1H, s, s), 10.60-11.30 (1H, br).

### Example 119:2-hexanoyl-7-(3-hydroxypropoxy)-N-(4-hydroxy-2,3,5-trimethylphenyl)-1,2,3,4-tetrahydroisoquinoline-(3S)-carboxamide

### (1) methyl 7-(3-benzyloxypropoxy)-2-tert-butoxycarbonyl-1,2,3,4-tetrahydroisoquinoline-(3S)-carboxylate

Methyl 2-tert-butoxycarbonyl-7-hydroxy-1,2,3,4-tetrahydroisoquinoline-(3S)-carboxylate (2.00 g), 1-benzyloxy-3-bromopropane (1.79 g), potassium carbonate (2.70 g) and tetraethylammonium fluoride (487 mg) were suspended in toluene (20 mL), and the suspension was stirred at 80°C for 12 hr. After allowing to cool, water (20 mL) was added, and the mixture was twice extracted with ethyl acetate (20 mL). The organic layers were combined, washed with saturated brine, and dried over sodium sulfate. Then, solvent was evaporated under reduced pressure. The residue was purified by column chromatography to give the title compound as an oil (2.92 g).
IR ν (neat) cm⁻¹; 1742, 1699, 1614, 1506, 1393, 1169, 866, 739, 698.
¹H-NMR (CDCl₃) δ (ppm); 1.46, 1.51 (9H, s, s), 1.8-2.2 (2H, m), 2.8-3.3 (2H, m), 3.5-3.8 (2H, m), 3.64 (3H, s), 4.04 (2H, t, J=6.1 Hz), 4.3-5.3 (3H, m), 4.51 (2H, s), 6.5-6.8 (2H, m), 7.05 (1H, d, J=8.6 Hz), 7.1-7.5 (5H, m).

### (2) 2-hexanoyl-7-(3-hydroxypropoxy)-N-(4-hydroxy-2,3,5-trimethylphenyl)-1,2,3,4-tetrahydroisoquinoline-(3S)-carboxamide

The title compound was synthesized according to Example 117.
IR ν (neat) cm⁻¹; 3600-3100, 1622, 1097, 1061, 999, 864.
¹H-NMR (CDCl₃+DMSO-d₆) δ (ppm); 0.91 (3H, br-t), 1.1-2.3 (8H, m), 1.89 (3H, s), 2.11 (6H, s), 2.52 (2H, br-t), 2.6-3.6 (2H, m), 3.78 (2H, t, J=6.0 Hz), 4.08 (2H, t, J=6.0 Hz), 4.4-5.4 (3H, m), 6.0-7.0 (1H, br), 6.5-7.0 (3H, m), 7.13 (1H, d, J=8.3 Hz), 8.00 (1H, br-s).

### Example 120: N-(2,6-diisopropylphenyl)-2-hexanoyl-7-(3-hydroxypropoxy)-1,2,3,4-tetrahydroisoquinoline-(3S)-carboxamide

The title compound was obtained according to Example 119. IR ν (Nujol) cm⁻¹; 1634, 1504, 1059, 799, 741.
¹H-NMR (CDCl₃) δ (ppm); 0.6-1.9 (23H, m), 1.9-2.3 (2H, m), 2.53 (2H, br-t), 2.8-3.6 (2H, m), 3.82 (2H, t, J=5.9 Hz), 4.09 (2H, t, J=5.9 Hz), 4.4-5.5 (3H, m), 6.5-6.8 (2H, m), 6.8-7.6 (5H, m).

### Example 121: 2-hexanoyl-N-(4-hydroxy-2,3,5-trimethylphenyl)-7-(piperazin-1-yl)-1,2,3,4-tetrahydroisoquinoline-(3S)-carboxamide

### (1) methyl 2-tert-butoxycarbonyl-7-trifluoromethanesulfonyloxy-1,2,3,4-tetrahydroisoquinoline-(3S)-carboxylate

Methyl 2-tert-butoxycarbonyl-7-hydroxy-1,2,3,4-tetrahydroisoquinoline-(3S)-carboxylate (5.00 g) was dissolved in methylene chloride (50 mL), 2,6-lutidine (2.7 mL) was added under ice-cooling. Then, trifluoromethanesulfonic anhydride (5.53 g) was added dropwise over 20 min, and the mixture was further stirred at the same temperature for 30 min. After methylene chloride was evaporated under reduced pressure, 1 M hydrochloric acid (25 mL) was added to the residue, and the mixture was extracted with diethyl ether (50 mL). The diethyl ether layer was washed successively with 1 M hydrochloric acid, saturated aqueous sodium hydrogen carbonate and saturated brine, and dried over sodium sulfate. Then, diethyl ether was evaporated under reduced pressure. The residue was purified by column chromatography to give the title compound as a viscous oil (6.92 g).
IR ν (neat) cm⁻¹; 1744, 1701, 1421, 1396, 1213, 1142, 949, 876. ¹H-NMR (CDCl₃) δ (ppm); 1.51 (9H, s), 3.0-3.3 (2H, m), 3.64 (3H, s), 4.3-5.3 (3H, m), 6.9-7.3 (3H, m).

### (2) methyl 7-(4-benzylpiperazin-1-yl)-2-tert-butoxycarbonyl-1,2,3,4-tetrahydroisoquinoline-(3S)-carboxylate

Under nitrogen atmosphere, palladium acetate (85 mg) and 2,2'-bis(diphenylphosphino)-1,1'-binaphthyl(rac-BINAP) (262 mg) were suspended in dehydrated dioxane (1 mL), and the suspension was stirred for 30 min. Then, a solution of the compound (1.42 g) of (1) and 1-benzylpiperazine (684 mg) dissolved in dioxane (4 mL) and cesium carbonate (1.47 g) were added. The reaction mixture was aerated with nitrogen for 10 min, and stirred at 80°C for 5 hr. After allowing to cool, ethyl acetate (20 mL) was added to the reaction mixture. Insoluble material was removed by filtration, and solvent was evaporated under reduced pressure. The residue was purified by column chromatography to give the title compound as a viscous oil (745 mg).
IR ν (neat) cm⁻¹; 1747, 1699, 1510, 1013, 880, 743, 700.
¹H-NMR (CDCl₃) δ (ppm); 1.6-1.8 (9H, m), 2.3-2.8 (4H, m), 3.0-3.4 (6H, m), 3.56 (2H, s), 3.61 (3H, s), 4.3-5.2 (3H, m), 6.6-6.9 (2H, m), 7.01 (1H, d, J=8.3 Hz), 7.2-7.5 (5H, m).

### (3) methyl 7-(4-benzylpiperazin-1-yl)-2-hexanoyl-1,2,3,4-tetrahydroisoquinoline-(3S)-carboxylate

According to Example 30 (2)-(3), the title compound was obtained as a viscous oil (794 mg) from the compound (988 mg) of (2).
¹H-NMR (CDCl₃) δ (ppm); 0.91 (3H, br-t), 1.1-2.0 (6H, m), 2.2-2.8 (6H, m), 3.0-3.4 (6H, m), 3.56 (2H, s), 3.60 (3H, s), 4.3-5.6 (3H, m), 6.6-6.9 (2H, m), 7.03 (1H, d, J=8.1 Hz), 7.2-7.5 (5H, m).

### (4) N-(4-acetoxy-2,3,5-trimethylphenyl)-7-(4-benzylpiperazin-1-yl)-2-hexanoyl-1,2,3,4-tetrahydroisoquinoline-(3S)-carboxamide

According to Example 2 (2)-(3), the title compound was obtained as a powder (782 mg) from the compound (781 mg) of (3).
¹H-NMR (CDCl₃) δ (ppm); 0.90(3H, br-t), 1.0-2.0 (6H, m), 1.98 (3H, s), 2.04 (6H, s), 2.30 (3H, s), 2.2-2.8 (6H, m), 2.9-3.5 (6H, m), 3.57 (2H, s), 4.3-5.4 (3H, m), 6.6-7.0 (2H, m), 7.0-7.6 (7H, m), 8.09 (1H, br-s).

### (5) 7-(4-benzylpiperazin-1-yl)-2-hexanoyl-N-(4-hydroxy-2,3,5-trimethylphenyl)-1,2,3,4-tetrahydroisoquinoline-(3S)-carboxamide

According to Example 48 (2), the title compound was obtained as a powder (590 mg) from the compound (834 mg) of (4).
IR ν (Nujol) cm⁻¹; 1651, 1514, 1236, 1200, 1144, 1097, 866, 741, 698.
¹H-NMR (CDCl₃) δ (ppm); 0.96 (3H, br-t), 1.0-2.0 (6H, m), 1.86 (3H, s), 2.11 (6H, s), 2.2-3.6 (12H, m), 3.56 (2H, s), 4.4-5.4 (3H, m), 6.4-7.6 (9H, m), 7.98 (1H, br-s).

### (6) 2-hexanoyl-N-(4-hydroxy-2,3,5-trimethylphenyl)-7-(piperazin-1-yl)-1,2,3,4-tetrahydroisoquinoline-(3S)-carboxamide

20% Palladium hydroxide carbon (110 mg) was suspended in methanol (20 mL), the compound (570 mg) of (5) was added, and the mixture was subjected to catalytic hydrogenation at 40°C for 9 hr under 4 kgf/cm². Chloroform was added to dissolve the precipitate, catalyst was removed by filtration, and solvent was evaporated under reduced pressure. The residue was purified by column chromatography to give the title compound as a powder (283 mg).
IR ν (Nujol) cm⁻¹; 1622, 1506, 1244, 1097, 866.
¹H-NMR (DMSO-d₆) δ (ppm); 0.87 (3H, br-t), 1.0-1.8 (6H, m), 1.69 (3H, s), 2.04 (6H, s), 2.1-4.0 (13H, m), 4.4-5.2 (3H, m), 6.51 (1H, s), 6.6-7.2 (3H, m), 9.13 (1H, br-s).

### Example 122: 2-(2,2-dimethylpropionyl)-N-(4-hydroxy-2,3,5-trimethylphenyl)-7-(4-methylpiperazin-1-yl)-1,2,3,4-tetrahydroisoquinoline-(3S)-carboxamide

### (1) N-(4-acetoxy-2,3,5-trimethylphenyl)-7-(4-benzylpiperazin-1-yl)-2-(2,2-dimethylpropionyl)-1,2,3,4-tetrahydroisoquinoline-(3S)-carboxamide

The title compound was synthesized according to Example 121.
¹H-NMR (CDCl₃) δ (ppm); 1.37 (9H, s), 1.95 (3H, br-s), 2.03, 2.07 (6H, s, s), 2.31 (3H, s), 2.61 (4H, t, J=4.9 Hz), 3.01 (1H, dd, J=15.7, 6.6 Hz), 3.16 (4H, t, J=4.9 Hz), 3.41 (1H, dd, J=15.7, 4.9 Hz), 4.57 (2H, s), 4.50, 4.89 (2H, AB-q, J=15.9 Hz), 5.24 (1H, dd, J=6.6, 4.9 Hz), 6.68 (1H, d, J=2.4 Hz), 6.82 (1H, dd, J=8.3, 2.4 Hz), 7.11 (1H, d, J=8.3 Hz), 7.25-7.45 (5H, m), 7.39 (1H, br-s), 7.82 (1H, br-s).

### (2) N-(4-acetoxy-2,3,5-trimethylphenyl)-2-(2,2-dimethylpropionyl)-7-(4-methylpiperazin-1-yl)-1,2,3,4-tetrahydroisoquinoline-(3S)-carboxamide

20% Palladium hydroxide carbon (270 mg) was suspended in methanol (13 mL), and the compound (674 mg) of (1), formalin (0.25 mL) and 2 M hydrochloric acid (1.1 mL) were added, and the mixture was subjected to catalytic hydrogenation at 35°C for 3 days under 4 kgf/cm². After catalyst was removed by filtration, methanol was evaporated under reduced pressure. Saturated aqueous sodium hydrogen carbonate was added to the residue, and the mixture was extracted with ethyl acetate (30 mL). The ethyl acetate layer was washed with saturated brine and dried over sodium sulfate. Then, ethyl acetate was evaporated under reduced pressure. The residue was purified by column chromatography to give the title compound as a powder (406 mg).
¹H-NMR (CDCl₃) δ (ppm); 1.38 (9H, s), 1.94 (3H, br-s), 2.03, 2.06 (6H, s, s), 2.31 (3H, s), 2.36 (3H, s), 2.59 (4H, t, J=4.9 Hz), 3.01 (1H, dd, J=15.7, 6.6 Hz), 3.18 (4H, t, J=4.9 Hz), 3.41 (1H, dd, J=15.7, 4.5 Hz), 4.52, 4.90 (2H, AB-q, J=15.8 Hz), 5.25(1H, dd, J=6.6, 4.5 Hz), 6.69 (1H, d, J=2.3 Hz), 6.83 (1H, dd, J=8.2, 2.3 Hz), 7.13 (1H, d, J=8.2 Hz), 7.39 (1H, br-s), 7.81 (1H, br-s).

### (3) 2-(2,2-dimethylpropionyl)-N-(4-hydroxy-2,3,5-trimethylphenyl)-7-(4-methylpiperazin-1-yl)-1,2,3,4-tetrahydroisoquinoline-(3S)-carboxamide

The compound (407 mg) of (2) was dissolved in a mixed solution of tetrahydrofuran-methanol (3:1, 7 mL), 1 M aqueous lithium hydroxide solution (2.3 mL) was added dropwise over 10 min under ice-cooling, and the mixture was stirred at the same temperature for 1 hr. After 2 M hydrochloric acid (1.2 mL) was added, tetrahydrofuran-methanol was evaporated under reduced pressure. Saturated aqueous sodium hydrogen carbonate was added, and the mixture was extracted with ethyl acetate (30 mL). The ethyl acetate layer was washed successively with saturated aqueous sodium hydrogen carbonate and saturated brine, and dried over sodium sulfate. Then, ethyl acetate was evaporated under reduced pressure. The obtained residue was dissolved by heating in methanol (2 mL), diethyl ether (20 mL) was added, and the mixture was stirred for 1 hr. The precipitate was collected by filtration to give the title compound as a powder (265 mg).
IR ν (Nujol) cm⁻¹; 3283, 1682, 1603, 1539, 1292, 1244, 1180, 1153, 1097, 1015, 833.
¹H-NMR (CDCl₃) δ (ppm); 1.38 (9H, s), 1.85 (3H, br-s), 2.07, 2.10 (6H, s, s), 2.35 (3H, s), 2.58 (4H, t, J=5.0 Hz), 3.02 (1H, dd, J=15.6, 6.4 Hz), 3.17 (4H, t, J=5.0 Hz), 3.42 (1H, dd, J=15.6, 4.9 Hz), 4.56, 4.89 (2H, AB-q, J=15.9 Hz), 4.95-5.20 (1H, br), 5.21 (1H, dd, J=6.4, 4.9 Hz), 6.71 (1H, d, J=2.2 Hz), 6.83 (1H, dd, J=8.3, 2.2 Hz), 6.91 (1H, br-s), 7.12 (1H, d, J=8.3 Hz), 7.56 (1H, br-s).

### Example 123: 7-aminomethyl-2-(2,2-dimethylbutyryl)-N-(4-hydroxy-2,3,5-trimethylphenyl)-1,2,3,4-tetrahydroisoquinoline-(3S)-carboxamide hydrochloride

### (1) methyl 2-tert-butoxycarbonyl-7-cyano-1,2,3,4-tetrahydroisoquinoline-(3S)-carboxylate

Under nitrogen atmosphere, palladium acetate (153 mg) and rac-BINAP (467 mg) were dissolved in dehydrated N-methyl-2-pyrrolidinone (3 mL), and the solution was stirred at room temperature for 1 hr. Potassium cyanide (892 mg) and a solution of the compound (3.00 g) of Example 121 (1) in N-methyl-2-pyrrolidinone (6 mL) were successively added, and the mixture was stirred at 80°C for 10 hr. After allowing to cool, water (60 mL) and diethyl ether (20 mL) were added, and the mixture was stirred for 10 min. Then, insoluble material was removed by filtration. The diethyl ether layer of the filtrate was separated, washed with saturated brine, and dried over sodium sulfate. Then, diethyl ether was evaporated under reduced pressure. The residue was purified by column chromatography. The obtained residue was washed with n-hexane (10 mL) and collected by filtration to give the title compound as a powder (1.65 g).
IR ν (neat) cm⁻¹; 2230, 1745, 1697, 1396, 1167, 1130, 1013, 756. ¹H-NMR (CDCl₃) δ (ppm); 1.50 (9H, s, s), 3.0-3.4 (2H, m), 3.63 (3H, s), 4.3-5.3 (3H, m), 7.25 (1H, d, J=8.1 Hz), 7.3-7.5 (2H, m).

### (2) methyl aminomethyl-2-tert-butoxycarbonyl-1,2,3,4-tetrahydroisoquinoline-(3S)-carboxylate

20% Palladium hydroxide carbon (0.22 g) was suspended in methanol (22 mL), the compound (2.19 g) of (1) and 28% aqueous ammonia (8.4 g) were successively added, and the mixture was subjected to catalytic hydrogenation at 35°C for 15 hr under 4 kgf/cm². After catalyst was removed by filtration, methanol was evaporated under reduced pressure. The residue was dissolved in chloroform, and the solution was dried over sodium sulfate. Then, chloroform was evaporated under reduced pressure. The residue was purified by column chromatography to give the title compound as an oil (712 mg).
IR ν (neat) cm⁻¹; 1745, 1697, 1396, 1367, 1167, 1130, 1013, 756. ¹H-NMR (CDCl₃) δ (ppm); 1.47, 1.50 (9H, s, s), 1.62 (2H, br-s), 3.0-3.4 (2H, m), 3.62 (3H, s), 3.82 (2H, s), 4.3-5.3 (3H, m), 7.0-7.4 (3H, m).

### (3) methyl 7-(benzyloxycarbonylamino)methyl-2-tert-butoxycarbonyl-1,2,3,4-tetrahydroisoquinoline-(3S)-carboxylate

The compound (900 mg) of (2) was dissolved in ethyl acetate (9 mL), magnesium oxide (340 mg) and benzyl chloroformate (0.60 mL) were successively added, and the mixture was stirred at room temperature for 24 hr. After insoluble material was removed by filtration, ethyl acetate was evaporated under reduced pressure. The residue was purified by column chromatography to give the title compound as an oil (882 mg).
IR ν (neat) cm⁻¹; 1699, 1394, 1242, 1167, 1045, 1013, 698.
¹H-NMR (CDCl₃) δ (ppm); 1.47, 1.51 (9H, s, s), 3.0-3.3 (2H, m), 3.61 (3H, s), 4.33 (2H, d, J=5.8 Hz), 4.4-5.4 (3H, m), 5.14 (2H, s), 7.0-7.2 (4H, m), 7.2-7.5 (5H, m).

### (4) N-(4-acetoxy-2,3,5-trimethylphenyl)-7-(benzyloxycarbonylamino)methyl-2-tert-butoxycarbonyl-1,2,3,4-tetrahydroisoquinoline-(3S)-carboxamide

According to Example 2 (2)-(3), the title compound was obtained as a powder (558 mg) from the compound (492 mg) of (3).
IR ν (Nujol) cm⁻¹; 1759, 1699, 1520, 1229, 1196, 1082.
¹H-NMR (CDCl₃) δ (ppm); 1.52 (9H, s), 1.80 (3H, br-s), 1.99, 2.03 (6H, s, s), 2.29 (3H, s), 3.05 (1H, dd, J=15.2, 6.2 Hz), 3.40 (1H, dd, J=15.2, 3.1 Hz), 4.34 (2H, d, J=5.9 Hz), 4.4-5.4 (3H, m), 5.13 (2H, s), 7.0-8.0 (11H, m).

### (5) N-(4-acetoxy-2,3,5-trimethylphenyl)-7-(benzyloxycarbonylamino)methyl-1,2,3,4-tetrahydroisoquinoline-(3S)-carboxamide

According to Example 27 (3), the title compound was obtained as a powder (324 mg) from the compound (530 mg) of (4).
IR ν (Nujol) cm⁻¹; 1753, 1688, 1537, 1234, 1196, 1080, 694.
¹H-NMR (CDCl₃) δ (ppm); 1.79 (3H, br-s), 1.8-2.2 (1H, br), 2.07, 2.11 (6H, s, s), 2.32 (3H, s), 2.88 (1H, dd, J=16.4, 9.7 Hz), 3.29 (1H, dd, J=16.4, 5.1 Hz), 3.70 (1H, dd, J=9.7, 5.1 Hz), 4.00 (2H, s), 4.32 (2H, d, J=5.8Hz), 5.13 (2H, s), 6.9-7.6 (9H, m), 7.64 (1H, br-s), 9.19 (1H, br-s).

### (6) N-(4-acetoxy-2,3,5-trimethylphenyl)-7-(benzyloxycarbonylamino)methyl-2-(2,2-dimethylbutyryl)-1,2,3,4-tetrahydroisoquinoline-(3S)-carboxamide

According to Example 30 (3), the title compound was obtained as a powder (336 mg) from the compound (300 mg) of (5).
IR ν (Nujol) cm⁻¹; 1757, 1699, 1227, 1194, 1082, 1045, 698.
¹H-NMR (CDCl₃) δ (ppm); 0.83 (3H, br-t), 1.31 (6H, s), 1.75 (2H, br-q), 2.03 (9H, s), 2.31 (3H, s), 3.00 (1H, dd, J=15.9, 6.6 Hz), 3.49 (1H, dd, J=15.9, 4.5 Hz), 4.35 (2H, d, J=5.9 Hz), 4.47, 4.98 (2H, AB-q, J=15.8 Hz), 5.14 (2H, s), 5.33 (1H, dd, J=6.6, 4.5 Hz), 7.0-7.6 (9H, m), 7.43 (1H, s), 8.00 (1H, br-s).

### (7) 7-(benzyloxycarbonylamino)methyl-2-(2,2-dimethylbutyryl)-N-(4-hydroxy-2,3,5-trimethylphenyl)-1,2,3,4-tetrahydroisoquinoline-(3S)-carboxamide

According to Example 48 (2), the title compound was obtained as a powder (245 mg) from the compound (325 mg) of (6).
IR ν (Nujol) cm⁻¹; 1682, 1668, 1607, 1242, 1045, 984, 698.
¹H-NMR (CDCl₃) δ (ppm); 0.85 (3H, br-t), 1.32, 1.34 (6H, s, s), 1.76 (2H, br-q), 1.93 (3H, s), 2.10, 2.12 (6H, s, s), 3.02 (1H, dd, J=16.0, 6.7 Hz), 3.50 (1H, dd, J=16.0, 5.0 Hz), 4.34 (2H, d, J=5.9 Hz), 4.4-5.4 (1H, br), 4.50, 4.97 (2H, AB-q, J=16.0 Hz), 5.13 (2H, s), 5.30 (1H, dd, J=6.7, 5.0 Hz), 7.0-7.5 (10H, m), 7.75 (1H, br-s).

### (8) 7-aminomethyl-2-(2,2-dimethylbutyryl)-N-(4-hydroxy-2,3,5-trimethylphenyl)-1,2,3,4-tetrahydroisoquinoline-(3S)-carboxamide hydrochloride

10% Palladium carbon (10 mg) was suspended in methanol (10 mL), the compound (238 mg) of (7) was added, and the mixture was subjected to catalytic hydrogenation at room temperature for 4 hr under 2 kgf/cm². After palladium carbon was removed by filtration, solvent was evaporated under reduced pressure.

The obtained residue (162 mg) was dissolved in methanol (0.5 mL). Under ice-cooling, 10 M hydrochloric acid (0.06 mL) was added, and the mixture was stirred for 5 min. Then, diethyl ether (15 mL) was added, and the mixture was stirred for 30 min. The precipitate was collected by filtration to give the title compound as a powder (150 mg).
IR ν (Nujol) cm⁻¹; 1668, 1607, 1231, 1096.
¹H-NMR (DMSO-d₆) δ (ppm); 0.80 (3H, br-t), 1.24 (6H, s, s), 1.6-2.0 (2H, m), 1.85 (3H, s), 2.06 (6H, s), 3.0-3.6 (2H, m), 3.8-4.2 (2H, m), 4.4-5.2 (3H, m), 6.62 (1H, s), 7.1-7.5 (3H, m), 7.5-8.8 (3H, br), 9.22 (1H, br-s).

### Example 124: 7-dimethylaminomethyl-2-(2,2-dimethylbutyryl)-N-(4-hydroxy-2,3,5-trimethylphenyl)-1,2,3,4-tetrahydroisoquinoline-(3S)-carboxamide hydrochloride

### (1) N-(4-acetoxy-2,3,5-trimethylphenyl)-7-dimethylaminomethyl-2-(2,2-dimethylbutyryl)-1,2,3,4-tetrahydroisoquinoline-(3S)-carboxamide

10% Palladium carbon (30 mg) was suspended in methanol (7 mL), the compound (360 mg) of Example 123 (6) was added, and the mixture was subjected to catalytic hydrogenation at 30°C for 13 hr under 3 kgf/cm². After palladium carbon was removed by filtration, methanol was evaporated under reduced pressure.

The obtained residue was dissolved in methanol (4 mL), formalin (192 mg) and sodium cyanoborohydride (82 mg) were successively added at room temperature, and the mixture was stirred for 45 min. Methanol was evaporated under reduced pressure, saturated aqueous sodium hydrogen carbonate (10 mL) was added, and the mixture was twice extracted with ethyl acetate (15 mL). The ethyl acetate layers were combined, washed with saturated brine, and dried over sodium sulfate. Then, ethyl acetate was evaporated under reduced pressure. The residue was purified by column chromatography to give the title compound as a powder (208 mg).
¹H-NMR (CDCl₃) δ (ppm); 0.85 (3H, t, J=7.4 Hz), 1.31, 1.34 (6H, s, s), 1.70-1.80 (2H, m), 2.00 (3H, br-s), 2.04, 2.07 (6H, s, s), 2.23 (6H, s), 2.32 (3H, s), 3.05 (1H, dd, J=16.1, 6.7 Hz), 3.40 (2H, s), 3.50 (1H, dd, J=16.1, 5.2 Hz), 4.52, 4.97 (2H, AB-q, J=16.2 Hz), 5.31 (1H, dd, J=6.7, 5.2 Hz), 7.10-7.35 (3H, m), 7.41 (1H, s), 7.99 (1H, br-s).

### (2) 7-dimethylaminomethyl-2-(2,2-dimethylbutyryl)-N-(4-hydroxy-2,3,5-trimethylphenyl)-1,2,3,4-tetrahydroisoquinoline-(3S)-carboxamide hydrochloride

According to Example 48 (2), the title compound was obtained as a powder (171 mg) from the compound (208 mg) of (1).
IR ν (Nujol) cm⁻¹; 1668, 1614, 1408, 1204, 1177, 1097.
¹H-NMR (DMSO-d₆) δ (ppm); 0.79 (3H, br-t), 1.23, 1.24 (6H, s, s), 1.65-1.80 (2H, m), 1.80 (3H, br-s), 2.04, 2.06 (6H, s, s), 2.68 (6H, s), 3.20-3.50 (2H, m), 4.23 (2H, s), 4.61, 4.92 (2H, AB-q, J=16.1 Hz), 4.95-5.20 (1H, m), 6.58 (1H, br-s), 7.30-7.45 (3H, m), 8.00 (1H, br-s), 9.20 (1H, br-s), 9.00-11.00 (1H, br- s).

The compounds of Example 125 and Example 126 were synthesized according to Example 124.

### Example 125: 7-dimethylaminomethyl-2-(2,2-dimethylbutyryl)-N-(3-hydroxy-2,4-dimethylphenyl)-1,2,3,4-tetrahydroisoquinoline-(3S)-carboxamide hydrochloride

IR ν (Nujol) cm⁻¹; 3600-3000, 1674, 1653, 1178, 1084, 949, 808. ¹H-NMR (DMSO-d₆) δ (ppm); 0.79 (3H, t, J=7.4 Hz), 1.23, 1.24 (6H, s, s), 1.60-1.80 (2H, m), 1.83 (3H, br-s), 2.11 (3H, s), 2.68 (6H, s), 3.20-3.50 (2H, m), 4.22 (2H, s), 4.62, 4.93 (2H, AB-q, J=16.4 Hz), 5.00-5.20 (1H, m), 6.50-6.60 (1H, m), 6.80 (1H, d, J=7.8 Hz), 7.30-7.45 (3H, m), 8.17 (1H, s), 9.28 (1H, br-s).

### Example 126: 7-dimethylaminomethyl-2-(2,2-dimethylpropionyl)-N-(4-hydroxy-2,3,5-trimethylphenyl)-1,2,3,4-tetrahydroisoquinoline-(3S)-carboxamide hydrochloride

IR ν (Nujol) cm⁻¹; 1668, 1622, 1412, 1202, 1180.
¹H-NMR (DMSO-d₆) δ (ppm); 1.29 (9H, s), 1.77 (3H, br-s), 2.04, 2.06 (6H, s, s), 2.66, 2.67 (6H, s, s), 3.15-3.40 (2H, m), 4.21 (2H, br-d), 4.60, 4.91 (2H, AB-q, J=16.7 Hz), 4.95-5.20 (1H, m), 6.56 (1H, br-s), 7.31 (1H, d, J=7.6 Hz), 7.39 (1H, d, J=7.6 Hz), 7.44 (1H, s), 8.00 (1H, br-s), 9.21 (1H, br-s), 10.53 (1H, br-s).

### Example 127: 3-[(2,6-diisopropylphenyl)carbamoyl]-2-(2,2-dimethylbutyryl)-1,2,3,4-tetrahydroisoquinoline-7-carboxylic acid

### (1) 2-tert-butoxycarbonyl-7-cyano-1,2,3,4-tetrahydroisoquinoline-(3S)-carboxylic acid

The compound (1.00 g) of Example 123 (1) was dissolved in a mixed solution of tetrahydrofuran-methanol (3:1, 13 mL), 1 M aqueous lithium hydroxide solution (3.2 mL) was added under ice-cooling, and the mixture was stirred at the same temperature for 1.5 hr. 1 M aqueous lithium hydroxide solution (0.79 mL) was further added, and the mixture was stirred for 1.5 hr. After the reaction mixture was acidified with 10% aqueous citric acid solution, tetrahydrofuran-methanol was evaporated under reduced pressure, and the residue was twice extracted with ethyl acetate (20 mL). The ethyl acetate layers were combined, washed with saturated brine, and dried over sodium sulfate. Then, ethyl acetate was evaporated under reduced pressure to give the title compound as a powder (990 mg).
IR ν (Nujol) cm⁻¹; 2230, 1709, 1666, 1506, 1340, 1169, 1132, 1094, 1001.
¹H-NMR (CDCl₃) δ (ppm); 1.48 (9H, s), 3.0-3.4 (1H, m), 3.34 (1H, dd, J=16.6, 5.8 Hz), 4.3-5.3 (4H, m), 7.26 (1H, d, J=7.8 Hz), 7.43 (1H, s), 7.47 (1H, d, J=7.8 Hz).

### (2) 2-tert-butoxycarbonyl-7-cyano-N-(2,6-diisopropylphenyl)-1,2,3,4-tetrahydroisoquinoline-(3S)-carboxamide

According to Example 52 (2), the title compound was obtained as a powder (480 mg) from the compound (937 mg) of (1) and 2,6-diisopropylaniline (605 mg).
¹H-NMR (CDCl₃) δ (ppm); 0.85-1.20 (12H, m), 1.60 (9H, s), 2.70-2.90 (2H, m), 3.15 (1H, dd, J=15.4, 5.5 Hz), 3.65 (1H, dd, J=15.4, 2.6 Hz), 4.55-5.25 (3H, m), 7.10 (2H, d, J=7.8 Hz), 7.25 (1H, t, J=7.8 Hz), 7.42 (1H, d, J=7.9 Hz), 7.50-7.65 (2H, m), 7.60 (1H, d, J=7.9 Hz).

### (3) 7-cyano-N-(2,6-diisopropylphenyl)-2-(2,2-dimethylbutyryl)-1,2,3,4-tetrahydroisoquinoline-(3S)-carboxamide

According to Example 30 (2)-(3), the title compound was obtained as a powder (770 mg) from the compound (780 mg) of (2).
¹H-NMR (CDCl₃) δ (ppm); 0.80-0.95 (3H, m), 1.08, 1.12 (12H, d, d, J=6.4 Hz, 6.8 Hz), 1.35, 1.38 (6H, s, s), 1.70-1.85 (2H, m), 2.60-3.00 (2H, m), 3.12 (1H, dd, J=16.4, 6.8 Hz), 3.64 (1H, dd, J=16.4, 4.9 Hz), 4.61, 5.05 (2H, AB-q, J=15.9 Hz), 5.40 (1H, dd, J=6.8, 4.9 Hz), 7.11 (2H, d, J=7.8 Hz), 7.25 (1H, t, J=7.8 Hz), 7.38 (1H, d, J=7.8 Hz), 7.48 (1H, d, J=1.3 Hz), 7.56 (1H, dd, J=7.8, 1.3 Hz), 7.50-7.65 (1H, br-s).

### (4) 7-carbamoyl-N-(2,6-diisopropylphenyl)-2-(2,2-dimethylbutyryl)-1,2,3,4-tetrahydroisoquinoline-3-carboxamide

The compound (710 mg) of (3) was dissolved in 2-ethoxyethanol (7 mL), 5 M aqueous sodium hydroxide solution (1.54 mL) was added, and the mixture was stirred at 100°C for 40 min. After allowing to cool, the reaction mixture was acidified with 10% aqueous citric acid solution, and twice extracted with diethyl ether (20 mL). The diethyl ether layers were combined, washed with 5% aqueous citric acid solution and saturated brine, and dried over sodium sulfate. Then, diethyl ether was evaporated under reduced pressure. The residue was purified by column chromatography. The obtained residue (0.4 g) was dissolved by heating in ethyl acetate (8 mL), diethyl ether (8 mL) was added, and the precipitate was collected by filtration to give the title compound as a powder (349 mg).
¹H-NMR (CDCl₃) δ (ppm); 0.90 (3H, t, J=7.4 Hz), 0.95-1.20 (12H, m), 1.38, 1.41 (6H, s, s), 1.75-1.95 (2H, m), 2.50-3.10 (2H, m), 3.15 (1H, dd, J=16.0, 6.5 Hz), 3.63 (1H, dd, J=16.0, 4.6 Hz), 4.72, 5.06 (2H, AB-q, J=15.8 Hz), 5.44 (1H, dd, J=6.5, 4.6 Hz), 5.50-5.80, 6.10-6.40 (2H, br, br), 7.13 (2H, d, J=7.8 Hz), 7.27 (1H, t, J=7.8 Hz), 7.33 (1H, d, J=7.8 Hz), 7.60 (1H, br-s), 7.64 (1H, d, J=7.8 Hz), 7.76 (1H, s).

### (5) 3-(2,6-diisopropylphenyl)carbamoyl-2-(2,2-dimethylbutyryl)-1,2,3,4-tetrahydroisoquinoline-7-carboxylic acid

The compound (337 mg) of (4) was dissolved in 2-ethoxyethanol (3 mL), 10 M aqueous sodium hydroxide solution (0.36 mL) was added, and the mixture was stirred at 80°C for 1 hr. Furthermore, 10 M aqueous sodium hydroxide solution (0.36 mL) was added, and the mixture was stirred at the same temperature for 2 hr. After allowing to cool, diethyl ether (20 mL) was added, and the mixture was extracted with water (30 mL). Then, the aqueous layer was acidified to pH 4 with citric acid, and twice extracted with diethyl ether (20 mL). The diethyl ether layers were combined, washed with 10% aqueous citric acid solution and saturated brine, and dried over sodium sulfate. Then, diethyl ether was evaporated under reduced pressure. The residue was purified by column chromatography. The residue was washed with diisopropyl ether (10 mL) and collected by filtration to give the title compound as a powder (163 mg).
IR ν (Nujol) cm⁻¹; 1711, 1616, 1506, 1219, 1184, 1101, 983, 928, 806, 750.
¹H-NMR (CDCl₃) δ (ppm); 0.80-0.95 (3H, m), 1.00-1.20 (12H, m), 1.36, 1.39 (6H, s, s), 1.75-1.90 (2H, m), 2.55-3.00 (2H, m), 3.15 (1H, dd, J=16.1, 6.5 Hz), 3.65 (1H, dd, J=16.1, 5.0 Hz), 4.67, 5.06 (2H, AB-q, J=16.1 Hz), 5.39 (1H, dd, J=6.5, 5.0 Hz), 7.10 (2H, d, J=7.6 Hz), 7.24 (1H, t, J=7.6 Hz), 7.37 (1H, d, J=8.0 Hz), 7.65 (1H, br-s), 7.89 (1H, s), 7.99 (1H, d, J=8.0 Hz).

### Example 128: 2-hexanoyl-3-(2,4,6-trimethylphenyl)carbamoyl-1,2,3,4-tetrahydroisoquinoline-7-carboxylic acid

The title compound was synthesized according to Example 127.
IR ν (Nujol) cm⁻¹; 3240, 1715, 1650, 1610.
¹H-NMR (DMSO-d₆) δ (ppm); 0.85-0.93 (3H, m), 1.25-1.30 (4H, m), 1.50-1.65 (2H, m), 1.65-1.80 (6H, m), 2.50 (3H, s), 2.40-2.70 (2H, m), 3.10-3.50 (2H, m), 4.65-4.90 (2H, m), 4.95-5.10 (1H, m), 6.74, 6.76 (2H, s, s), 7.34 (1H, d, J=7.8 Hz), 7.70-7.80 (1H, m), 7.86 (1H, s), 8.81, 9.19 (1H, s, s), 12.00-13.00 (1H, br).

### Example 129: 3-[2-(2,2-dimethylbutyryl)-(3S)-(4-hydroxy-2,3,5-trimethylphenyl)carbamoyl-1,2,3,4-tetrahydroisoquinolin-7-yl]propionic acid

### (1) benzyl 2-tert-butoxycarbonyl-7-hydroxy-1,2,3,4-tetrahydroisoquinoline-(3S)-carboxylate

Methyl 2-tert-butoxycarbonyl-7-hydroxy-1,2,3,4-tetrahydroisoquinoline-(3S)-carboxylate (4.00 g) was dissolved in a mixed solution of tetrahydrofuran-methanol (3:1, 65 mL), 1 M aqueous lithium hydroxide solution (52 mL) was added under ice-cooling, and the mixture was stirred at 40°C for 4 hr. 2 M hydrochloric acid (26 mL) was added under ice-cooling, and tetrahydrofuran-methanol was evaporated under reduced pressure. 5% Aqueous citric acid solution was added, and the mixture was twice extracted with ethyl acetate (30 mL). The ethyl acetate layers were combined, washed with saturated brine, and dried over sodium sulfate. Then, ethyl acetate was evaporated under reduced pressure.

The obtained residue (4.11 g) and bromobenzyl bromide (2.45 g) were dissolved in N,N-dimethylformamide (20 mL), water (10 mL) and sodium bicarbonate (2.18 g) were added, and the mixture was stirred at 60°C for 11 hr. After allowing to cool, the reaction mixture was poured into ice water (100 mL), and the mixture was twice extracted with diethyl ether (40 mL). The diethyl ether layers were combined, washed successively with saturated aqueous sodium hydrogen carbonate and saturated brine, and dried over sodium sulfate. Then, diethyl ether was evaporated under reduced pressure. The obtained residue was washed with n-hexane (30 mL) and collected by filtration to give the title compound as a white powder (3.53 g).
¹H-NMR (CDCl₃) δ (ppm); 1.41, 1.46 (9H, s, s), 3.00-3.25 (1H, m), 3.49 (1H, dd, J=15.9, 4.5 Hz), 4.45, 4.61 (2H, AB-q, J=16.0 Hz), 4.81 (0.5H, t, J=5.1 Hz), 4.95-5.10 (2H, m), 5.10-5.25 (1H, m), 5.60-5.80 (0.5H, m), 6.55-6.70 (2H, m), 6.90-7.00 (1H, d, J=8.3 Hz), 7.10-7.20 (2H, m), 7.25-7.30 (3H, m).

### (2) methyl 3-[(3S)-benzyloxycarbonyl-2-tert-butoxycarbonyl-1,2,3,4-tetrahydroisoquinolin-7-yl]acrylate

The compound (3.02 g) of (1) was dissolved in methylene chloride (30 mL), 2,6-lutidine (1.4 mL) and trifluoromethanesulfonate anhydride (1.6 g) were added under ice-cooling, and the mixture was stirred at the same temperature for 1.5 hr. Methylene chloride was evaporated under reduced pressure, 1 M hydrochloric acid (30 mL) was added to the residue, and the mixture was extracted with diethyl ether (50 mL). Then, the diethyl ether layer was washed successively with 1 M hydrochloric acid and saturated brine, and dried over sodium sulfate. Then, diethyl ether was evaporated under reduced pressure. The residue was purified by column chromatography.

Under nitrogen atmosphere, palladium acetate (180 mg) and rac-BINAP (548 mg) were suspended in dehydrated dioxane (4 mL), and the suspension was stirred for 20 min. Then, a solution of the residue (4.09 g) dissolved in dioxane (8 mL), diisopropylethylamine (2.8 mL) and methyl acrylate (2.9 mL) were successively added. The reaction mixture was aerated with nitrogen for 10 min, and stirred at 80°C for 20 hr in a sealed tube. After allowing to cool, the reaction mixture was poured into diethyl ether (60 mL), and the mixture was stirred for 30 min. Then, insoluble material was removed by filtration. Solvent was evaporated under reduced pressure, and the residue was purified by column chromatography. The obtained residue was dissolved by heating in methanol (2 mL), and the solution was stood still. The precipitate was collected by filtration to give the title compound as a powder (1.42 g).
¹H-NMR (CDCl₃) δ (ppm) ; 1.41, 1.52 (9H, s, s), 3.10-3.25 (1.5H, m), 3.30 (0.5H, dd, J=16.1, 2.4 Hz), 3.81 (3H, s), 4.51 (1H, dd, J=16.6, 3.4 Hz), 4.68 (1H, d, J=16.6 Hz), 4.87 (0.5H, t, J=5.1 Hz), 4.95-5.10 (2H, m), 5.22 (0.5H, dd, J=6.0, 2.8 Hz), 6.40 (1H, d, J=15.8 Hz), 7.05-7.35 (8H, m), 7.64 (1H, d, J=15.8 Hz).

### (3) methyl 3-[(3S)-(4-acetoxy-2,3,5-trimethylphenylcarbamoyl)-2-tert-butoxycarbonyl-1,2,3,4-tetrahydroisoquinolin-7-yl]propionate

10% Palladium carbon (70 mg) was suspended in methanol (14 mL), the compound (700 mg) of (2) was added thereto, and the mixture was subjected to catalytic hydrogenation at 20°C for 17.5 hr under 2 kgf/cm². After palladium carbon was removed by filtration, methanol was evaporated under reduced pressure.

The obtained residue and the compound (300 mg) of Reference Example 15 were dissolved in methylene chloride (10 mL), 1-ethyl-3-(3-dimethylaminopropyl)carbodiimide hydrochloride (357 mg) was added under ice-cooling, and the mixture was stirred at the same temperature for 30 min. Methylene chloride was evaporated under reduced pressure, 2 M hydrochloric acid was added to the residue, and the mixture was extracted with ethyl acetate (30 mL). The ethyl acetate layer was washed successively with 2 M hydrochloric acid, saturated aqueous sodium hydrogen carbonate and saturated brine, and dried over sodium sulfate. Then, ethyl acetate was evaporated under reduced pressure. The residue was purified by column chromatography to give the title compound as a powder (728 mg).
¹H-NMR (CDCl₃) δ (ppm); 1.52 (9H, br-s), 1.70-2.10 (9H, m), 2.30 (3H, s), 2.61 (2H, t, J=7.8 Hz), 2.92 (2H, t, J=7.8 Hz), 3.00-3.20 (1H, m), 3.25-3.45 (1H, m), 3.66 (3H, s), 4.35-5.10 (3H, m), 6.95-7.20 (4H, m), 7.20-7.45, 7.80-7.95 (1H, br, br).

### (4) methyl 3-[(3S)-(4-acetoxy-2,3,5-trimethylphenyl)carbamoyl-1,2,3,4-tetrahydroisoquinolin-7-yl]propionate

According to Example 27 (3), the title compound was obtained as a powder (510 mg) from the compound (721 mg) of (3).
¹H-NMR (CDCl₃) δ (ppm); 1.68 (1H, br-s), 2.08 (3H, s), 2.12 (6H, s), 2.33 (3H, s), 2.62 (2H, t, J=7.8 Hz), 2.80-2.95 (1H, m), 2.92 (2H, t, J=7.8 Hz), 3.30 (1H, dd, J=16.4, 5.6 Hz), 3.67 (3H, s), 3.72 (1H, dd, J=9.8, 5.6Hz), 3.99, 4.05 (2H, AB-q, J=16.2 Hz), 6.93 (1H, s), 7.04 (1H, d, J=7.9 Hz), 7.13 (1H, d, J=7.9 Hz), 7.66 (1H, s), 9.23 (1H, s).

### (5) methyl 3-[(3S)-(4-acetoxy-2,3,5-trimethylphenyl)carbamoyl-2-(2,2-dimethylbutyryl)-1,2,3,4-tetrahydroisoquinolin-7-yl]propionate

According to Example 30 (3), the title compound was obtained as a powder (290 mg) from the compound (250 mg) of (4) and 2,2-dimethylbutyrylchloride (92 mg).
¹H-NMR (CDCl₃) δ (ppm); 0.83 (3H, t, J=7.5 Hz), 1.31, 1.34 (6H, s, s), 1.76 (2H, q, J=7.5 Hz), 1.99 (3H, br-s), 2.04, 2.07 (6H, s, s), 2.31 (3H, s), 2.61 (2H, t, J=7.8 Hz), 2.92 (2H, t, J=7.8 Hz), 3.03 (1H, dd, J=15.9, 6.8 Hz), 3.47 (1H, dd, J=15.9, 5.1 Hz), 3.66 (3H, s), 4.49, 4.94 (2H, AB-q, J=15.8 Hz), 5.29 (1H, dd, J=6.8, 5.1 Hz), 6.98 (1H, s), 7.08 (1H, d, J=7.8 Hz), 7.17 (1H, d, J=7.8 Hz), 7.42 (1H, s), 7.99 (1H, br-s).

### (6) 3-[2-(2,2-dimethylbutyryl)-(3S)-(4-hydroxy-2,3,5-trimethylphenyl)carbamoyl-1,2,3,4-tetrahydroisoquinolin-7-yl]propionic acid

The compound (285 mg) of (5) was dissolved in a mixed solution of tetrahydrofuran-methanol (3:1, 3 mL), 2 M aqueous lithium hydroxide solution (1.06 mL) was added under ice-cooling, and the mixture was stirred at the same temperature for 30 min, and then at room temperature for 1 hr. After 2 M hydrochloric acid (1.3 mL) was added under ice-cooling, tetrahydrofuran-methanol was evaporated under reduced pressure. Water was added to the residue, and 2 M aqueous lithium hydroxide solution (1.2 mL) was added to dissolve the precipitate under ice-cooling. Then, 2 M hydrochloric acid (1.3 mL) was added at the same temperature, and the mixture was stirred for 30 min. The precipitate was collected by filtration to give the title compound as a powder (216 mg).
IR ν (Nujol) cm⁻¹; 3600-3100, 1715, 1668, 1607, 1202, 1124, 1096.
¹H-NMR (CDCl₃) δ (ppm); 0.83 (3H, t, J=7.3 Hz), 1.32, 1.34 (6H, s, s), 1.77 (2H, q, J=7.3 Hz), 1.82 (3H, s), 1.99, 2.05 (6H, s, s), 2.66 (2H, br-t), 2.90 (2H, br-t), 3.03 (1H, dd, J=16.0, 5.7 Hz), 3.40-3.55 (1H, m), 4.40-5.50 (1H, br), 4.56, 4.91 (2H, AB-q, J=15.7 Hz), 5.15-5.30 (1H, m), 6.75-6.95 (1H, br), 6.99 (1H, s), 7.05-7.20 (2H, m), 7.75 (1H, br-s).

### Example 130: 3-[2-(2,2-dimethylhexanoyl)-(3S)-(4-hydroxy-2,3,5-trimethylphenylcarbamoyl)-1,2,3,4-tetrahydroisoquinolin-7-yl]propionic acid

The title compound was synthesized according to Example 129.
IR ν (Nujol) cm⁻¹; 3600-3100, 1715, 1645, 1607, 1200, 1096.
¹H-NMR (CDCl₃) δ (ppm); 0.79 (3H, t, J=6.7 Hz), 1.10-1.30 (4H, m), 1.33, 1.35 (6H, s, s), 1.60-1.80 (2H, m), 1.82 (3H, br-s), 2.00, 2.06 (6H, s, s), 2.65 (2H, t, J=7.4 Hz), 2.90 (2H, t, J=7.4 Hz), 3.02 (1H, dd, J=15.8, 6.6 Hz), 3.48 (1H, dd, J=15.8, 4.8 Hz), 4.40-5.50 (1H, br), 4.56, 4.92 (2H, AB-q, J=15.7 Hz), 5.25 (1H, dd, J=6.6, 4.8 Hz), 6.87 (1H, br-s), 6.99 (1H, s), 7.08 (1H, d, J=7.8 Hz), 7.15 (1H, d, J=7.8 Hz), 7.75 (1H, br-s).

### Example 131: 2-{[2-(2,2-dimethylbutyryl)-(3S)-(4-hydroxy-2,3,5-trimethylphenyl)carbamoyl-1,2,3,4-tetrahydroisoquinolin-7-yl]oxy}-2-methylpropionic acid

### (1) benzyl 2-tert-butoxycarbonyl-7-(1-ethoxycarbonyl-1-methylethoxy)-1,2,3,4-tetrahydroisoquinoline-(3S)-carboxylate

To toluene (17 mL) were added the compound (835 mg) of Example 129 (1), ethyl 2-bromoisobutylate (638 mg), potassium carbonate (904 mg) and tetraethylammonium fluoride (163 mg), and the mixture was refluxed at 80°C for 35 hr. After allowing to cool, water (10 mL) was added, and the mixture was extracted with ethyl acetate (10 mL). The ethyl acetate layer was washed successively with water and saturated brine, and dried over sodium sulfate. Then, solvent was evaporated under reduced pressure. The residue was purified by column chromatography to give the title compound as a viscous oil (732 mg).
¹H-NMR (CDCl₃) δ (ppm); 1.23, 1.24 (3H, t, t, J=7.0 Hz, 7.2 Hz), 1.40, 1.50 (9H, s, s), 1.56, 1.57 (6H, s, s), 3.00-3.25 (2H, m), 4.22 (2H, q, J=7.1 Hz), 4.41, 4.60 (1H, AB-q, J=16.1 Hz), 4.41, 4.62 (1H, AB-q, J=16.4 Hz), 4.82 (0.45H, t, J=5.1 Hz), 4.90-5.15 (2H, m), 5.10-5.25 (0.55H, m), 6.55-6.70 (2H, m), 6.95 (1H, d, J=8.3 Hz), 7.10-7.20 (2H, m), 7.20-7.35 (2H, m), 7.37 (1H, d, J=4.1 Hz).

### (2) 2-tert-butoxycarbonyl-7-(1-ethoxycarbonyl-1-methylethoxy)-1,2,3,4-tetrahydroisoquinoline-(3S)-carboxylic acid

10% Palladium carbon (70 mg) was suspended in methanol (15 mL), the compound (726 mg) of (1) was added thereto, and the mixture was subjected to catalytic hydrogenation at 25°C for 2 hr under 3 kgf/cm². After palladium carbon was removed by filtration, methanol was evaporated under reduced pressure to give the title compound as a powder (573 mg).
¹H-NMR (CDCl₃) δ (ppm); 1.22 (3H, t, J=7.1 Hz), 1.42, 1.50 (9H, s, s), 1.57 (6H, s), 3.00-3.25 (2H, m), 4.21 (2H, q, J=7.1 Hz), 4.37, 4.59 (1H, AB-q, J=16.1 Hz), 4.37, 4.64 (1H, AB-q, J=16.1 Hz), 4.74 (0.45H, br-t), 5.03 (0.55H, br-t), 6.55-6.70 (2H, m), 7.00 (1H, d, J=7.8 Hz).

### (3) ethyl 2-{[(3S)-(4-acetoxy-2,3,5-trimethylphenyl)carbamoyl-2-tert-butoxycarbonyl-1,2,3,4-tetrahydroisoquinolin-7-yl]oxy}-2-methylpropionate

According to Example 2 (3), the title compound was obtained as a powder (1.15 g) from the compound (957 mg) of (2) and the compound (454 mg) of Reference Example 15.
¹H-NMR (CDCl₃) δ (ppm); 1.23 (3H, t, J=7.1 Hz), 1.51, 1.57 (15H, s, s), 2.00 (3H, s), 2.04, 2.05 (6H, s, s), 2.30 (3H, s), 2.95-3.15 (1H, m), 3.25-3.35 (1H, m), 4.22 (2H, q, J=7.1 Hz), 4.25-5.05 (3H, m), 6.60-6.80 (2H, m), 7.00-7.40 (2.5H, m), 7.70-7.95 (0.5H, br).

### (4) ethyl 2-{[(3S)-(4-acetoxy-2,3,5-trimethylphenyl)carbamoyl-2-(2,2-dimethylbutyryl)-1,2,3,4-tetrahydroisoquinolin-7-yl]oxy}-2-methylpropionate

According to Example 30 (2)-(3), the title compound (561 mg) was obtained from the compound (600 mg) of (3).
IR ν (Nujol) cm⁻¹; 3279, 1757, 1732, 1622, 1501, 1225, 1196, 1142, 1082, 984.
¹H-NMR (CDCl₃) δ (ppm); 0.83 (3H, t, J=7.5 Hz), 1.25 (3H, t, J=7.1 Hz), 1. 30, 1. 33 (6H, s, s), 1. 57 (6H, s), 1. 65-1. 80 (2H, m), 2.01 (3H, br-s), 2.04, 2.07 (6H, s, s), 2.32 (3H, s), 3.00 (1H, dd, J=16.0, 7.0 Hz), 3.45 (1H, dd, J=16.0, 5.2 Hz), 4.22 (2H, q, J=7.1 Hz), 4.43, 4.89 (2H, AB-q, J=15.7 Hz), 5.26 (1H, dd, J=7.0, 5.2 Hz), 6.68 (1H, d, J=2.4 Hz), 6.73 (1H, dd, J=8.3, 2.4 Hz), 7.10 (1H, d, J=8.3 Hz), 7.41 (1H, s), 7.97 (1H, br-s).

### (5) 2-{[2-(2,2-dimethylbutyryl)-(3S)-(4-hydroxy-2,3,5-trimethylphenyl)carbamoyl-1,2,3,4-tetrahydroisoquinolin-7-yl]oxy}-2-methylpropionic acid

The compound (540 mg) of (4) was dissolved in methanol (10.8 mL), 1 M aqueous lithium hydroxide solution (2.8 mL) was added, and the mixture was stirred at 50°C for 2 hr. Water (50 mL) was added, and methanol was evaporated under reduced pressure. The aqueous layer was washed twice with diethyl ether (20 mL), and diethyl ether in the aqueous layer was evaporated under reduced pressure. The residue was acidified with 2 M hydrochloric acid under ice-cooling, and the mixture was stirred at the same temperature for 30 min stirring. Then, the precipitate was collected by filtration to give the title compound as a powder (340 mg).
IR ν (Nujol) cm⁻¹; 3304, 1734, 1668, 1612, 1501, 1246, 1151, 986.
¹H-NMR (CDCl₃) δ (ppm); 0.85 (3H, t, J=7.4 Hz), 1.31, 1.33 (6H, s, s), 1.57 (6H, s), 1.65-1.80 (2H, m), 1.89 (3H, br-s), 2.05, 2.08 (6H, s, s), 3.01 (1H, dd, J=15.6, 6.2 Hz), 3.43 (1H, dd, J=15.6, 5.4 Hz), 4.49, 4.89 (2H, AB-q, J=15.8 Hz), 5.26 (1H, dd, J=6.2, 5.4 Hz), 6.74 (1H, d, J=2.4 Hz), 6.78 (1H, dd, J=8.3, 2.4 Hz), 6.88 (1H, br-s), 7.07 (1H, d, J=8.3 Hz), 7.80 (1H, br-s).

### Example 132: 2-{[2-(2,2-dimethylnonyl)-(3S)-(4-hydroxy-2,3,5-trimethylphenyl)-1,2,3,4-tetrahydroisoquinolin-7-yl]oxy}-2-methylpropionic acid

The title compound was synthesized according to Example 131.
IR ν (Nujol) cm⁻¹; 3304, 1734, 1668, 1612, 1246, 1151.
¹H-NMR (CDCl₃) δ (ppm); 0.83 (3H, t, J=7.2 Hz), 1.10-1.25 (10H, m), 1.32, 1.33 (6H, s, s), 1.57 (6H, s), 1.60-1.75 (2H, m), 1.88 (3H, br-s), 2.06, 2.09 (6H, s, s), 3.00 (1H, dd, J=15.8, 6.7 Hz), 3.35-3.50 (1H, m), 4.50, 4.90 (2H, AB-q, J=16.0 Hz), 5.26 (1H, br-t), 6.74 (1H, s), 6.78 (1H, d, J=8.3 Hz), 6.90 (1H, br-s), 7.09 (1H, d, J=8.3 Hz), 7.77 (1H, br-s).

### Example 133: 7-diethylaminomethyl-2-(2,2-dimethylpropionyl)-N-(4-hydroxy-2,3,5-trimethylphenyl)-1,2,3,4-tetrahydroisoquinoline-(3S)-carboxamide

### (1) methyl 2-tert-butoxycarbonyl-7-formyl-1,2,3,4-tetrahydroisoquinoline-(3S)-carboxylate

Under nitrogen atmosphere, the compound (3.50 g) of Example 121 (1) was dissolved in dehydrated N,N-dimethylformamide (9 mL), bis(triphenylphosphine)palladium dichloride (442 mg), triphenylphosphine (165 mg) and lithium formate (622 mg) were added, and the mixture was stirred at 100°C for 3 hr while aerating with carbon monoxide. After allowing to cool, saturated aqueous sodium hydrogen carbonate was added, and the mixture was extracted with diethyl ether (70 mL). The diethyl ether layer was washed successively with saturated aqueous sodium hydrogen carbonate and saturated brine, and dried over sodium sulfate. Then, diethyl ether was evaporated under reduced pressure. The residue was purified by column chromatography to give the title compound as a viscous oil (673 mg).
¹H-NMR (CDCl₃) δ (ppm); 1.47, 1.53, 1.59 (9H, s, s, s), 3.15-3.30 (1.5H, m), 3.35 (0.5H, dd, J=16.7, 2.0 Hz), 3.62, 3.64 (3H, s, s), 4.55, 4.79 (1H, AB-q, J=16.3 Hz), 4.59, 4.81 (1H, AB-q, J=16.5 Hz), 4.87 (0.5H, br-t), 5.20 (0.5H, dd, J=6.4, 2.0 Hz), 7.32 (1H, d, J=7.8 Hz), 7.60-7.80 (2H, m), 9.96 (1H, s).

### (2) methyl 2-tert-butoxycarbonyl-7-hydroxymethyl-1,2,3,4-tetrahydroisoquinoline-(3S)-carboxylate

The compound (518 mg) of (1) was dissolved in diethyl ether (10 mL), tert-butylamine-borane complex (169 mg) was added under ice-cooling, and the mixture was stirred at room temperature for 2 hr. After 1 M hydrochloric acid was added, the mixture was extracted with ethyl acetate (20 mL). The ethyl acetate layers were combined, washed successively with 1 M hydrochloric acid and saturated brine, and dried over sodium sulfate. Then, ethyl acetate was evaporated under reduced pressure. The residue was purified by column chromatography to give the title compound as a viscous oil (435 mg).
¹H-NMR (CDCl₃) δ (ppm); 1.46, 1.52 (9H, s, s), 1.60-2.00 (1H, br), 3.05-3.35 (2H, m), 3.61, 3.63 (3H, s, s), 4.48, 4.71 (1H, AB-q, J=16.6 Hz), 4.52, 4.73 (1H, AB-q, J=16.6 Hz), 4.65 (2H, s), 4.78 (0.55H, t, J=5.2 Hz), 5.14 (0.45H, dd, J=5.9, 2.9 Hz), 7.05-7.40 (3H, m).

### (3) 2-tert-butoxycarbonyl-7-hydroxymethyl-1,2,3,4-tetrahydroisoquinoline-(3S)-carboxylic acid

According to Example 2 (2), the title compound was obtained as a powder (273 mg) from the compound (609 mg) of (2).
IR ν (Nujol) cm⁻¹; 3700-3100, 1728, 1682, 1325, 1167, 1096, 1055, 907, 854, 822.
¹H-NMR (DMSO-d₆) δ (ppm); 1.39, 1.46 (9H, s, s), 2.95-3.20 (2H, m), 4.36, 4.50 (1H, AB-q, J=16.5 Hz), 4.42, 4.58 (1H, AB-q, J=16.4 Hz), 4.43 (2H, d, J=5.8 Hz), 4.55-4.70 (0.55H, m), 4.80-4.90 (0.45H, m), 5.05-5.25 (1H, m), 7.05-7.25 (3H, m), 12.25-13.00 (1H, br).

### (4) N-(4-acetoxy-2,3,5-trimethylphenyl)-2-tert-butoxycarbonyl-7-hydroxymethyl-1,2,3,4-tetrahydroisoquinoline-(3S)-carboxamide

According to Example 2 (3), the title compound was obtained as a powder (636 mg) from the compound (437 mg) of (3) and the compound (274 mg) of Reference Example 15.
¹H-NMR (CDCl₃) δ (ppm); 1.52 (9H, br-s), 2.00 (3H, br-s), 2.04 (6H, s), 2.30 (3H, s), 3.00-3.30 (1H, m), 3.30-3.60 (1H, m), 4.35-5.20 (3H, m), 4.67 (2H, s), 7.00-7.65 (4H, m), 7.70-8.10 (1H, br).

### (5) N-(4-acetoxy-2,3,5-trimethylphenyl)-7-diethylaminomethyl-1,2,3,4-tetrahydroisoquinoline-(3S)-carboxamide

The compound (810 mg) of (4) was dissolved in methylene chloride (8 mL), triethylamine (0.35 mL) and methanesulfonyl chloride (0.16 mL) were added under ice-cooling, and the mixture was stirred at the same temperature for 20 min. Methylene chloride was evaporated under reduced pressure, saturated aqueous sodium hydrogen carbonate was added, and the mixture was extracted with ethyl acetate (30 mL). The ethyl acetate layer was washed successively with 5% aqueous citric acid solution and saturated brine, and dried over sodium sulfate. Then, ethyl acetate was evaporated under reduced pressure.

The obtained residue (0.97 g) was dissolved in tetrahydrofuran (10 mL), diethylamine (0.70 mL) was added, and the mixture was stirred at 45°C for 4.5 hr. Tetrahydrofuran was evaporated under reduced pressure, saturated aqueous sodium hydrogen carbonate was added, and the mixture was twice extracted with ethyl acetate (20 mL). The ethyl acetate layers were combined, washed successively with saturated aqueous sodium hydrogen carbonate and saturated brine, and dried over sodium sulfate. Then, ethyl acetate was evaporated under reduced pressure. The residue was purified by column chromatography.

According to Example 27 (3), the title compound was obtained as a powder (395 mg) from the obtained residue (823 mg).
¹H-NMR (CDCl₃) δ (ppm); 1.05 (6H, t, J=7.2 Hz), 1.45-2.00 (1H, br), 2.08 (3H, s), 2.13 (6H, s), 2.34 (3H, s), 2.53 (4H, q, J=7.2 Hz), 2.92 (1H, dd, J=16.3, 9.8 Hz), 3.32 (1H, dd, J=16.3, 5.6 Hz), 3.53 (2H, s), 3.74 (1H, dd, J=9.8, 5.6 Hz), 4.02, 4.08 (2H, AB-q, J=15.7 Hz), 7.09 (1H, s), 7.10-7.20 (2H, m), 7.67 (1H, s), 9.26 (1H, br-s).

### (6) N-(4-acetoxy-2,3,5-trimethylphenyl)-7-diethylaminomethyl-2-(2,2-dimethylpropionyl)-1,2,3,4-tetrahydroisoquinoline-(3S)-carboxamide

According to Example 30 (3), the title compound wa obtained as a powder (384 mg) from the compound (387 mg) of (5) and pivaloyl chloride (0.12 mL).
IR ν (Nujol) cm⁻¹; 3219, 1751, 1666, 1622, 1537, 1229, 1204, 1082, 993.
¹H-NMR (CDCl₃) δ (ppm); 1.03 (6H, t, J=7.2 Hz), 1.37 (9H, s), 1.98 (3H, br-s), 2.03, 2.07 (6H, s, s), 2.32 (3H, s), 2.51 (4H, q, J=7.2 Hz), 3.06 (1H, dd, J=16.0, 7.0 Hz), 3.50 (1H, dd, J=16.0, 4.9 Hz), 3.53 (2H, s), 4.53, 4.95 (2H, AB-q, J=15.7 Hz), 5.27 (1H, dd, J=7.0, 4.9 Hz), 7.15 (1H, s), 7.15-7.25 (2H, m), 7.41 (1H, br-s), 7.95 (1H, br-s).

### (7) 7-diethylaminomethyl-2-(2,2-dimethylpropionyl)-N-(4-hydroxy-2,3,5-trimethylphenyl)-1,2,3,4-tetrahydroisoquinoline-(3S)-carboxamide

The compound (428 mg) of (6) was dissolved in a mixed solution of tetrahydrofuran-methanol (3:1, 7.5 mL), 1 M aqueous lithium hydroxide solution (2.5 mL) was added dropwise over 30 min under ice-cooling, and the mixture was stirred at the same temperature for 1.5 hr. After 2 M hydrochloric acid (1.25 mL) was added, tetrahydrofuran-methanol was evaporated under reduced pressure. Saturated aqueous sodium hydrogen carbonate was added, and the mixture was twice extracted with ethyl acetate (20 mL). The ethyl acetate layers were combined, washed successively with saturated aqueous sodium hydrogen carbonate and saturated brine, and dried over sodium sulfate. Then, ethyl acetate was evaporated under reduced pressure. The residue (0.42 g) was dissolved in 2 M hydrochloric acid (5 mL), pH of the solution was adjusted to about 8 with sodium bicarbonate, and the precipitate was collected by filtration to give the title compound as a powder (248 mg).
IR ν (Nujol) cm⁻¹; 3288, 1666, 1614, 1204, 1175, 1097.
¹H-NMR (CDCl₃) δ (ppm); 1.05 (6H, t, J=7.1 Hz), 1.38 (9H, s), 1.90 (3H, br-s), 2.10, 2.13 (6H, s, s), 2.51 (4H, q, J=7.1 Hz), 3.07 (1H, dd, J=15.9, 6.6 Hz), 3.50 (1H, dd, J=15.9, 4.9 Hz), 3.53 (2H, s), 4.56, 4.95 (2H, AB-q, J=15.6 Hz), 4.81 (1H, br-s), 5.24 (1H, dd, J=6.6, 4.9 Hz), 6.98 (1H, br-s), 7.10-7.30 (2H, m), 7.15 (1H, m), 7.70 (1H, br-s).

The compounds of Example 134-Example 145 were synthesized according to Example 133.

### Example 134: 2-(2,2-dimethylpropionyl)-N-(4-hydroxy-2,3,5-trimethylphenyl)-7-(pyrrolidin-1-yl)methyl-1,2,3,4-tetrahydroisoquinoline-(3S)-carboxamide hydrochloride

IR ν (Nujol) cm⁻¹; 1674, 1616, 1410, 1242, 1097.
¹H-NMR (DMSO-d₆) δ (ppm); 1.29 (9H, s), 1.70-1.95 (4H, m), 2.00 (3H, br-s), 2.04, 2.06 (6H, s, s), 2.95-3.10 (2H, m), 3.15-3.45 (4H, m), 4.28 (2H, br-s), 4.60, 4.91 (2H, AB-q, J=16.7 Hz), 4.95-5.20 (1H, m), 6.57 (1H, br-s), 7.30 (1H, d, J=7.1 Hz), 7.35-7.55 (2H, m), 7.99 (1H, br-s), 9.18 (1H, br-s), 10.20-11.00 (1H, br).

### Example 135: 7-diisopropylaminomethyl-N-(4-hydroxy-2,3,5-trimethylphenyl)-2-isobutyryl-1,2,3,4-tetrahydroisoquinoline-(3S)-carboxamide

IR ν (Nujol) cm⁻¹; 3600-3100, 1661, 1651, 1645, 1520, 1229, 1209, 1180, 1094.
¹H-NMR (CDCl₃) δ (ppm); 1.01 (12H, d, J=6.1 Hz), 1.17 (1.8H, d, J=6.8 Hz), 1.23, 1.24 (4.2H, d, d, J=6.6 Hz, 6.8 Hz), 1.48 (0.9H, s), 1.93 (2.1H, s), 2.01, 2.07 (1.8H, s, s), 2.10, 2.13 (4.2H, s, s), 2.84 (0.3H, septet, J=6.8 Hz), 2.85-3.20 (3.4H, m), 3.16 (0.3H, dd, J=15.0, 5.0 Hz), 3.40-3.60 (1H, m), 3.61 (2H, s), 4.49, 4.99 (0.6H, AB-q, J=16.4 Hz), 4.54, 4.73 (1.4H, AB-q, J=15.4 Hz), 4.60-5.10 (1H, br), 4.84 (0.3H, br-t), 5.28 (0.7H, br-t), 6.49 (0.3H, s), 6.98 (0.3H, br-s), 7.06 (0.7H, s), 7.10-7.35 (3H, m), 7.89 (0.7H, br-s).

### Example 136: 2-(2,2-dimethylpropionyl)-N-(4-hydroxy-2,3,5-trimethylphenyl)-7-(N-methylpropylamino)methyl-1,2,3,4-tetrahydroisoquinoline-(3S)-carboxamide

IR ν (Nujol) cm⁻¹; 3238, 1680, 1601, 1229, 1208, 1178, 1099, 991.
¹H-NMR (CDCl₃) δ (ppm); 0.88 (3H, t, J=7.5 Hz), 1.38 (9H, br-s), 1.52 (2H, sextet, J=7.5 Hz), 1.90 (3H, br-s), 2.09, 2.10, 2.12, 2.13 (6H, s, s, s, s), 2.18 (3H, s), 2.31 (2H, t, J=7.5 Hz), 3.07 (1H, dd, J=15.8, 6.8 Hz), 3.45 (2H, s), 3.51 (1H, dd, J=15.8, 5.1 Hz), 4.56, 4.95 (2H, AB-q, J=15.9 Hz), 4.70-5.00 (1H, br), 5.35 (1H, dd, J=6.8, 5.1 Hz), 6.95-7.05 (1H, br), 7.13 (1H, s), 7.15-7.25 (2H, m), 7.69 (1H, br-s).

### Example 137: 2-(2,2-dimethylpropionyl)-N-(4-hydroxy-2,3,5-trimethylphenyl)-7-propylaminomethyl-1,2,3,4-tetrahydroisoquinoline-(3S)-carboxamide

IR ν (Nujol) cm⁻¹; 3238, 1678, 1599, 1229, 1205, 1177, 1097, 988.
¹H-NMR (CDCl₃) δ (ppm); 0.91 (3H, t, J=7.3 Hz), 1.38 (9H, br-s), 1.54 (2H, sextet, J=7.3 Hz), 1.89 (3H, br-s), 2.08, 2.11 (6H, s, s), 2.59 (2H, t, J=7.3 Hz), 3.06 (1H, dd, J=15.9, 6.6 Hz), 3.48 (1H, dd, J=15.9, 5.1 Hz), 3.77 (2H, s), 4.54, 4.94 (2H, AB-q, J=15.9 Hz), 5.24 (1H, dd, J=6.6, 5.1 Hz), 6.96 (1H, br-s), 7.14 (1H, s), 7.15-7.25 (2H, m), 7.70 (1H, br-s).

### Example 138: N-(4-hydroxy-2,3,5-trimethylphenyl)-2-isobutyryl-7-(N-methylpropylamino)methyl-1,2,3,4-tetrahydroisoquinoline-(3S)-carboxamide

IR ν (Nujol) cm⁻¹; 3254, 1645, 1520, 1229, 1092, 858.
¹H-NMR (CDCl₃) δ (ppm); 0.89 (3H, br-t), 1.17, 1.24 (6H, d, d, J=6.3 Hz, 6.1 Hz), 1.45-1.60 (2H, m), 1.85-2.25 (11H, m), 2.32 (2H, t, J=7.5 Hz), 2.75-3.20 (2H, m), 3.40-3.55 (1H, m), 3.45 (2H, s), 4.54 (1H, d, J=14.9 Hz), 4.65-5.20 (2.3H, m), 5.30 (0.7H, br-t), 6.46 (0.3H, br-d), 6.95-7.25 (4H, m), 7.88 (0.7H, br-s).

### Example 139: 2-(2,2-dimethylpropionyl)-7-(2-fluoro-N-methylethylamino)methyl-N-(4-hydroxy-2,3,5-trimethylphenyl)-1,2,3,4-tetrahydroisoquinoline-(3S)-carboxamide

IR ν (Nujol) cm⁻¹; 3250, 1666, 1609, 1541.
¹H-NMR (CDCl₃) δ (ppm); 1.38 (9H, s), 1.90 (3H, s), 2.08 (3H, s), 2.11 (3H, s), 2.30 (3H, s), 2.71 (2H, dt, J=27.3, 4.9 Hz), 2.71 (1H, dd, J=15.8, 6.6 Hz), 3.51 (1H, dd, J=15.8, 5.1 Hz), 3.56 (2H, s), 4.54 (2H, dt, J=47.6, 4.9 Hz), 4.56, 4.96 (2H, AB-q, J=15.9 Hz), 4.80-5.10 (1H, br), 5.23-5.28 (1H, m), 6.95 (1H, s), 7.13-7.20 (3H, m), 7.70 (1H, s).

### Example 140: 2-(2,2-dimethylpropionyl)-7-(2-fluoroethylamino)methyl-N-(4-hydroxy-2,3,5-trimethylphenyl)-1,2,3,4-tetrahydroisoquinoline-(3S)-carboxamide hydrochloride IR ν (Nujol) cm⁻¹; 3280, 1664, 1618, 1508.

¹H-NMR (DMSO-d₆) δ (ppm); 1.29 (9H, s), 1.81 (3H, s), 2.05 (3H, s), 2.07 (3H, s), 3.15-3.40 (4H, m), 4.14 (2H, s), 4.60, 4.91 (2H, AB-q, J=16.6 Hz), 4.76 (2H, dt, J=47.1, 4.2 Hz), 5.00-5.20 (1H, m), 6.59 (1H, s), 7.30 (1H, d, J=7.6 Hz), 7.30 (1H, d, J=7.6 Hz), 7.43 (1H, s), 8.00 (1H, s), 9.21 (1H, s), 9.53 (2H, s).

### Example 141: 2-(2,2-dimethylpropionyl)-7-(3-fluoroazetidin-1-yl)methyl-N-(4-hydroxy-2,3,5-trimethylphenyl)-1,2,3,4-tetrahydroisoquinoline-(3S)-carboxamide hydrochloride

IR ν (Nujol) cm⁻¹; 1668, 1616, 1242, 1198.
¹H-NMR (DMSO-d₆) δ (ppm); 1.29 (9H, br-s), 1.79 (3H, br-s), 2.06, 2.04 (6H, s, s), 3.10-3.40 (2H, m), 4.10-4.50 (6H, m), 4.59, 4.91 (2H, AB-q, J=16.7 Hz), 4.90-5.20 (1H, m), 5.20-5.55 (1H, br-q), 6.58 (1H, br-s), 7.15-7.50 (3H, m), 8.00 (1H, br-s), 9.20 (1H, br-s), 10.8-11.7 (1H, br).

### Example 142: 2-(2,2-dimethylpropionyl)-7-(3-hydroxyazetidin-1-yl)methyl-N-(4-hydroxy-2,3,5-trimethylphenyl)-1,2,3,4-tetrahydroisoquinoline-(3S)-carboxamide

IR ν (Nujol) cm⁻¹; 1668, 1614, 1178, 1084.
¹H-NMR (CDCl₃) δ (ppm); 1.37 (9H, br-s), 1.86 (3H, br-s), 2.00-2.20 (6H, m), 2.93 (2H, br-t), 3.05 (1H, dd, J=15.6, 6.4 Hz), 3.40-3.70 (5H, m), 4.30-4.55 (1H, m), 4.53 (1H, d, J=15.4 Hz), 4.91, 4.94 (1H, d, d, J=15.8Hz, 15.4 Hz), 5.10-5.30 (1H, m), 6.80-6.95 (1H, m), 6.95-7.20 (3H, m), 7.71 (1H, br-s).

### Example 143: 2-(2,2-dimethylpropionyl)-N-(4-hydroxy-2,3,5-trimethylphenyl)-7-(3-methoxyazetidin-1-yl)methyl-1,2,3,4-tetrahydroisoquinoline-(3S)-carboxamide hydrochloride

IR ν (Nujol) cm⁻¹; 1749, 1653, 1606, 1196, 1080, 989.
¹H-NMR (DMSO-d₆) δ (ppm); 1.28 (9H, br-s), 1.79 (3H, br-s), 2.04, 2.06 (6H, s, s), 3.10-3.30 (5H, m), 3.80-4.00 (2H, br), 4.10-4.40 (5H, m), 4.58, 4.91 (2H, AB-q, J=16.6 Hz), 4.95-5.10 (1H, m), 6.58 (1H, br-s), 7.20-7.45 (3H, m), 8.00 (1H, br-s), 9.20 (1H, br-s), 10.7-11.3 (1H, br).

### Example 144: 2-(2,2-dimethylpropionyl)-7-(N-ethyl-2-methoxyethylamino)methyl-N-(4-hydroxy-2,3,5-trimethylphenyl)-1,2,3,4-tetrahydroisoquinoline-(3S)-carboxamide hydrochloride

IR ν (Nujol) cm⁻¹; 3300-3050, 1668, 1614, 1242, 1200, 1121.
¹H-NMR (CDCl₃) δ (ppm); 1.35-1.45 (3H, m), 1.37 (9H, s), 1.97 (3H, br-s), 2.09, 2.10, 2.12, 2.13 (6H, s, s, s, s), 3.07 (1H, dd, J=16.1, 6.8 Hz), 3.10-3.25 (4H,m), 3.38, 3.39 (3H, s, s), 3.50 (1H, br-d), 3.85-4.00 (2H,m),4.10-4.35 (2H, m), 4.50 (1H, br-d), 5.00-5.20 (2H, m), 5.32 (1H, br-t), 7.01 (1H, br-s), 7.25-7.35 (1H, m), 7.39 (1H, d, J=7.8 Hz), 7.71 (1H, br-s), 7.89 (1H, br-d), 12.20-12.50 (1H, br).

### Example 145: 2-(2,2-dimethylpropionyl)-7-(N-ethylpropylamino)methyl-N-(4-hydroxy-2,3,5-trimethylphenyl)-1,2,3,4-tetrahydroisoquinoline-(3S)-carboxamide hydrochloride

IR ν (Nujol) cm⁻¹; 3300-3050, 1668, 1614, 1242, 1178, 1096.
¹H-NMR (CDCl₃) δ (ppm); 0.95 (3H, br-t), 1.30-1.45 (12H, m), 1.80-2.10 (2H, m), 1.96 (3H, s), 2.09, 2.12 (6H, s, s), 2.70-3.15 (4H,m),3.08 (1H, dd, J=16.2, 6.7 Hz), 3.51 (1H, br-dd), 4.00-4.15 (2H, m), 4.49 (1H, br-d), 5.05 (1H, br-d), 5.10-5.60 (2H, m), 6.90-7.05 (1H, m), 7.25-7.40 (2H, m), 7.68 (1H, br-s), 7.92 (1H, br-s), 12.00-12.30 (1H, br).

To clarify the superior properties of the compound of the present invention, the ACAT inhibitory activity, THP-1 cell-derived macrophage foam cell formation-inhibitory activity, mouse liver lipid secretion-inhibitory activity, plasma concentration by oral administration, stable radical-inhibitory activity and lipid peroxidation-inhibitory activity were measured in the following manner.

### Experimental Example 1: ACAT inhibitory activity

Male Japanese white rabbits (body weight 2-2.5 kg) were bred on 100 g of a high cholesterol feed (feed added with cholesterol 1%, CLEA Japan, Inc.) daily for 4 weeks and sacrificed by exsanguination under anesthesia. The liver was removed, homogenated and centrifuged at 10,000 rpm and 4°C for 15 min. The obtained supernatant was further centrifuged at 41,000 rpm and 4°C for 60 min to give a microsome fraction. To a 0.15 M phosphate buffer were added the microsome suspension as an enzyme specimen, dimethyl sulfoxide (DMSO) or a solution (3 µL) of a test compound in DMSO and [1-14C]-oleoyl-CoA to be a reaction substrate to the total amount of 300 µL. After incubation at 37°C for 20 min, a mixed solution of chloroform-methanol was added to quench the reaction. Water was added thereto and mixed, and the chloroform layer was separated. The solvent was evaporated to dryness and the residue was redissolved in n-hexane. The resulting solution was subjected to thin layer chromatography using a silica gel plate. The spot of cholesteryl oleate on the silica gel plate was scraped and quantified on a liquid scintillation counter. The ACAT inhibitory activity of the test compound is shown in cholesteryl oleate production inhibitory rate (%) [level of inhibition of cholesteryl oleate production as compared to the control]. The results are shown in Table 1.

**Table 1**

| test compound | liver ACAT inhibition rate (%) (concentration: 10⁻⁶ M) |
|---|---|
| Example 3 | 93.3 |
| Example 9 | 88.6 |
| Example 26 | 83.8 |
| Example 49 | 96.8 |
| Example 72 | 91.4 |
| Example 73 | 97.7 |
| Example 75 | 88.8 |
| Example 76 | 76.7 |
| Example 80 | 93.4 |
| Example 83 | 97.0 |
| Example 104 | 80.6 |
| Example 106 | 89.6 |
| Example 114 | 87.5 |
| Example 116 | 97.2 |

From the results, it is appreciated that the compound of the present invention has a superior ACAT inhibitory activity. **Experimental Example 2:** cholesterol ester accumulation-inhibitory activity (THP-1 cell-derived macrophage foam cell formation suppressive action)

THP-1 cells (DAINIPPON PHARMACEUTICAL CO., LTD.) were subcultured in RPMI-1640 medium containing 10% fetal calf serum (FBS), and cells at 6-13th passage after purchase were used. The cells were suspended in FBS-containing RPMI-1640 medium to give a suspension having a concentration of 4×10⁵ cells/mL. The cell suspension was seeded on 12-well microplate by 1 mL, and treated with phorbol 12-myristate 13-acetate (PMA, 200 nM) as a differentiation inducer into macrophage. Then, acetyl LDL (400 µg/mL) separately prepared from LDL derived from plasma of a rabbit with genetic hyperlipidemia (KHC rabbit, Japan Laboratory Animals Inc.) was added. A test compound further dissolved in dimethyl sulfoxide (DMSO) and diluted with FBS-containing RPMI-1640 medium and a control solvent were applied. After cultivation in a carbon dioxide incubator for 3 days, the cells were washed with phosphate-buffered saline (pH 7.0), and the lipid was extracted with hexane-isopropanol (3:2). In addition, the cells were lysed with 1N-NaOH, and the protein content was measured. Free cholesterol and cholesterol ester in the lipid extraction sample were measured according to the method of Kunitomo et al. [Japan. J. Pharmacol., 34, 153-158 (1984)]. Cholesterol esters in the control cells and the cells treated with the test compound were compared, and the cholesterol ester accumulation inhibitory rate of the test compound was calculated. The results are shown in Table 2.

**Table 2**

| test compound | cholesteryl ester accumulation inhibitory rate (%) (concentration: 10⁻⁶ M) | test compound | cholesteryl ester accumulation inhibitory rate (concentration: 10⁻⁶ M) |
|---|---|---|---|
| Example 19 | 66.4 | Example 95 | 80.4 |
| Example 69 | 99.4 | Example 104 | 77.3 |
| Example 71 | 79.2 | Example 106 | 76.9 |
| Example 72 | 80.5 | Example 118 | 68.6 |
| Example 73 | 91.8 | Example 119 | 84.5 |
| Example 74 | 74.7 | Example 121 | 86.8 |
| Example 75 | 93.4 | Example 136 | 66.4 |
| Example 80 | 94.5 | Example 139 | 84.6 |
| Example 83 | 100.2 | Example 141 | 88.0 |
| Example 91 | 71.1 | Example 143 | 60.9 |
| Example 93 | 83.4 | | |

From the results, it is appreciated that the compound of the present invention has a superior cholesterol ester accumulation-inhibitory activity.

### Experimental Example 3: mouse liver lipid secretion-inhibitory activity (Triton WR-1339 method)

About 5-week-old male Slc: ICR mice (Japanese SLC) were preliminarily bred for 1 week by feeding only in the daytime (9:00-18:00). During this period, the mice were allowed to freely take tap water even during night. The mice were divided into a control group and a test compound group (6 mice per group) so that the average and standard deviation of the body weight would be almost the same. Blood (about 80 µL) was drawn from the orbital venous plexus under anesthesia using a glass capillary, and a test compound solution (10 mL/kg) obtained by previously suspending the compound in 5% gum arabic solution at 30 min after the blood sampling was orally administered. At 30 min after the administration, Triton WR-1339 (60 mg/mL) solution previously prepared with physiological saline was administered from the tail vein at a dose of 5 mL/kg. Blood was drawn again from the orbital venous plexus at 3 hr after the administration of Triton WR-1339. Plasma was separated from the blood taken and the total plasma cholesterol (TC) was measured using a commercially available measurement kit (Wako Pure Chemical Industries, Ltd.). The changes in the blood concentration over 3 hr after the Triton WR-1339 administration were calculated and taken as the cholesterol secretion rate from the liver. The secretion rates were compared between the control group and the test compound group, and the cholesterol secretion inhibitory rate by the test compound was calculated. The results are shown in Table 3.

**Table 3**

| test compound | Cholesterol secretion inhibitory rate (%) (10 mg)/kg/3 hr |
|---|---|
| Example 69 | 31.4 |
| Example 70 | 23.0 |
| Example 73 | 32.9 |

From the results, it is appreciated that the compound of the present invention has a superior cholesterol secretion-inhibitory activity.

### Experimental Example 4: oral administration experiment

The test compound (10 mg)/kg suspended in 5% gum arabic solution was forcedly administered orally to male SD rats (body weight 200 - 250 g). At 0.5, 1, 2, 4, 6 hr after administration, blood was drawn without anesthesia, and heparin plasma was separated by a conventional method. The concentration of the test compound in the plasma was measured by high performance liquid chromatography. The results are shown in Table 4.

**Table 4**

| test compound | maximum blood concentration (µg/mL) |
|---|---|
| Example 72 | 0.91 |
| Example 73 | 0.55 |
| Example 122 | 0.60 |
| Example 133 | 0.48 |
| Example 138 | 0.42 |
| Example 140 | 0.44 |
| Example 141 | 0.49 |
| Example 143 | 0.46 |

From the results, it is appreciated that the compound of the present invention has superior oral absorbability. **Experimental Example 5:** stable radical elimination action

0.1 mM Diphenyl-2-picrylhydrazyl (DPPH) acetonitrile (CH₃CN) solution (3 mL) was placed in a glass test tube, and a DMSO solution of a test compound was added at a 1% dose (30 µL). As a control, 0.1 mM DPPH CH₃CN solution (3 mL) containing DMSO (30 µL) was used and for a blank test, CH₃CN (3 mL) containing DMSO (30 µL) was used. After standing the solutions at room temperature for 60 min (20 min for Examples 69, 72 and 73), the absorbance at 517 nm was measured by a spectrophotometer. A stable radical inhibitory rate when control was 100% was calculated from [(absorbance of test substance - absorbance of blank test)÷(absorbance of control - absorbance of blank test)].

**Table 5**

| test compound | inhibitory rate (%) (10⁻⁴M) | test compound | inhibitory rate (%) (10⁻⁴M) |
|---|---|---|---|
| Example 47 | 66.1 | Example 122 | 72.3 |
| Example 48 | 91.7 | Example 132 | 80.7 |
| Example 51 | 63.8 | Example 133 | 65.5 |
| Example 69 | 97.0 | Example 136 | 71.3 |
| Example 72 | 97.0 | Example 141 | 63.7 |
| Example 73 | 97.0 | Example 143 | 61.0 |
| Example 84 | 71.9 | | |

From the results, it is appreciated that the compound of the present invention has a superior stable radical-inhibitory activity.

### Experimental Example 6: lipid peroxidation-inhibitory action

The brain was removed from male SD rat, and a 50 mM phosphate buffer (pH 7.4, 5-fold amount of brain weight) was added. The mixture was homogenized under ice-cooling for 30 sec, and centrifuged at 4°C, 3200 rpm for 10 min. The supernatant was diluted 5-fold with 50 mM phosphate buffer to give a brain homogenate. A test compound (10 µL) was placed in a glass test tube, the brain homogenate (1 mL) was added, and the mixture was incubated at 37°C for 30 min. 20% Trichloroacetic acid (1 mL) was added to quench the reaction. The reaction mixture was centrifuged at room temperature, 4000 rpm for 10 min. Thiobarbituric acid (0.2 mL) was added to the supernatant (1 mL) and the mixture was heated at 95°C for 15 min. The absorbance at 532 nm was measured, and lipid peroxide was quantified as malondialdehyde (MDA). The MDA level was compared between the control and the test compound-added sample, and the lipid peroxidation inhibitory rate was calculated.

**Table 6**

| test compound | inhibitory rate (%) (10⁻⁴M) | test compound | inhibitory rate (%) (10⁻⁴M) |
|---|---|---|---|
| Example 13 | 100.1 | Example 122 | 75.9 |
| Example 47 | 100.1 | Example 132 | 105.6 |
| Example 48 | 100.5 | Example 136 | 89.6 |
| Example 50 | 85.7 | Example 139 | 99.7 |
| Example 51 | 100.3 | Example 141 | 94.8 |
| Example 84 | 100.9 | Example 143 | 64.1 |

From the results, it is appreciated that the compound of the present invention has a lipid peroxidation-inhibitory activity.

### Industrial Applicability

The compound of the present invention has an ACAT-inhibitory activity, and effectively and certainly decreases blood cholesterol and accumulation of cholesterol ester in arterial walls. Therefore, it can prevent or treat various vascular pathologies caused thereby and the diseases related thereto. Moreover, since a compound having a 1,2,3,4-tetrahydroisoquinoline skeleton, which is a skeleton common to the compounds, has a superior ACAT-inhibitory activity, it is highly useful for pharmaceutical use (e.g., prophylaxis and/or treatment of hyperlipidemia, arteriosclerosis, inflammatory disease, angina pectoris, myocardial infarction, cerebral infarction, cerebral apoplexy, Alzheimer's disease, renal diseases, ophthalmic diseases and the like).

This application is based on a patent application No. 2005-067596 filed in Japan, the contents of which are incorporated in full herein by this reference.

## Claims

1. A compound represented by the formula (I) 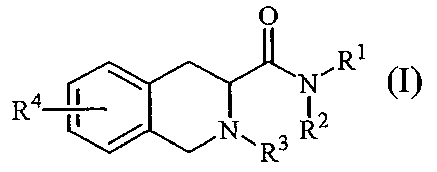 wherein
R¹ and R² are the same or different and each is a hydrogen atom, an alkyl group, a cycloalkyl group, an optionally substituted aryl group, or an optionally substituted heterocycle, or, R¹ and R² form, together with the nitrogen atom bonded thereto, a heterocycle,
R³ is an optionally substituted alkyl group, an optionally substituted carbamoyl group, an optionally substituted alkylcarbonyl group optionally having a double bond, an arylcarbonyl group, an alkylsulfonyl group, an alkylcarbonylcarbonyl group, or an optionally substituted aminosulfonyl group, and
R⁴ is a hydrogen atom, a hydroxyl group, an optionally substituted alkoxy group, an optionally substituted amino group, an optionally substituted heterocycle, an optionally substituted aminoalkyl group, an optionally esterified carboxy group, an optionally esterified carboxyalkyl group, an optionally substituted heterocyclyl-alkyl group, a hydroxyalkyl group, or an alkoxyalkyl group,
or a pharmaceutically acceptable salt thereof.

2. The compound of claim 1, which is represented by the formula (Ia) 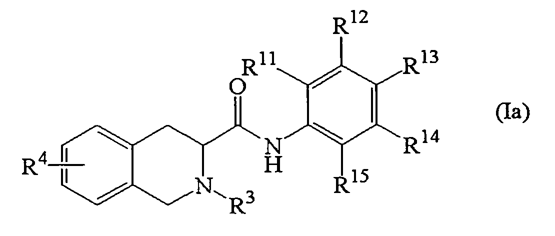 wherein
R¹¹, R¹², R¹³, R¹⁴ and R¹⁵ are each independently a hydrogen atom, a halogen atom, an alkyl group, a hydroxyl group, an optionally substituted amino group, an alkoxy group, an optionally esterified carboxy group, an alkanoyloxy group, an optionally esterified carboxyalkyl group, an aminosulfonyl group or an optionally substituted carbamoyl group, and
R³ and R⁴ are as defined in the formula (I),
or a pharmaceutically acceptable salt thereof.

3. The compound of claim 2, wherein R³ is an optionally substituted alkyl group, or an optionally substituted alkylcarbonyl group optionally having a double bond, and
R⁴ is a hydrogen atom, a hydroxyl group or a group represented by 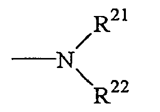 wherein R²¹ and R²² are the same or different and each is a hydrogen atom, an alkyl group or an alkylsulfonyl group, or,
R²¹ and R²² form, together with the nitrogen atom bonded thereto, an optionally substituted heterocycle optionally containing hetero atom(s) besides the nitrogen atom bonded to R²¹ and R²², or a pharmaceutically acceptable salt thereof.

4. The compound of claim 3, wherein the compound is selected from the group consisting of
(1) N-(2,6-diisopropylphenyl)-2-(2,2-dimethylpropionyl)-1,2,3,4-tetrahydroisoquinoline-(3S)-carboxamide
(2) N-(2,6-diisopropylphenyl)-2-octanoyl-1,2,3,4-tetrahydroisoquinoline-(3S)-carboxamide
(3) N-(2,4-difluorophenyl)-2-octanoyl-1,2,3,4-tetrahydroisoquinoline-(3S)-carboxamide
(4) 2-octanoyl-N-(2,4,6-trimethylphenyl)-1,2,3,4-tetrahydroisoquinoline-(3S)-carboxamide
(5) 2-(2,2-dimethylhexanoyl)-N-(2,4,6-trimethylphenyl)-1,2,3,4-tetrahydroisoquinoline-(3S)-carboxamide
(6) 2-hexanoyl-N-(2,4,6-trimethylphenyl)-1,2,3,4-tetrahydroisoquinoline-(3S)-carboxamide
(7) 2-hexanoyl-N-(3-methanesulfonylamino-2,4,6-trimethylphenyl)-1,2,3,4-tetrahydroisoquinoline-(3S)-carboxamide
(8) 2-(2,2-dimethylpropionyl)-N-(3-methanesulfonylamino-2,4,6-trimethylphenyl)-1,2,3,4-tetrahydroisoquinoline-(3S)-carboxamide
(9) 2-[(2E,4E)-hexadienoyl]-N-(3-methanesulfonylamino-2,4,6-trimethylphenyl)-1,2,3,4-tetrahydroisoquinoline-(3S)-carboxamide
(10) 2-(3,3-dimethylbutyryl)-N-(3-methanesulfonylamino-2,4,6-trimethylphenyl)-1,2,3,4-tetrahydroisoquinoline-(3S)-carboxamide
(11) 2-(2,2-dimethylbutyryl)-N-(3-methanesulfonylamino-2,4,6-trimethylphenyl)-1,2,3,4-tetrahydroisoquinoline-(3S)-carboxamide
(12) 2-[(2E,4E)-hexadienoyl]-N-(2,4,6-trimethylphenyl)-1,2,3,4-tetrahydroisoquinoline-(3S)-carboxamide
(13) 2-octanoyl-N-(2,4,6-trimethylphenyl)-1,2,3,4-tetrahydroisoquinoline-(3R)-carboxamide
(14) N-(4-amino-2,6-diisopropylphenyl)-2-octanoyl-1,2,3,4-tetrahydroisoquinoline-(3S)-carboxamide
(15) 2-(2,2-dimethylbutyryl)-N-(2-isopropyl-5-methanesulfonylaminophenyl)-1,2,3,4-tetrahydroisoquinoline-(3S)-carboxamide
(16) 7-dimethylamino-2-octanoyl-N-(2,4,6-trimethylphenyl)-1,2,3,4-tetrahydroisoquinoline-(3S)-carboxamide
(17) 7-dimethylamino-2-(2,2-dimethylpropionyl)-N-(3-methanesulfonylamino-2,4,6-trimethylphenyl)-1,2,3,4-tetrahydroisoquinoline-(3S)-carboxamide
(18) 7-dimethylamino-2-(2,2-dimethylpropionyl)-N-(2,4,6-trimethylphenyl)-1,2,3,4-tetrahydroisoquinoline-(3S)-carboxamide
(19) N-(2,6-diisopropylphenyl)-7-hydroxy-2-octanoyl-1,2,3,4-tetrahydroisoquinoline-(3S)-carboxamide
(20) N-(2,4-difluorophenyl)-7-hydroxy-2-octanoyl-1,2,3,4-tetrahydroisoquinoline-(3S)-carboxamide
(21) N-(2,6-diisopropylphenyl)-2-(2,2-dimethylpropionyl)-7-hydroxy-1,2,3,4-tetrahydroisoquinoline-(3S)-carboxamide
(22) N-(4-amino-2,6-diisopropylphenyl)-2-hexanoyl-7-hydroxy-1,2,3,4-tetrahydroisoquinoline-(3S)-carboxamide and
(23) N-(2,6-diisopropylphenyl)-2-(2,2-dimethylpropyl)-1,2,3,4-tetrahydroisoquinoline-(3S)-carboxamide,
or a pharmaceutically acceptable salt thereof.

5. The compound of claim 2, wherein either of R¹³ and R¹⁴ is a hydroxyl group and the other is a hydrogen atom, an alkyl group or a halogen atom, R¹¹, R¹² and R¹⁵ are each independently a hydrogen atom, an alkyl group or a halogen atom, or a pharmaceutically acceptable salt thereof.

6. The compound of claim 5, wherein R³ is an optionally substituted alkyl group, or an optionally substituted alkylcarbonyl group optionally having a double bond, and R¹³ is a hydroxyl group, or a pharmaceutically acceptable salt thereof.

7. The compound of claim 6, wherein R⁴ is a hydrogen atom, hydroxyl group, or an optionally substituted alkoxy group, or a pharmaceutically acceptable salt thereof.

8. The compound of claim 7, wherein the compound is selected from the group consisting of
(1) 2-(2,2-dimethylpropyl)-N-(4-hydroxy-2,3,5-trimethylphenyl)-1,2,3,4-tetrahydroisoquinoline-(3S)-carboxamide
(2) N-(4-hydroxy-2,3,5-trimethylphenyl)-2-octanoyl-1,2,3,4-tetrahydroisoquinoline-(3S)-carboxamide
(3) N-(4-hydroxy-2,3,5-trimethylphenyl)-2-isobutyryl-1,2,3,4-tetrahydroisoquinoline-(3S)-carboxamide
(4) N-(4-hydroxy-2,3,5-trimethylphenyl)-2-pentyl-1,2,3,4-tetrahydroisoquinoline-(3S)-carboxamide
(5) 2-hexanoyl-7-hydroxy-N-(4-hydroxy-2,3,5-trimethylphenyl)-1,2,3,4-tetrahydroisoquinoline-(3S)-carboxamide
(6) 7-(2-aminoethoxy)-2-hexanoyl-N-(4-hydroxy-2,3,5-trimethylphenyl)-1,2,3,4-tetrahydroisoquinoline-(3S)-carboxamide
(7) 2-hexanoyl-N-(4-hydroxy-2,3,5-trimethylphenyl)-7-[2-(pyrrolidin-1-yl)ethoxy]-1,2,3,4-tetrahydroisoquinoline-(3S)-carboxamide
(8) 2-hexanoyl-7-(3-hydroxypropoxy)-N-(4-hydroxy-2,3,5-trimethylphenyl)-1,2,3,4-tetrahydroisoquinoline-(3S)-carboxamide and
(9) 2-{[2-(2,2-dimethylnonyl)-(3S)-(4-hydroxy-2,3,5-trimethylphenyl)-1,2,3,4-tetrahydroisoquinolin-7-yl]oxy}-2-methylpropionic acid, or a pharmaceutically acceptable salt thereof.

9. The compound of claim 6, wherein R⁴ is a group represented by 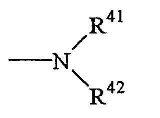 wherein R⁴¹ and R⁴² are the same or different and each is a hydrogen atom, an alkyl group or an alkylsulfonyl group, or R⁴¹ and R⁴² form, together with the nitrogen atom bonded thereto, an optionally substituted heterocycle optionally containing hetero atom(s) besides the nitrogen atom bonded to R⁴¹ and R⁴², or a pharmaceutically acceptable salt thereof.

10. The compound of claim 9, which is selected from the group consisting of
(1) 7-dimethylamino-2-(2,2-dimethylbutyryl)-N-(4-hydroxy-2,3,5-trimethylphenyl)-1,2,3,4-tetrahydroisoquinoline-(3S)-carboxamide
(2) 7-dimethylamino-2-[(2E,4E)-hexadienoyl]-N-(4-hydroxy-2,3,5-trimethylphenyl)-1,2,3,4-tetrahydroisoquinoline-(3S)-carboxamide
(3) 2-butyryl-7-dimethylamino-N-(4-hydroxy-2,3,5-trimethylphenyl)-1,2,3,4-tetrahydroisoquinoline-(3S)-carboxamide
(4) 7-dimethylamino-N-(4-hydroxy-2,3,5-trimethylphenyl)-2-isobutyryl-1,2,3,4-tetrahydroisoquinoline-(3S)-carboxamide
(5) 7-dimethylamino-2-(2,2-dimethylpropionyl)-N-(4-hydroxy-2,3,5-trimethylphenyl)-1,2,3,4-tetrahydroisoquinoline-(3S)-carboxamide
(6) 7-dimethylamino-2-hexanoyl-N-(4-hydroxy-2,3,5-trimethylphenyl)-1,2,3,4-tetrahydroisoquinoline-(3R)-carboxamide
(7) 7-dimethylamino-2-(2,2-dimethylbutyryl)-N-(4-hydroxy-2,3,5-trimethylphenyl)-1,2,3,4-tetrahydroisoquinoline-(3R)-carboxamide
(8) 7-amino-2-hexanoyl-N-(4-hydroxy-2,3,5-trimethylphenyl)-1,2,3,4-tetrahydroisoquinoline-(3S)-carboxamide
(9) 7-dimethylamino-2-hexanoyl-N-(4-hydroxy-2,3,5-trimethylphenyl)-1,2,3,4-tetrahydroisoquinoline-(3S)-carboxamide
(10) 7-dimethylamino-N-(4-hydroxy-2,3,5-trimethylphenyl)-2-propyl-1,2,3,4-tetrahydroisoquinoline-(3S)-carboxamide
(11) 7-amino-2-(2,2-dimethylpropionyl)-N-(4-hydroxy-2,3,5-trimethylphenyl)-1,2,3,4-tetrahydroisoquinoline-(3S)-carboxamide
(12) 2-(2,2-dimethylpropionyl)-N-(4-hydroxy-2,3,5-trimethylphenyl)-7-methanesulfonylamino-1,2,3,4-tetrahydroisoquinoline-(3S)-carboxamide
(13) N-(4-hydroxy-2,3,5-trimethylphenyl)-2-pentanoyl-7-(pyrrolidin-1-yl)-1,2,3,4-tetrahydroisoquinoline-(3S)-carboxamide
(14) N-(4-hydroxy-2,3,5-trimethylphenyl)-2-propionyl-7-(pyrrolidin-1-yl)-1,2,3,4-tetrahydroisoquinoline-(3S)-carboxamide
(15) 2-acetyl-N-(4-hydroxy-2,3,5-trimethylphenyl)-7-(pyrrolidin-1-yl)-1,2,3,4-tetrahydroisoquinoline-(3S)-carboxamide
(16) N-(4-hydroxy-2,3,5-trimethylphenyl)-2-isobutyryl-7-(pyrrolidin-1-yl)-1,2,3,4-tetrahydroisoquinoline-(3S)-carboxamide
(17) 2-hexanoyl-N-(4-hydroxy-2,3,5-trimethylphenyl)-7-methanesulfonylamino-1,2,3,4-tetrahydroisoquinoline-(3S)-carboxamide
(18) 7-amino-N-(4-hydroxy-2,3,5-trimethylphenyl)-2-[5-methyl-(2E)-hexenoyl]-1,2,3,4-tetrahydroisoquinoline-(3S)-carboxamide
(19) 7-amino-2-(5-hexenoyl)-N-(4-hydroxy-2,3,5-trimethylphenyl)-1,2,3,4-tetrahydroisoquinoline-(3S)-carboxamide
(20) 7-amino-N-(4-hydroxy-2,3,5-trimethylphenyl)-2-(4-methylpentanoyl)-1,2,3,4-tetrahydroisoquinoline-(3S)-carboxamide
(21) 7-amino-N-(4-hydroxy-2,3,5-trimethylphenyl)-2-pentanoyl-1,2,3,4-tetrahydroisoquinoline-(3S)-carboxamide
(22) 7-amino-2-(2,2-dimethylbutyryl)-N-(4-hydroxy-2,3,5-trimethylphenyl)-1,2,3,4-tetrahydroisoquinoline-(3S)-carboxamide
(23) 7-amino-2-(3,3-dimethylbutyryl)-N-(4-hydroxy-2,3,5-trimethylphenyl)-1,2,3,4-tetrahydroisoquinoline-(3S)-carboxamide
(24) 2-(3,3-dimethylbutyryl)-N-(4-hydroxy-2,3,5-trimethylphenyl)-7-methanesulfonylamino-1,2,3,4-tetrahydroisoquinoline-(3S)-carboxamide
(25) 2-hexanoyl-N-(4-hydroxy-2,3,5-trimethylphenyl)-7-(piperazin-1-yl)-1,2,3,4-tetrahydroisoquinoline-(3S)-carboxamide and
(26) 2-(2,2-dimethylpropionyl)-N-(4-hydroxy-2,3,5-trimethylphenyl)-7-(4-methylpiperazin-1-yl)-1,2,3,4-tetrahydroisoquinoline-(3S)-carboxamide,
or a pharmaceutically acceptable salt thereof.

11. The compound of claim 6, wherein R⁴ is a group represented by 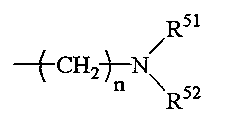 wherein n is an integer of 1 to 3, R⁵¹ and R⁵² are the same or different and each is a hydrogen atom or an optionally substituted alkyl group, or R⁵¹ and R⁵² form, together with the nitrogen atom bonded thereto, an optionally substituted heterocycle optionally containing hetero atom(s) besides the nitrogen atom bonded to R⁵¹ and R⁵², or a pharmaceutically acceptable salt thereof.

12. The compound of claim 11, which is selected from the group consisting of
(1) 7-dimethylaminomethyl-2-(2,2-dimethylbutyryl)-N-(4-hydroxy-2,3,5-trimethylphenyl)-1,2,3,4-tetrahydroisoquinoline-(3S)-carboxamide
(2) 7-diethylaminomethyl-2-(2,2-dimethylpropionyl)-N-(4-hydroxy-2,3,5-trimethylphenyl)-1,2,3,4-tetrahydroisoquinoline-(3S)-carboxamide
(3) 7-diisopropylaminomethyl-N-(4-hydroxy-2,3,5-trimethylphenyl)-2-isobutyryl-1,2,3,4-tetrahydroisoquinoline-(3S)-carboxamide
(4) 2-(2,2-dimethylpropionyl)-N-(4-hydroxy-2,3,5-trimethylphenyl)-7-(N-methylpropylamino)methyl-1,2,3,4-tetrahydroisoquinoline-(3S)-carboxamide
(5) N-(4-hydroxy-2,3,5-trimethylphenyl)-2-isobutyryl-7-(N-methylpropylamino)methyl-1,2,3,4-tetrahydroisoquinoline-(3S)-carboxamide
(6) 2-(2,2-dimethylpropionyl)-7-(2-fluoro-N-methylethylamino)methyl-N-(4-hydroxy-2,3,5-trimethylphenyl)-1,2,3,4-tetrahydroisoquinoline-(3S)-carboxamide
(7) 2-(2,2-dimethylpropionyl)-7-(2-fluoroethylamino)methyl-N-(4-hydroxy-2,3,5-trimethylphenyl)-1,2,3,4-tetrahydroisoquinoline-(3S)-carboxamide
(8) 2-(2,2-dimethylpropionyl)-7-(3-fluoroazetidin-1-yl)methyl-N-(4-hydroxy-2,3,5-trimethylphenyl)-1,2,3,4-tetrahydroisoquinoline-(3S)-carboxamide and
(9) 2-(2,2-dimethylpropionyl)-N-(4-hydroxy-2,3,5-trimethylphenyl)-7-(3-methoxyazetidin-1-yl)methyl-1,2,3,4-tetrahydroisoquinoline-(3S)-carboxamide,
or a pharmaceutically acceptable salt thereof.

13. A pharmaceutical composition comprising a compound described in any of claims 1 to 12 or a pharmaceutically acceptable salt thereof.

14. An antioxidant comprising a compound described in any of claims 6 to 12 or a pharmaceutically acceptable salt thereof.

15. An acyl-Coenzyme A: cholesterol acyltransferase inhibitor comprising a 1,2,3,4-tetrahydroisoquinoline compound having an optionally substituted carbamoyl group at the 3-position, or a pharmaceutically acceptable salt thereof.

16. The inhibitor of claim 15, wherein the 2-position of the 1,2,3,4-tetrahydroisoquinoline compound is substituted by a group represented by R³ in claim 1.

17. The inhibitor of claim 15, wherein the 1,2,3,4-tetrahydroisoquinoline compound is any of the compounds of claims 1 to 12.

18. An agent for the prophylaxis or treatment of hyperlipidemia, arteriosclerosis or inflammatory disease, which comprises any of the compounds of claims 1 to 12, or a pharmaceutically acceptable salt thereof.

19. A method of preventing or treating hyperlipidemia, arteriosclerosis or inflammatory disease, which comprises administering an effective amount of any of the compounds of claims 1 to 12 or a pharmaceutically acceptable salt thereof.

20. Use of any of the compounds of claims 1 to 12 or a pharmaceutically acceptable salt thereof, for the production of a pharmaceutical agent for the prophylaxis or treatment of hyperlipidemia, arteriosclerosis or inflammatory disease.

21. A commercial package comprising an agent for the prophylaxis or treatment of hyperlipidemia, arteriosclerosis or inflammatory disease, which comprises any of the compounds of claims 1 to 12 or a pharmaceutically acceptable salt thereof, and a written matter stating that the agent can or should be used for the prophylaxis or treatment of hyperlipidemia, arteriosclerosis or inflammatory disease.
